# EUROPEAN PATENT APPLICATION

(11) **EP 3 760 229 A2**
(43) Date of publication of application: **06.01.2021**
(21) Application number: 20178309.9
(22) Date of filing: 15.05.2015
(51) Int. Cl.: A61K 39/395, A61K 45/06, C07K 16/28, C07K 16/22, C07K 16/30

(54) **TREATMENT OF LUNG CANCER USING A COMBINATION OF AN ANTI-PD-1 ANTIBODY AND ANOTHER ANTI-CANCER AGENT**

(30) Priority: 15.05.2014 US 201461993917 P; 30.05.2014 US 201462005640 P; 24.04.2015 US 201562152669 P
(62) Divisional of application: 15726468.0
(71) Applicant: Bristol-Myers Squibb Company, Princeton, NJ 08543 (US)
(72) Inventor: FELTQUATE, David, Princeton, NJ 08543 (US); CHEN, Allen C., Princeton, NJ 08543 (US)
(74) Representative: Mewburn Ellis LLP

(57) **Abstract**

This disclosure provides a method for treating a subject afflicted with a lung cancer, which method comprises administering to the subject therapeutically effective amounts of: (a) an anti-cancer agent which is an antibody or an antigen-binding portion thereof that specifically binds to a Programmed Death-1 (PD-1) receptor and inhibits PD-1 activity; and (b) another anti-cancer agent. The other anti-cancer agent can be a platinum-based doublet chemotherapy, an EGFR-targeted tyrosine kinase inhibitor, bevacizumab, an anti- Cytotoxic T-Lymphocyte Antigen-4 (CTLA-4) antibody, or any other therapy used to treat lung cancer in the art or disclosed herein.

## Description

Throughout this application, various publications are referenced in parentheses by author name and date, or by Patent No. or Patent Publication No. Full citations for these publications can be found at the end of the specification immediately preceding the claims. The disclosures of these publications are hereby incorporated in their entireties by reference into this application in order to more fully describe the state of the art as known to those skilled therein as of the date of the invention described and claimed herein. However, the citation of a reference herein should not be construed as an acknowledgement that such reference is prior art to the present invention.

### FIELD OF THE INVENTION

This invention relates to methods for treating lung cancer in a subject comprising administering to the subject a combination of an anti-cancer agent which is an anti-Programmed Death-1 (PD-1) antibody and another anti-cancer agent such as an anti-Cytotoxic T-Lymphocyte Antigen-4 (CTLA-4) antibody, a targeted tyrosine kinase inhibitor, an anti-VEGF antibody, or a platinum-based doublet chemotherapeutic agent.

### BACKGROUND OF THE INVENTION

Human cancers harbor numerous genetic and epigenetic alterations, generating neoantigens potentially recognizable by the immune system (Sjoblom *et al.,* 2006). The adaptive immune system, comprised of T and B lymphocytes, has powerful anti-cancer potential, with a broad capacity and exquisite specificity to respond to diverse tumor antigens. Further, the immune system demonstrates considerable plasticity and a memory component. The successful harnessing of all these attributes of the adaptive immune system would make immunotherapy unique among all cancer treatment modalities.

Until recently, cancer immunotherapy had focused substantial effort on approaches that enhance anti-tumor immune responses by adoptive-transfer of activated effector cells, immunization against relevant antigens, or providing non-specific immune-stimulatory agents such as cytokines. In the past decade, however, intensive efforts to develop specific immune checkpoint pathway inhibitors have begun to provide new immunotherapeutic approaches for treating cancer, including the development of an antibody (Ab), ipilimumab (YERVOY®), that binds to and inhibits CTLA-4 for the treatment of patients with advanced melanoma (Hodi *et al.,* 2010) and the development of Abs such as nivolumab and pembrolizumab (formerly lambrolizumab; USAN Council Statement, 2013) that bind specifically to the Programmed Death -1 (PD-1) receptor and block the inhibitory PD-1/PD-1 ligand pathway (Topalian *et al.,* 2012a, b; Topalian *et al.,* 2014; Hamid *et al.,* 2013; Hamid and Carvajal, 2013; McDermott and Atkins, 2013).

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. Two cell surface glycoprotein ligands for PD-1 have been identified, Programmed Death Ligand-1 (PD-L1) and Programmed Death Ligand-2 (PD-L2), that are expressed on antigen-presenting cells as well as many human cancers and have been shown to downregulate T cell activation and cytokine secretion upon binding to PD-1. Inhibition of the PD-1/PD-L1 interaction mediates potent antitumor activity in preclinical models (U.S. Patent Nos. 8,008,449 and 7,943,743), and the use of Ab inhibitors of the PD-1/PD-L1 interaction for treating cancer has entered clinical trials (Brahmer *et al.,* 2010; Topalian *et al.,* 2012a; Topalian *et al.,* 2014; Hamid *et al.,* 2013; Brahmer *et al.,* 2012; Flies *et al.,* 2011; Pardoll, 2012; Hamid and Carvajal, 2013).

Nivolumab (formerly designated 5C4, BMS-936558, MDX-1106, or ONO-4538) is a fully human IgG4 (S228P) PD-1 immune checkpoint inhibitor Ab that selectively prevents interaction with PD-1 ligands (PD-L1 and PD-L2), thereby blocking the down-regulation of antitumor T-cell functions (U.S. Patent No. 8,008,449; Wang *et al.,* 2014). Nivolumab has shown activity in a variety of advanced solid tumors including renal cell carcinoma (renal adenocarcinoma, or hypernephroma), melanoma, and non-small cell lung cancer (NSCLC) (Topalian *et al.,* 2012a; Topalian *et al.,* 2014; Drake *et al.,* 2013; WO 2013/173223).

Ipilimumab (YERVOY®) is a fully human, IgG1 monoclonal Ab that blocks the binding of CTLA-4 to its B7 ligands, thereby stimulating T cell activation and improving overall survival (OS) in patients with advanced melanoma (Hodi *et al*., 2010). Concurrent therapy with nivolumab and ipilimumab in a Phase 1 clinical trial produced rapid and deep tumor regression in a substantial proportion of patients with advanced melanoma, and was significantly more effective than either Ab alone (Wolchok *et al.,* 2013; WO 2013/173223). However, it was hitherto not known whether this combination of immunoregulatory Abs would be similarly effective in other tumor types.

NSCLC is the leading cause of cancer death in the U.S. and worldwide (NCCN GUIDELINES®, 2013 - Non-Small Cell Lung Cancer). NSCLCs are relatively insensitive to chemotherapy but patients with Stage IV disease who have a good performance status (PS) benefit from treatment with chemotherapeutic drugs, including platinum agents (*e.g.,* cisplatin, carboplatin), taxanes agents (*e.g.,* paclitaxel, albumin-bound paclitaxel, docetaxel), vinorelbine, vinblastine, etoposide, pemetrexed and gemcitabine, and various combinations of these drugs.

Specific targeted therapies have also been developed for the treatment of advanced lung cancer. For example, bevacizumab (AVASTIN®) is a mAb that blocks vascular endothelial growth factor A (VEGF-A). Erlotinib (TARCEVA®) is a small-molecule TKI of epidermal growth factor receptor (EGFR). Crizotinib (XALKORI®) is a small-molecule TKI that targets ALK and MET, and is used to treat NSCLC in patients carrying the mutated *ALK* fusion gene. Cetuximab (ERBITUX®) is a mAb that targets EGFR.

As described herein, significant antitumor activity of nivolumab in combination with platinum-based doublet chemotherapy, the TKI erlotinib, the VEGF-A mAb bevacizumab, or the anti-CTLA-4 mAb ipilimumab, has been demonstrated in clinical trials.

### SUMMARY OF THE INVENTION

The present disclosure provides a method for treating a subject afflicted with a lung cancer comprising administering to the subject a combination of therapeutically effective amounts of: (a) an Ab or an antigen-binding portion thereof that specifically binds to and inhibits PD-1; and either (b) a platinum-based doublet chemotherapy, (c) an EGFR-targeted TKI, or (d) an Ab or an antigen-binding portion thereof that specifically binds to and inhibits CTLA-4. In some embodiments, the lung cancer is non-small cell lung cancer (NSCLC). In certain embodiments of any of the therapeutic methods disclosed herein, the anti-PD-1 Ab is nivolumab. In other embodiments, the anti-PD-1 Ab is pembrolizumab. In certain other embodiments of any of the therapeutic methods disclosed herein, the anti-CTLA-4 Ab is ipilimumab. In other embodiments, the anti-CTLA-4 Ab is tremelimumab.

In certain embodiments, the subject has been pre-treated for the lung cancer. In other embodiments, the lung cancer is an advanced, metastatic and/or refractory cancer. In some embodiments, the administration of the combination of the Ab or antigen-binding portion thereof and the other anti-cancer agent induces a durable clinical response in the subject.

The disclosure also provides a kit for treating a subject afflicted with a lung cancer, the kit comprising: (a) a dosage ranging from 0.1 to 10 mg/kg body weight of an anti-cancer agent which is an antibody or an antigen-binding portion thereof that specifically binds to the PD-1 receptor and inhibits PD-1 activity; (b) a dosage of another anti-cancer agent which is (i) a platinum-based doublet chemotherapy; (ii) an EGFR-targeted tyrosine kinase inhibitor; (iii) bevacizumab; or (iv) a dosage ranging from 0.1 to 10 mg/kg body weight of an antibody or an antigen-binding portion thereof that specifically binds to and inhibits CTLA-4; and (c) instructions for using the anti-PD-1 antibody and the other anti-cancer agent for treating the subject.

Other features and advantages of the instant invention will be apparent from the following detailed description and examples which should not be construed as limiting. The contents of all cited references, including scientific articles, newspaper reports, GenBank entries, patents and patent applications cited throughout this application are expressly incorporated herein by reference.

### BRIEF DESCRIPTION OF THE DRAWINGS

Figure 1 shows the CA209-012 study design: nivolumab in combination with platinum-based doublet chemotherapy.
Figure 2 shows characteristics of ORs by treatment arm in NSCLC patients treated with nivolumab plus platinum-based chemotherapy.
Figure 3 shows percent change in target lesions from baseline in NSCLC patients treated with nivolumab plus platinum-based chemotherapy.
Figure 4 shows best percent change in target lesion tumor burden from baseline in NSCLC patients treated with nivolumab plus platinum-based chemotherapy.
Figure 5 shows PFS and OS in NSCLC patients treated with nivolumab plus platinum-based chemotherapy,
Figure 6 shows the CA209-012 study design: nivolumab in combination with erlotinib.
Figure 7 shows characteristics of ORs in NSCLC patients treated with nivolumab plus erlotinib.
Figure 8 shows percent changes in target lesion tumor burden in NSCLC patients treated with nivolumab plus erlotinib. A) Percent change in lesion tumor burden from baseline; B) best percent change in target lesion tumor burden from baseline.
Figure 9 shows PFS and OS in NSCLC patients treated with nivolumab plus erlotinib. mPFS= median PFS. mOS= median OS
Figure 10 shows the CA209-012 study design: nivolumab in combination with ipilimumab.
Figure 11 shows characteristics of ORs by treatment arm in NSCLC patients treated with nivolumab plus ipilimumab.
Figure 12 shows best percent change in target lesion tumor burden from baseline in NSCLC patients treated with nivolumab plus ipilimumab.
Figure 13 shows percent change in target lesions from baseline in NSCLC patients treated with nivolumab plus ipilimumab.
Figure 14 shows PFS and OS in NSCLC patients treated with nivolumab plus ipilimumab.
Figure 15 shows response by PD-L1 status in NSCLC patients treated with nivolumab plus ipilimumab: A) best percent change in target lesion tumor burden from baselinea and B) PFS.
Figure 16 shows OS by nivolumab dose in NSCLC patients.
Figure 17 shows OS by histology in NSCLC patients.
Figure 18 shows characteristics of responses by histology in patients with NSCLC treated with nivolumab.
Figure 19 shows best change in target lesion tumor burden by *EGFR* and *KRAS* mutation status.
Figure 20 shows best change in target lesion tumor burden by tumor PD-L1 expression.
Figure 21 shows PFS by tumor PD-L1 expression in patients with NSCLC.
Figure 22 shows OS by tumor PD-L1 expression in patients with NSCLC.
Figure 23 shows a study design schematic for an Open-Label, Randomized Phase 3 Trial of Nivolumab versus platinum-based doublet chemotherapy and Nivolumab plus Ipilimumab versus platinum-based doublet chemotherapy in subjects with chemotherapy-naive stage IV or Recurrent Non-Small Cell Lung Cancer (NSCLC).

### DETAILED DESCRIPTION OF THE INVENTION

The present invention relates to methods for treating a lung cancer patient comprising administering to the patient a combination of an anti-PD-1 Ab and another anti-cancer agent.

### Terms

In order that the present disclosure can be more readily understood, certain terms are first defined. As used in this application, except as otherwise expressly provided herein, each of the following terms shall have the meaning set forth below. Additional definitions are set forth throughout the application.

"Administering" refers to the physical introduction of a composition comprising a therapeutic agent to a subject, using any of the various methods and delivery systems known to those skilled in the art. Routes of administration for the anti-PD-1 Ab include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as *in vivo* electroporation. The TKI is typically administered via a non-parenteral route, in some embodiments, orally. Other non-parenteral routes include a topical, epidermal or mucosal route of administration, for example, intranasally, vaginally, rectally, sublingually or topically. Administering can also be performed, for example, once, a plurality of times, and/or over one or more extended periods.

An "adverse event" (AE) as used herein is any unfavorable and generally unintended or undesirable sign (including an abnormal laboratory finding), symptom, or disease associated with the use of a medical treatment. For example, an adverse event can be associated with activation of the immune system or expansion of immune system cells (*e.g.,* T cells) in response to a treatment. A medical treatment can have one or more associated AEs and each AE can have the same or different level of severity. Reference to methods capable of "altering adverse events" means a treatment regime that decreases the incidence and/or severity of one or more AEs associated with the use of a different treatment regime.

An "antibody" (Ab) shall include, without limitation, a glycoprotein immunoglobulin which binds specifically to an antigen and comprises at least two heavy (H) chains and two light (L) chains interconnected by disulfide bonds, or an antigen-binding portion thereof Each H chain comprises a heavy chain variable region (abbreviated herein as V*_{H}*) and a heavy chain constant region. The heavy chain constant region comprises three constant domains, C_{*H*1}, C_{*H*2} and C_{*H*3}. Each light chain comprises a light chain variable region (abbreviated herein as V*_{L}*) and a light chain constant region. The light chain constant region is comprises one constant domain, C*_{L}*. The V*_{H}* and V*_{L}* regions can be further subdivided into regions of hypervariability, termed complementarity determining regions (CDRs), interspersed with regions that are more conserved, termed framework regions (FR). Each V*_{H}* and V*_{L}* comprises three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the Abs can mediate the binding of the immunoglobulin to host tissues or factors, including various cells of the immune system (*e.g*., effector cells) and the first component (C1q) of the classical complement system.

An immunoglobulin can derive from any of the commonly known isotypes, including but not limited to IgA, secretory IgA, IgG and IgM. IgG subclasses are also well known to those in the art and include but are not limited to human IgG1, IgG2, IgG3 and IgG4. "Isotype" refers to the Ab class or subclass (*e.g*., IgM or IgG1) that is encoded by the heavy chain constant region genes. The term "antibody" includes, by way of example, both naturally occurring and non-naturally occurring Abs; monoclonal and polyclonal Abs; chimeric and humanized Abs; human or nonhuman Abs; wholly synthetic Abs; and single chain Abs. A nonhuman Ab can be humanized by recombinant methods to reduce its immunogenicity in man. Where not expressly stated, and unless the context indicates otherwise, the term "antibody" also includes an antigen-binding fragment or an antigen-binding portion of any of the aforementioned immunoglobulins, and includes a monovalent and a divalent fragment or portion, and a single chain Ab.

An "isolated antibody" refers to an Ab that is substantially free of other Abs having different antigenic specificities (*e.g*., an isolated Ab that binds specifically to PD-1 is substantially free of Abs that bind specifically to antigens other than PD-1). An isolated Ab that binds specifically to PD-1 can, however, have cross-reactivity to other antigens, such as PD-1 molecules from different species. Moreover, an isolated Ab can be substantially free of other cellular material and/or chemicals.

The term "monoclonal antibody" ("mAb") refers to a non-naturally occurring preparation of Ab molecules of single molecular composition, *i.e.,* Ab molecules whose primary sequences are essentially identical, and which exhibits a single binding specificity and affinity for a particular epitope. A mAb is an example of an isolated Ab. MAbs can be produced by hybridoma, recombinant, transgenic or other techniques known to those skilled in the art.

A "human" antibody (HuMAb) refers to an Ab having variable regions in which both the framework and CDR regions are derived from human germline immunoglobulin sequences. Furthermore, if the Ab contains a constant region, the constant region also is derived from human germline immunoglobulin sequences. The human Abs of the invention can include amino acid residues not encoded by human germline immunoglobulin sequences (e.g., mutations introduced by random or site-specific mutagenesis *in vitro* or by somatic mutation *in vivo*)*.* However, the term "human antibody," as used herein, is not intended to include Abs in which CDR sequences derived from the germline of another mammalian species, such as a mouse, have been grafted onto human framework sequences. The terms "human" Abs and "fully human" Abs and are used synonymously.

A "humanized antibody" refers to an Ab in which some, most or all of the amino acids outside the CDR domains of a non-human Ab are replaced with corresponding amino acids derived from human immunoglobulins. In one embodiment of a humanized form of an Ab, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the Ab to bind to a particular antigen. A "humanized" Ab retains an antigenic specificity similar to that of the original Ab.

A "chimeric antibody" refers to an Ab in which the variable regions are derived from one species and the constant regions are derived from another species, such as an Ab in which the variable regions are derived from a mouse Ab and the constant regions are derived from a human Ab.

An "anti-antigen" Ab refers to an Ab that binds specifically to the antigen. For example, an anti-PD-1 Ab binds specifically to PD-1 and an anti-CTLA-4 Ab binds specifically to CTLA-4.

An "antigen-binding portion" of an Ab (also called an "antigen-binding fragment") refers to one or more fragments of an Ab that retain the ability to bind specifically to the antigen bound by the whole Ab.

A "cancer" refers a broad group of various diseases characterized by the uncontrolled growth of abnormal cells in the body. Unregulated cell division and growth divide and grow results in the formation of malignant tumors that invade neighboring tissues and can also metastasize to distant parts of the body through the lymphatic system or bloodstream.

"Cytotoxic T-Lymphocyte Antigen-4 (CTLA-4) refers to an immunoinhibitory receptor belonging to the CD28 family. CTLA-4 is expressed exclusively on T cells *in vivo,* and binds to two ligands, CD80 and CD86 (also called B7-1 and B7-2, respectively). The term "CTLA-4" as used herein includes human CTLA-4 (hCTLA-4), variants, isoforms, and species homologs of hCTLA-4, and analogs having at least one common epitope with hCTLA-4. The complete hCTLA-4 sequence can be found under GenBank Accession No. AAB59385.

The term "immunotherapy" refers to the treatment of a subject afflicted with, or at risk of contracting or suffering a recurrence of, a disease by a method comprising inducing, enhancing, suppressing or otherwise modifying an immune response. "Treatment" or "therapy" of a subject refers to any type of intervention or process performed on, or the administration of an active agent to, the subject with the objective of reversing, alleviating, ameliorating, inhibiting, slowing down or preventing the onset, progression, development, severity or recurrence of a symptom, complication or condition, or biochemical indicia associated with a disease.

"Programmed Death-1 (PD-1)" refers to an immunoinhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1. The complete hPD-1 sequence can be found under GenBank Accession No. U64863.

"Programmed Death Ligand-1 (PD-L1)" is one of two cell surface glycoprotein ligands for PD-1 (the other being PD-L2) that downregulate T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The complete hPD-L1 sequence can be found under GenBank Accession No. Q9NZQ7.

A "subject" includes any human or nonhuman animal. The term "nonhuman animal" includes, but is not limited to, vertebrates such as nonhuman primates, sheep, dogs, and rodents such as mice, rats and guinea pigs. In some embodiments, the subject is a human. The terms, "subject" and "patient" are used interchangeably herein.

A "therapeutically effective amount" or "therapeutically effective dosage" of a drug or therapeutic agent is any amount of the drug that, when used alone or in combination with another therapeutic agent, protects a subject against the onset of a disease or promotes disease regression evidenced by a decrease in severity of disease symptoms, an increase in frequency and duration of disease symptom-free periods, or a prevention of impairment or disability due to the disease affliction. The ability of a therapeutic agent to promote disease regression can be evaluated using a variety of methods known to the skilled practitioner, such as in human subjects during clinical trials, in animal model systems predictive of efficacy in humans, or by assaying the activity of the agent in *in vitro* assays.

By way of example, an "anti-cancer agent" promotes cancer regression in a subject or prevents further tumor growth. In certain embodiments, a therapeutically effective amount of the drug promotes cancer regression to the point of eliminating the cancer. "Promoting cancer regression" means that administering an effective amount of the drug, alone or in combination with an anti-neoplastic agent, results in a reduction in tumor growth or size, necrosis of the tumor, a decrease in severity of at least one disease symptom, an increase in frequency and duration of disease symptom-free periods, or a prevention of impairment or disability due to the disease affliction. In addition, the terms "effective" and "effectiveness" with regard to a treatment includes both pharmacological effectiveness and physiological safety. Pharmacological effectiveness refers to the ability of the drug to promote cancer regression in the patient. Physiological safety refers to the level of toxicity, or other adverse physiological effects at the cellular, organ and/or organism level (adverse effects) resulting from administration of the drug.

By way of example for the treatment of tumors, a therapeutically effective amount of an anti-cancer agent can inhibit cell growth or tumor growth by at least about 20%, by at least about 40%, by at least about 60%, or by at least about 80% relative to untreated subjects. In other embodiments of the invention, tumor regression can be observed and continue for a period of at least about 20 days, at least about 40 days, or at least about 60 days. Notwithstanding these ultimate measurements of therapeutic effectiveness, evaluation of immunotherapeutic drugs must also make allowance for "immune-related" response patterns.

An "immune-related" response pattern refers to a clinical response pattern often observed in cancer patients treated with immunotherapeutic agents that produce antitumor effects by inducing cancer-specific immune responses or by modifying native immune processes. This response pattern is characterized by a beneficial therapeutic effect that follows an initial increase in tumor burden or the appearance of new lesions, which in the evaluation of traditional chemotherapeutic agents would be classified as disease progression and would be synonymous with drug failure. Accordingly, proper evaluation of immunotherapeutic agents can require long-term monitoring of the effects of these agents on the target disease.

A therapeutically effective amount of a drug includes a "prophylactically effective amount," which is any amount of the drug that, when administered alone or in combination with an anti-neoplastic agent to a subject at risk of developing a cancer (e.g., a subject having a pre-malignant condition) or of suffering a recurrence of cancer, inhibits the development or recurrence of the cancer. In certain embodiments, the prophylactically effective amount prevents the development or recurrence of the cancer entirely. "Inhibiting" the development or recurrence of a cancer means either lessening the likelihood of the cancer's development or recurrence, or preventing the development or recurrence of the cancer entirely.

The use of the alternative (e.g., "or") should be understood to mean either one, both, or any combination thereof of the alternatives. As used herein, the indefinite articles "a" or "an" should be understood to refer to "one or more" of any recited or enumerated component.

The terms "about" or "comprising essentially of" refer to a value or composition that is within an acceptable error range for the particular value or composition as determined by one of ordinary skill in the art, which will depend in part on how the value or composition is measured or determined, i.e., the limitations of the measurement system. For example, "about" or "comprising essentially of" can mean within 1 or more than 1 standard deviation per the practice in the art. Alternatively, "about" or "comprising essentially of" can mean a range of up to 10% or 20% (i.e., ±10% or ±20%). For example, about 3mg can include any number between 2.7 mg and 3.3 mg (for 10%) or between 2.4 mg and 3.6 mg (for 20%). Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

The term "about once a week" as used herein means approximate number, and "about once a week" can include every seven days ± two days, i.e., every five days to every nine days. The dosing frequency of "once a week" thus can be every five days, every six days, every seven days, every eight days, or every nine days.

As described herein, any concentration range, percentage range, ratio range or integer range is to be understood to include the value of any integer within the recited range and, when appropriate, fractions thereof (such as one tenth and one hundredth of an integer), unless otherwise indicated.

Various aspects of the invention are described in further detail in the following subsections.

### Antii-PD-1 antibodies and anti-PD-L1 antibodies

HuMAbs that bind specifically to PD-1 with high affinity have been disclosed in U.S. Patent No. 8,008,449. Other anti-PD-1 mAbs have been described in, for example, U.S. Patent Nos. 6,808,710, 7,488,802, 8,168,757 and 8,354,509, and PCT Publication No. WO 2012/145493. Each of the anti-PD-1 HuMAbs disclosed in U.S. Patent No. 8,008,449 has been demonstrated to exhibit one or more of the following characteristics: (a) binds to human PD-1 with a K_{D} of 1 x 10⁻⁷ M or less, as determined by surface plasmon resonance using a Biacore biosensor system; (b) does not substantially bind to human CD28, CTLA-4 or ICOS; (c) increases T-cell proliferation in a Mixed Lymphocyte Reaction (MLR) assay; (d) increases interferon-y production in an MLR assay; (e) increases IL-2 secretion in an MLR assay; (f) binds to human PD-1 and cynomolgus monkey PD-1; (g) inhibits the binding of PD-L1 and/or PD-L2 to PD-1; (h) stimulates antigen-specific memory responses; (i) stimulates Ab responses; and (j) inhibits tumor cell growth *in vivo.* Anti-PD-1 Abs usable in the present invention include mAbs that bind specifically to human PD-1 and exhibit at least one, in some embodiments, at least five, of the preceding characteristics. In some embodiments, the anti-PD-1 Ab is nivolumab. In one embodiment, the anti-PD-1 Ab is pembrolizumab.

In one embodiment, the anti-PD-1 Ab is nivolumab. Nivolumab (also known as "OPDIVO®"; formerly designated 5C4, BMS-936558, MDX-1106, or ONO-4538) is a fully human IgG4 (S228P) PD-1 immune checkpoint inhibitor Ab that selectively prevents interaction with PD-1 ligands (PD-L1 and PD-L2), thereby blocking the down-regulation of antitumor T-cell functions (U.S. Patent No. 8,008,449; Wang et al., 2014 Cancer Immunol Res. 2(9):846-56).

In another embodiment, the anti-PD-1 Ab is pembrolizumab. Pembrolizumab (also known as "KEYTRUDA®", lambrolizumab, and MK-3475) is a humanized monoclonal IgG4 antibody directed against human cell surface receptor PD-1 (programmed death-1 or programmed cell death-1). Pembrolizumab is described, for example, in U.S. Patent Nos. 8,354,509 and 8,900,587; see also http://www.cancer.gov/drugdictionary?cdrid=695789 (last accessed: December 14, 2014). Pembrolizumab has been approved by the FDA for the treatment of relapsed or refractory melanoma.

In other embodiments, the anti-PD-1 Ab is MEDI0608 (formerly AMP-514), which is a monoclonal antibody. MEDI0608 is described, for example, in US Pat. No. 8,609,089B2 or in http://www.cancer.gov/drugdictionary?cdrid=756047 (last accessed December 14, 2014).

In some embodiments, the anti-PD-1 antibody is Pidilizumab (CT-011), which is a humanized monoclonal antibody. Pidilizumab is described in US Pat. No. 8,686,119 B2 or WO 2013/014668 A1.

Anti-PD-1 Abs usable in the disclosed methods also include isolated Abs that bind specifically to human PD-1 and cross-compete for binding to human PD-1 with nivolumab (*see, e.g.,* U.S. Patent No. 8,008,449 and 8,779,105; WO 2013/173223). The ability of Abs to cross-compete for binding to an antigen indicates that these Abs bind to the same epitope region of the antigen and sterically hinder the binding of other cross-competing Abs to that particular epitope region. These cross-competing Abs are expected to have functional properties very similar those of nivolumab by virtue of their binding to the same epitope region of PD-1. Cross-competing Abs can be readily identified based on their ability to cross-compete with nivolumab in standard PD-1 binding assays such as Biacore analysis, ELISA assays or flow cytometry (*see, e.g.,* WO 2013/173223).

In certain embodiments, the Abs that cross-compete for binding to human PD-1 with, or bind to the same epitope region of human PD-1 as, nivolumab are mAbs. For administration to human subjects, these cross-competing Abs are chimeric Abs, or humanized or human Abs. Such chimeric, humanized or human mAbs can be prepared and isolated by methods well known in the art.

Anti-PD-1 Abs usable in the methods of the disclosed invention also include antigen-binding portions of the above Abs. It has been amply demonstrated that the antigen-binding function of an Ab can be performed by fragments of a full-length Ab. Examples of binding fragments encompassed within the term "antigen-binding portion" of an Ab include (i) a Fab fragment, a monovalent fragment consisting of the V*_{L}*, V*_{H}*, C*_{L}* and C_{*H*1} domains; (ii) a F(ab')₂ fragment, a bivalent fragment comprising two Fab fragments linked by a disulfide bridge at the hinge region; (iii) a Fd fragment consisting of the V*_{H}* and C_{*H*1} domains; and (iv) a Fv fragment consisting of the V*_{L}* and V*_{H}* domains of a single arm of an Ab.

Anti-PD-1 Abs suitable for use in the disclosed methods or compositions are Abs that bind to PD-1 with high specificity and affinity, block the binding of PD-L1 and or PD-L2, and inhibit the immunosuppressive effect of the PD-1 signaling pathway. In any of the compositions or methods disclosed herein, an anti-PD-1 "antibody" includes an antigen-binding portion or fragment that binds to the PD-1 receptor and exhibits the functional properties similar to those of whole Abs in inhibiting ligand binding and upregulating the immune system. In certain embodiments, the anti-PD-1 Ab or antigen-binding portion thereof cross-competes with nivolumab for binding to human PD-1. In other embodiments, the anti-PD-1 Ab or antigen-binding portion thereof is a chimeric, humanized or human monoclonal Ab or a portion thereof. In certain embodiments, the Ab is a humanized Ab. In other embodiments, the Ab is a human Ab. Abs of an IgG1, IgG2, IgG3 or IgG4 isotype can be used.

In certain embodiments, the anti-PD-1 Ab or antigen-binding portion thereof comprises a heavy chain constant region which is of a human IgG1 or IgG4 isotype. In certain other embodiments, the sequence of the IgG4 heavy chain constant region of the anti-PD-1 Ab or antigen-binding portion thereof contains an S228P mutation which replaces a serine residue in the hinge region with the proline residue normally found at the corresponding position in IgG1 isotype antibodies. This mutation, which is present in nivolumab, prevents Fab arm exchange with endogenous IgG4 antibodies, while retaining the low affinity for activating Fc receptors associated with wild-type IgG4 antibodies (Wang et al., 2014). In yet other embodiments, the Ab comprises a light chain constant region which is a human kappa or lambda constant region. In other embodiments, the anti-PD-1 Ab or antigen-binding portion thereof is a mAb or an antigen-binding portion thereof. In certain embodiments of any of the therapeutic methods described herein comprising administration of an anti-PD-1 Ab, the anti-PD-1 Ab is nivolumab. In other embodiments, the anti-PD-1 Ab is pembrolizumab. In other embodiments, the anti-PD-1 Ab is chosen from the human antibodies 17D8, 2D3, 4H1, 4A11, 7D3 and 5F4 described in U.S. Patent No. 8,008,449. In still other embodiments, the anti-PD-1 Ab is MEDI0608 (formerly AMP-514), AMP-224, or Pidilizumab (CT-011).

In certain embodiments, an anti-PD-1 antibody used in the methods can be replaced with another PD-1 or anti-PD-L1 antagonist. For example, because an anti-PD-L1 antibody prevents interaction between PD-1 and PD-L1, thereby exerting similar effects to the signaling pathway of PD-1, an anti-PD-L1 antibody can replace the use of an anti-PD-1 antibody in the methods disclosed herein. Therefore, in one embodiment, the present invention is directed to a method for treating a subject afflicted with a lung cancer comprising administering to the subject a combination of therapeutically effective amounts of: (a) an anti-cancer agent which is an antibody or an antigen-binding portion thereof that binds specifically to a Programmed Death-Ligandl (PD-L1) receptor and inhibits PD-L1 activity (an anti-PD-L1 antibody); and (b) another anti-cancer agent (e.g., platinum-based doublet chemotherapy, an anti-VEGF antibody, an EGFR-targeted TKI, or an anti-CTLA4 antibody). In certain embodiments, the anti-PD-L1 Ab is BMS-936559 (formerly 12A4 or MDX-1105) (see, e.g., U.S. Patent No. 7,943,743; WO 2013/173223). In other embodiments, the anti-PD-L1 Ab is MPDL3280A (also known as RG7446) (see, e.g., Herbst; U.S. Patent No. 8,217,149), MEDI4736 (also called Durvalumab; Khleif, 2013, See US Patent No. 8,779,108 or US 2014/0356353, filed May 6, 2014), or MSB0010718C (also called Avelumab; See US 2014/0341917).

### Anti-CTLA-4 antibodies

Anti-CTLA-4 antibodies of the instant invention bind to human CTLA-4 so as to disrupt the interaction of CTLA-4 with a human B7 receptor. Because the interaction of CTLA-4 with B7 transduces a signal leading to inactivation of T-cells bearing the CTLA-4 receptor, disruption of the interaction effectively induces, enhances or prolongs the activation of such T cells, thereby inducing, enhancing or prolonging an immune response.

HuMAbs that bind specifically to CTLA-4 with high affinity have been disclosed in U.S. Patent Nos. 6,984,720 and 7,605,238. Other anti-PD-1 mAbs have been described in, for example, U.S. Patent Nos. 5,977,318, 6,051,227, 6,682,736, and 7,034,121. The anti-PD-1 HuMAbs disclosed in U.S. Patent No. Nos. 6,984,720 and 7,605,238 have been demonstrated to exhibit one or more of the following characteristics: (a) binds specifically to human CTLA-4 with a binding affinity reflected by an equilibrium association constant (K*ₐ*) of at least about 10⁷ M⁻¹, or about 10⁹ M⁻¹, or about 10¹⁰ M⁻¹ to 10¹¹ M⁻¹ or higher, as determined by Biacore analysis; (b) a kinetic association constant (k*ₐ*) of at least about 10³, about 10⁴, or about 10⁵ m⁻¹ s⁻¹; (c) a kinetic disassociation constant (k*_{d}*) of at least about 10³, about 10⁴, or about 10⁵ m⁻¹ s⁻¹; and (d) inhibits the binding of CTLA-4 to B7-1 (CD80) and B7-2 (CD86). Anti-CTLA-4 Abs usable in the present invention include mAbs that bind specifically to human CTLA-4 and exhibit at least one, and, in one embodiment,at least three of the preceding characteristics. An exemplary clinical anti-CTLA-4 Ab is the human mAb 10D1 (now known as ipilimumab and marketed as YERVOY®) as disclosed in U.S. Patent No. 6,984,720. Ipilimumab is an anti-CTLA-4 Ab for use in the methods disclosed herein. Another anti-CTLA-4 Ab usable in the present methods is tremelimumab.

An exemplary clinical anti-CTLA-4 Ab is the human mAb 10D1 (now known as ipilimumab and marketed as YERVOY®) as disclosed in U.S. Patent No. 6,984,720. Ipilimumab is an anti-CTLA-4 Ab for use in the methods disclosed herein. Ipilimumab is a fully human, IgG1 monoclonal Ab that blocks the binding of CTLA-4 to its B7 ligands, thereby stimulating T cell activation and improving overall survival (OS) in patients with advanced melanoma.

Another anti-CTLA-4 Ab useful for the present methods is tremelimumab (also known as CP-675,206). Tremelimumab is human IgG2 monoclonal anti-CTLA-4 antibody. Tremelimumab is described in WO/2012/122444, U.S. Publ. No. 2012/263677, or WO Publ. No. 2007/113648 A2.

Anti-CTLA-4 Abs usable in the disclosed methods also include isolated Abs that bind specifically to human PD-1 and cross-compete for binding to human CTLA-4 with ipilimumab or tremelimumab or bind to the same epitope region of human CTLA-4 as ipilimumab or tremelimumab. In certain embodiments, the Abs that cross-compete for binding to human CTLA-4 with, or bind to the same epitope region of human PD-1 as does ipilimumab or tremelimumab, are Abs comprising a heavy chain of the human IgG1 isotype. For administration to human subjects, these cross-competing Abs are chimeric Abs, or humanized or human Abs. Usable anti-CTLA-4 Abs also include antigen-binding portions of the above Abs such as Fab, F(ab')₂, Fd or Fv fragments.

### Anti-VEGF Antibody

Vascular endothelial growth factor ("VEGF") is an endothelial cell-specific mitogen and an inducer of angiogenesis. VEGF has a prominent role in angiogenesis and tumor growth and development. In some embodiments of this invention, the anti-PD1 antibody is administered in combination with an anti-VEGF antagonist. In certain embodiments, the anti-VEGF antagonist is an anti-VEGF antibody, antigen binding molecule or fragment thereof. In certain embodiments, the anti-VEGF antibody is bevacizumab (described in U.S. Patent Number 7,169,901), or any other VEGF antibody known in the art including ranibizumab (U.S. Patent Number 7,297,334), VGX-100 (U.S. Patent Number 7,423,125), r84 (U.S. Patent Number 8,034,905), aflibercept (U.S. Patent Number 5,952,199), IMC-18F1 (U.S. Patent Number 7,972,596), IMC-1C11 (PCT/US2000/02180), and ramucirumab (U.S. Patent Number 7,498,414).

### Chemotherapy and Platinum-based Chemotherapy

In some embodiments, the anti-PD1 antibody is administered in combination with any chemotherapy known in the art. In certain embodiments, the chemotherapy is a platinum based-chemotherapy. Platinum-based chemotherapies are coordination complexes of platinum. In some embodiments, the platinum-based chemotherapy is a platinum-doublet chemotherapy. In one embodiments, the chemotherapy is administered at the approved dose for the particular indication. In other embodiments, the chemotherapy is administered at any dose disclosed herein. In some embodiments, the platinum-based chemotherapy is cisplatin, carboplatin, oxaliplatin, satraplatin, picoplatin, Nedaplatin, Triplatin, Lipoplatin, or combinations thereof. In certain embodiments, the platinum-based chemotherapy is any other platinum-based chemotherapy known in the art. In some embodiments, the chemotherapy is the nucleotide analog gemcitabine. In an embodiment, the chemotherapy is a folate antimetabolite. In an embodiment, the folate antimetabolite is pemetrexed. In certain embodiments the chemotherapy is a taxane. In other embodiments, the taxane is paclitaxel. In some embodiments, the chemotherapy is any other chemotherapy known in the art. In certain embodiments, at least one, at least two or more chemotherapeutic agents are administered in combination with the anti-PD1 antibody. In some embodiments, the anti-PD1 antibody is administered in combination with gemcitabine and cisplatin. In some embodiments, the anti-PD1 antibody is administered in combination with pemetrexed and cisplatin. In certain embodiments, the anti-PD1 antibody is administered in combination with gemcitabine and pemetrexed. In one embodiment, the anti-PD1 antibody is administered in combination with paclitaxel and carboplatin. In an embodiment, an anti-CTLA4 antibody is additionally administered.

### Tyrosine Kinase Inhibitors

In certain embodiments, the anti-PD-1 antibody is administered in combination with a tyrosine kinase inhibitor. In certain embodiments, the tyrosine kinase inhibitor is gefitinib, erlotinib, combinations thereof or any other tyrosine kinase inhibitor known in the art. In some embodiments, the tyrosine kinase inhibitor act on the epidermal growth factor receptor (EGFR). In an embodiment, an anti-CTLA4 antibody is additionally administered.

### Standard-of-Care Therapies for Lung Cancer

Standard-of-care therapies for different types of cancer are well known by persons of skill in the art. For example, the National Comprehensive Cancer Network (NCCN), an alliance of 21 major cancer centers in the USA, publishes the NCCN Clinical Practice Guidelines in Oncology (NCCN GUIDELINES®) that provide detailed up-to-date information on the standard-of-care treatments for a wide variety of cancers (*see* NCCN GUIDELINES®, 2014).

NSCLC is the leading cause of cancer death in the U.S. and worldwide, exceeding breast, colon and prostate cancer combined. In the U.S., an estimated 228,190 new cases of lung and bronchial will be diagnosed in the U.S., and some 159,480 deaths will occur because of the disease (Siegel *et al.,* 2013). The majority of patients (approximately 78%) are diagnosed with advanced/recurrent or metastatic disease. Metastases to the adrenal gland from lung cancer are a common occurrence, with about 33% of patients having such metastases. NSCLC therapies have incrementally improved OS, but benefit has reached a plateau (median OS for late stage patients is just 1 year). Progression after 1L therapy occurred in nearly all of these subjects and the 5-year survival rate is only 3.6% in the refractory setting. From 2005 to 2009, the overall 5-year relative survival rate for lung cancer in the U.S. was 15.9% (NCCN GUIDELINES®, 2014 - Non-Small Cell Lung Cancer).

Surgery, radiation therapy (RT) and chemotherapy are the three modalities commonly used to treat NSCLC patients. As a class, NSCLCs are relatively insensitive to chemotherapy and RT, compared to small cell carcinoma. In general, for patients with Stage I or II disease, surgical resection provides the best chance for cure, with chemotherapy increasingly being used both pre-operatively and post-operatively. RT can also be used as adjuvant therapy for patients with resectable NSCLC, the primary local treatment, or as palliative therapy for patients with incurable NSCLC.

Patients with Stage IV disease who have a good performance status (PS) benefit from chemotherapy. Many drugs, including platinum agents (*e.g.*, cisplatin, carboplatin), taxanes agents (*e.g.*, paclitaxel, albumin-bound paclitaxel, docetaxel), vinorelbine, vinblastine, etoposide, pemetrexed and gemcitabine are useful for Stage IV NSCLC. Combinations using many of these drugs produce 1-year survival rates of 30% to 40% and are superior to single agents. Specific targeted therapies have also been developed for the treatment of advanced lung cancer. For example, bevacizumab (AVASTIN®) is a mAb that blocks vascular endothelial growth factor A (VEGF-A). Erlotinib (TARCEVA®) is a small-molecule TKI of epidermal growth factor receptor (EGFR). Crizotinib (XALKORI®) is a small-molecule TKI that targets ALK and MET, and is used to treat NSCLC in patients carrying the mutated *ALK* fusion gene. Cetuximab (ERBITUX®) is a mAb that targets EGFR.

There is a particular unmet need among patients who have squamous cell NSCLC (representing up to 25% of all NSCLC) as there are few treatment options after first line (1L) therapy. Single-agent chemotherapy is standard of care following progression with platinum-based doublet chemotherapy (Pt-doublet), resulting in median OS of approximately 7 months. Docetaxel remains the benchmark treatment in this line of therapy although erlotinib can also be used with less frequency. Pemetrexed has also been shown to produce clinically equivalent efficacy outcomes but with significantly fewer side effects compared with docetaxel in the second line (2L) treatment of patients with advanced NSCLC (Hanna *et al.,* 2004). No therapy is currently approved for use in lung cancer beyond the third line (3L) setting. Pemetrexed and bevacizumab are not approved in squamous NSCLC, and molecularly targeted therapies have limited application. The unmet need in advanced lung cancer has been compounded by the recent failure of Oncothyreon and Merck KgaA's STIMUVAX® to improve OS in a phase 3 trial, inability of ArQule's and Daiichi Sankyo's c-Met kinase inhibitor, tivantinib, to meet survival endpoints, failure of Eli Lilly's ALIMTA® in combination with Roche's AVASTIN® to improve OS in a late-stage study, and Amgen's and Takeda Pharmaceutical's failure to meet clinical endpoints with the small-molecule VEGF-R antagonist, motesanib, in late-stage trials.

### Immunotherapy of Lung Cancer

A clear need exists for effective agents for patients who have progressed on multiple lines of targeted therapy, as well as for therapies that extend survival for longer periods beyond the current standard treatments. Newer approaches involving immunotherapy, especially blockade of immune checkpoints including the CTLA-4, PD-1, and PD-L1 inhibitory pathways, have recently shown promise (Greelan *et al.,* 2014). Thus, ipilimumab in combination with chemotherapy has exhibited encouraging results in small-cell and non-small-cell lung cancer alike. In addition, dual checkpoint blockade strategies, such as those combining anti-PD-1 and anti-CTLA-4 have proven to be highly effective in treating melanoma (Wolchok *et al,* 2013; WO 2013/173223), and other combinations including anti-PD-Ll, anti-LAG-3, or anti-KIR, are being tested to increase the proportion and durability of tumor responses. By analogy to melanoma, NSCLC patients can to benefit either from the combination of different immunotherapeutic drugs or the combination of such drugs with targeted agents or other treatments including, surgery, radiation, standard cancer chemotherapies, or vaccines. However, surprising and unexpected complications have sometimes been observed when immunotherapeutics are combined with other anti-cancer agents. Thus, the combination of immunotherapy (including an immune checkpoint inhibitor drug such as an anti-CTLA-4 or anti-PD-1 Ab) with other anti-cancer agents is unpredictable and must be carefully assessed for safety as well as efficacy in clinical trials. Although the combination of nivolumab and ipilimumab has proven to be very efficacious in treating melanoma with manageable toxicity (Wolchok *et al.,* 2013), it was not hitherto known whether this combination would be significantly more effective in human subjects than treatment of NSCLC and other cancers with the individual agents.

### Pharmaceutical Compositions and Dosages

Therapeutic agents of the present invention can be constituted in a composition, *e.g.*, a pharmaceutical composition containing an Ab or a TKI and a pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. In one embodiment, the carrier for a composition containing an Ab is suitable for intravenous, intramuscular, subcutaneous, parenteral, spinal or epidermal administration (*e.g.*, by injection or infusion), whereas the carrier for a composition containing a TKI is suitable for non-parenterat *e.g.*, oral, administration. A pharmaceutical composition of the invention can include one or more pharmaceutically acceptable salts, anti-oxidant, aqueous and non-aqueous carriers, and/or adjuvants such as preservatives, wetting agents, emulsifying agents and dispersing agents.

Dosage regimens are adjusted to provide the optimum desired response, *e.g.*, a maximal therapeutic response and/or minimal adverse effects. For administration of an anti-PD-1 Ab, especially in combination with another anti-cancer agent, the dosage can range from about 0.01 to about 20 mg/kg, about 0.1 to about 10 mg/kg, about 0.1 to about 5 mg/kg, about 1 to about 5 mg/kg, about 2 to about 5 mg/kg, about 7.5 to about 12.5 mg/kg, or about 0.1 to about 30 mg/kg of the subject's body weight. For example, dosages can be about 0.1, about 0.3, about 1, about 2, about 3, about 5 or about 10 mg/kg body weight, or , about 0.3, about 1, about 2, about 3, or about 5 mg/kg body weight. The dosing schedule is typically designed to achieve exposures that result in sustained receptor occupancy (RO) based on typical pharmacokinetic properties of an Ab. An exemplary treatment regime entails administration about once per week, about once every 2 weeks, about once every 3 weeks, about once every 4 weeks, about once a month, about once every 3-6 months or longer. In certain embodiments, an anti-PD-1 Ab such as nivolumab is administered to the subject about once every 2 weeks. In other embodiments, the Ab is administered about once every 3 weeks. The dosage and scheduling can change during a course of treatment. For example, a dosing schedule for anti-PD-1 monotherapy can comprise administering the Ab: (i) about every 2 weeks in about 6-week cycles; (ii) about every 4 weeks for about six dosages, then about every three months; (iii) about every 3 weeks; (iv) about 3- about 10 mg/kg once followed by about 1 mg/kg every about 2-3 weeks. Considering that an IgG4 Ab typically has a half-life of 2-3 weeks, a dosage regimen for an anti-PD-1 Ab of the invention comprises about 0.3-1 about 0 mg/kg body weight, 1-5 mg/kg body weight, or about 1- about 3 mg/kg body weight via intravenous administration, with the Ab being given every about 14-21 days in up to about 6-week or about 12-week cycles until complete response or confirmed progressive disease. In some embodiments, the antibody treatment, or any combination treatment disclosed herein, is continued for at least about 1 month, at least about 3 months, at least about 6 months, at least about 9 months, at least about 1 year, at least about 18 months, at least about 24 months, at least about 3 years, at least about 5 years, or at least about 10 years.

When used in combinations with other cancer agents, the dosage of an anti-PD-1 Ab can be lowered compared to the monotherapy dose. For example, a dosage of nivolumab that is significantly lower than the typical about 3 mg/kg about every 3 weeks, for instance about 0. 1 mg/kg or less about every 3 or 4 weeks, is regarded as a subtherapeutic dosage. Receptor-occupancy data from 15 subjects who received 0.3 mg/kg to 10 mg/kg dosing with nivolumab indicate that PD-1 occupancy appears to be dose-independent in this dose range. Across all doses, the mean occupancy rate was 85% (range, 70% to 97%), with a mean plateau occupancy of 72% (range, 59% to 81%) (Brahmer *et al.,* 2010). Thus, 0.3 mg/kg dosing can allow for sufficient exposure to lead to maximal biologic activity.

Although higher nivolumab monotherapy dosing up to about 10 mg/kg every two weeks has been achieved without reaching the maximum tolerated does (MTD), the significant toxicities reported in other trials of checkpoint inhibitors plus anti-angiogenic therapy (*see, e.g.,* Johnson *et al.,* 2013; Rini *et al.,* 2011) support the selection of a nivolumab dose lower than 10 mg/kg.

In certain embodiments, the dose of an anti-PD1 antibody (or an anti-PD-L1 antibody) is a fixed dose in a pharmaceutical composition. In other embodiments, the method of the present invention can be used with a flat dose (a dose given to a patient irrespective of the body weight of the patient). For example, a flat dose of a nivolumab can be about 240mg. For example, a flat dose of pembrolizumab can be about 200 mg.

Ipilimumab (YERVOY®) is approved for the treatment of melanoma at 3 mg/kg given intravenously once every 3 weeks for 4 doses. Thus, in some embodiments, about 3 mg/kg is the highest dosage of ipilimumab used in combination with the anti-PD-1 Ab though, in certain embodiments, an anti-CTLA-4 Ab such as ipilimumab can be dosed within the range of about about 0.3 to about 10 mg/kg, about 0.5 to about 10 mg/kg, about 0.5 to about 5 mg/kg, or about 1 to about 5 mg/kg. body weight about every two or three weeks when combined with nivolumab. In other embodiments, ipilimumab is administered on a different dosage schedule from nivolumab. In some embodiments, ipilimumab is administered about every week, about every two weeks, about every three weeks, about every 4 weeks, about every five weeks, about every six weeks, about every seven weeks, about every eight weeks, about every nine weeks, about every ten weeks, about every eleven weeks, about every twelve weeks or about every fifteen weeks. A dosage of ipilimumab that is significantly lower than the approved about 3 mg/kg about every 3 weeks, for instance about 0.3 mg/kg or less about every 3 or 4 weeks, is regarded as a subtherapeutic dosage. It has been shown that combination dosing of nivolumab at 3 mg/kg and ipilimumab at 3 mg/kg exceeded the MTD in a melanoma population, whereas a combination of nivolumab at 1 mg/kg plus ipilimumab at 3 mg/kg or nivolumab at 3 mg/kg plus ipilimumab at 1 mg/kg was found to be tolerable in melanoma patients (Wolchok *et al.,* 2013). Accordingly, although nivolumab is tolerated up to 10 mg/kg given intravenously every 2 weeks, in certain embodiments doses of the anti-PD-1 Ab do not exceed about 3 mg/kg when combined with ipilimumab. In certain embodiments, based on risk-benefit and PK-PD assessments, the dosage used comprises a combination of nivolumab at about 1 mg/kg plus ipilimumab at about 3 mg/kg, nivolumab at about 3 mg/kg plus ipilimumab at about 1 mg/kg, or nivolumab at about 3 mg/kg plus ipilimumab at about 3 mg/kg is used, each administered at a dosing frequency of once about every 2-4 weeks, in certain embodiments, once about every 3 weeks. In certain other embodiments, nivolumab is administered at a dosage of about 0.1, about 0.3, about 1, about 2, about 3 or about 5 mg/kg in combination with ipilimumab administered at a dosage of about 0.1, about 0.3, about 1, about 2, about 3 or about 5 mg/kg, once about every 2 weeks, once about every 3 weeks, or once about every 4 weeks.

In certain embodiments, the combination of an anti-PD-1 Ab and an anti-CTLA-4 Ab is administered intravenously to the subject in an induction phase about every 2 or 3 weeks for 1, 2, 3 or 4 administrations. In certain embodiments, the combination of nivolumab and ipilimumab is administered intravenously in the induction phase about every 3 weeks for about 4 administrations. The induction phase is followed by a maintenance phase during which only the anti-PD-1 Ab is administered to the subject at a dosage of about 0.1, about 0.3, about 1, about 2, about 3, about 5 or about 10 mg/kg every two or three weeks for as long as the treatment proves efficacious or until unmanageable toxicity or disease progression occurs. In certain embodiments, nivolumab is administered during the maintenance phase at a dose of about 3 mg/kg body about every 2 weeks.

In certain embodiments, the anti-PD1 antibody and the anti-CTLA-4 antibody is formulated as a single composition, wherein the dose of the anti-PD1 antibody and the dose of the anti-CTLA04 antibody are combined at a ratio of 1:50, 1:40, 1:30, 1:20, 1:10. 1:5, 1:3, 1:1, 3:1, 5:1, 10:1, 20:1, 30:1, 40:1, or 50:1. In other embodiments, the dose of the anti-CTLA-4 antibody is a fixed dose. In certain embodiments, the dose of the anti-CTLA-4 antibody is a flat dose, which is given to a patient irrespective of the body weight. In a specific embodiment, the flat dose of the anti-CTLA-4 antibody is about 80 mg.

For combination of nivolumab with other anti-cancer agents, these agents are administered at their approved dosages. Treatment is continued as long as clinical benefit is observed or until unacceptable toxicity or disease progression occurs. Nevertheless, in certain embodiments, the dosages of these anti-cancer agents administered are significantly lower than the approved dosage, *i.e.*, a subtherapeutic dosage, of the agent is administered in combination with the anti-PD-1 Ab. The anti-PD-1 Ab can be administered at the dosage that has been shown to produce the highest efficacy as monotherapy in clinical trials, *e.g*., about 3 mg/kg of nivolumab administered once about every three weeks (Topalian *et al.,* 2012a; Topalian *et al.,* 2012), or at a significantly lower dose, *i.e.,* at a subtherapeutic dose.

Dosage and frequency vary depending on the half-life of the Ab in the subject. In general, human Abs show the longest half-life, followed by humanized Abs, chimeric Abs, and nonhuman Abs. The dosage and frequency of administration can vary depending on whether the treatment is prophylactic or therapeutic. In prophylactic applications, a relatively low dosage is typically administered at relatively infrequent intervals over a long period of time. Some patients continue to receive treatment for the rest of their lives. In therapeutic applications, a relatively high dosage at relatively short intervals is sometimes required until progression of the disease is reduced or terminated, or until the patient shows partial or complete amelioration of symptoms of disease. Thereafter, the patient can be administered a prophylactic regime.

Actual dosage levels of the active ingredients in the pharmaceutical compositions of the present invention can be varied so as to obtain an amount of the active ingredient which is effective to achieve the desired therapeutic response for a particular patient, composition, and mode of administration, without being unduly toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts. A composition of the present invention can be administered via one or more routes of administration using one or more of a variety of methods well known in the art. As will be appreciated by the skilled artisan, the route and/or mode of administration will vary depending upon the desired results.

### Methods of the Invention

This disclosure provides a method of treating a subject afflicted with a lung cancer, which method comprises administering to the subject a combination of therapeutically effective amounts of: (a) an anti-cancer agent which is an Ab or an antigen-binding portion thereof that specifically binds to and a PD-1 receptor and inhibits PD-1 activity; and (b) another anti-cancer agent. As NSCLC comprises more than 85% of lung tumors, in embodiments the lung cancer is NSCLC. In other embodiments, the subject is a human patient. In certain embodiments, the subject is a chemotherapy-naïve patient (e.g., a patient who has not previously received any chemotherapy). In other embodiments, the subject has received another cancer therapy (*e.g*., a chemotherapy), but is resistant or refractory to such another cancer therapy. In certain specific embodiments, the subject has cancer cells expressing mutated forms of the *EGFR* or *KRAS* gene. In certain embodiments, the subject has cancer cells that are PDL1+. In certain embodiments, the subject has cancer cells that are PDL1-. In certain embodiments, the subject has cancer cells that are squamous. In certain embodiments, the subject has cancer cells that are nonsquamous.

In certain embodiments, the combination therapy of the present invention (e.g., administration of an anti-PD-1 antibody and another anti-cancer agent) effectively increases the duration of survival of the subject. For example, the duration of survival of the subject is increased by at least about 2 months when compared to another subject treated with only one therapy (e.g., an anti-PD-1 antibody or another anti-cancer agent). In certain embodiments, the combination therapy of the present invention (e.g., administration of an anti-PD-1 antibody and another anti-cancer agent) effectively increases the duration of progression free survival of the subject. For example, the progression free survival of the subject is increased by at least about 2 months when compared to another subject treated with only one therapy (*e.g*., an anti-PD-1 antibody or another anti-cancer agent). In certain embodiments, the combination therapy of the present invention (e.g., administration of an anti-PD-1 antibody and another anti-cancer agent) effectively increases the response rate in a group of subjects. For example, the response rate in a group of subjects is increased by at least about 10%, at least about 9%, at least about 8%, at least about 7%, at least about 6%, at least about 5%, at least about 4%, at least about 3%, at least about 2%, or at least about 1% when compared to another group of subjects treated with only one therapy (*e.g*., an anti-PD-1 antibody or another anti-cancer agent). In certain embodiments, the response rate is increased by at least about 2% when compared to another group of subjects treated with only one therapy.

### Anti-PD-1 and anti-PD-L1 antibodies suitable for use in the disclosed methods

Anti-PD-1 Abs suitable for use in the disclosed methods are Abs that bind to PD-1 with high specificity and affinity, block the binding of PD-L1 and or PD-L2, and inhibit the immunosuppressive effect of the PD-1 signaling pathway. In any of the therapeutic methods disclosed herein, an anti-PD-1 or anti-CTLA-4 "antibody" includes an antigen-binding portion or fragment that binds to the PD-1 or CTLA-4 receptor, respectively, and exhibits the functional properties similar to those of whole Abs in inhibiting ligand binding and upregulating the immune system. In certain embodiments, the anti-PD-1 Ab or antigen-binding portion thereof cross-competes with nivolumab for binding to human PD-1. In other embodiments, the anti-PD-1 Ab or antigen-binding portion thereof is a chimeric, humanized or human monoclonal Ab or a portion thereof. In certain embodiments for treating a human subject, the Ab is a humanized Ab. In other embodiments for treating a human subject, the Ab is a human Ab. Abs of an IgG1, IgG2, IgG3 or IgG4 isotype can be used.

In certain embodiments, the anti-PD-1 Ab or antigen-binding portion thereof comprises a heavy chain constant region which is of a human IgG1 or IgG4 isotype. In certain other embodiments, the sequence of the IgG4 heavy chain constant region of the anti-PD-1 Ab or antigen-binding portion thereof contains an S228P mutation which replaces a serine residue in the hinge region with the proline residue normally found at the corresponding position in IgG1 isotype antibodies. This mutation, which is present in nivolumab, prevents Fab arm exchange with endogenous IgG4 antibodies, while retaining the low affinity for activating Fc receptors associated with wild-type IgG4 antibodies (Wang *et al.,* 2014). In yet other embodiments, the Ab comprises a light chain constant region which is a human kappa or lambda constant region. In other embodiments, the anti-PD-1 Ab or antigen-binding portion thereof is a mAb or an antigen-binding portion thereof. In certain embodiments of any of the therapeutic methods described herein comprising administration of an anti-PD-1 Ab, the anti-PD-1 Ab is nivolumab. In other embodiments, the anti-PD-1 Ab is pembrolizumab. In other embodiments, the anti-PD-1 Ab is chosen from the human antibodies 17D8, 2D3, 4H1, 4A11, 7D3 and 5F4 described in U.S. Patent No. 8,008,449. In still other embodiments, the anti-PD-1 Ab is MEDI0608 (formerly AMP-514), AMP-224, or Pidilizumab (CT-011). Because anti-PD-1 and anti-PD-L1 target the same signaling pathway and have been shown in clinical trials to exhibit similar levels of efficacy in a variety of cancers, including RCC (*see* Brahmer *et al.,* 2012; Topalian *et al.,* 2012a; WO 2013/173223), an anti-PD-L1 Ab can be substituted for the anti-PD-1 Ab in any of the therapeutic methods disclosed herein. In certain embodiments, the anti-PD-L1 Ab is BMS-936559 (formerly 12A4 or MDX-1105) (*see, e.g.,* U.S. Patent No. 7,943,743; WO 2013/173223). In other embodiments, the anti-PD-L1 Ab is MPDL3280A (also known as RG7446) (*see, e.g.,* Herbst *et al.* 2013; U.S. Patent No. 8,217,149) or MEDI4736 (Khleif, 2013).

### Combination of an anti-PD-1 Ab with an anti-CTLA-4 Ab for treating NSCLC

This disclosure also provides combination therapy methods for treating RCC wherein an anti-PD-1 Ab is combined with another anti-cancer agent which is an Ab or an antigen-binding portion thereof that binds specifically to CTLA-4 and inhibits CTLA-4 activity. The combination of the anti-PD-1 Ab, nivolumab, and the anti-CTLA-4 Ab, ipilimumab has been demonstrated herein (*see* Example 4) to produce early, durable antitumor activity in NSCLC patients. Accordingly, in certain embodiments, the anti-CTLA-4 Ab that is used in combination with the anti-PD-1 Ab is ipilimumab. In embodiments, the anti-CTLA-4 Ab is tremelimumab. In other embodiments, the anti-CTLA-4 Ab or antigen-binding portion thereof is an Ab or portion thereof that cross-competes with ipilimumab for binding to human CTLA-4. In certain other embodiments, the anti-CTLA-4 Ab or antigen-binding portion thereof is a chimeric, humanized or human mAb or a portion thereof. In yet other embodiments, the anti-CTLA-4 Ab or antigen-binding portion thereof comprises a heavy chain constant region which is of a human IgG1 or IgG4 isotype. In some embodiments, the anti-CTLA-4 Ab comprises a heavy chain constant region which is of a human IgG1 isotype.

For the combination of an anti-PD-1 and an anti-CTLA-4 Ab, the dosing regimen comprises an induction period (also referred to herein as an induction phase) during which one or more, in some embodiments about four, combination doses of the anti-PD-1 and anti-CTLA-4 Abs are administered to the patient, followed by a maintenance period or phase comprising dosing with the anti-PD-1 Ab alone, *i.e.,* not including the anti-CTLA-4 Ab. In certain embodiments, the method comprises (a) an induction phase, wherein the anti-PD-1 and anti-CTLA-4 antibodies or antigen-binding portions thereof are administered in combination in about 2, about 4, about 6, about 8 or about 10 doses, each dose ranging from about 0.1 to about 10.0 mg/kg body weight administered at least once about every 2 weeks, once about every 3 weeks, or once about every 4 weeks, followed by (b) a maintenance phase, wherein no anti-CTLA-4 antibody or antigen-binding portion thereof is administered and the anti-PD-1 antibody or antigen-binding portion thereof is repeatedly administered at a dose ranging from about 0.1 to about 10 mg/kg, about 1 to about 10 mg/kg, about 0.1 to about 5 mg/kg, about 1 to about 5 mg/kg or about 2.5 to about 12.5 mg/kg at least once about every 2 weeks, once about every 3 weeks, or once about every 4 weeks.

In certain embodiments, (a) the induction phase comprises at least about 4 doses administered at 3-week intervals, wherein the anti-PD-1 and anti-CTLA-4 Abs are administered at the following dosages: (i) about 0.1 mg/kg anti-PD-1 Ab and about 3 mg/kg of anti-CTLA-4 Ab; (ii) about 0.3 mg/kg anti-PD-1 Ab and about 3 mg/kg of anti-CTLA-4 Ab; (iii) about 1 mg/kg anti-PD-1 Ab and about 3 mg/kg of anti-CTLA-4 Ab; (iv) about 3 mg/kg anti-PD-1 Ab and about 3 mg/kg of anti-CTLA-4 Ab; (v) about 5 mg/kg anti-PD-1 Ab and about 3 mg/kg of anti-CTLA-4 Ab; (vi) about 10 mg/kg anti-PD-1 Ab and about 3 mg/kg of anti-CTLA-4 Ab; (vii) about 0.1 mg/kg anti-PD-1 Ab and about 1 mg/kg of anti-CTLA-4 Ab; (viii) about 0.3 mg/kg anti-PD-1 Ab and about 1 mg/kg of anti-CTLA-4 Ab; (ix) about 1 mg/kg anti-PD-1 Ab and about 1 mg/kg of anti-CTLA-4 Ab; (x) about 3 mg/kg anti-PD-1 Ab and about 1 mg/kg of anti-CTLA-4 Ab; (xi) about 5 mg/kg anti-PD-1 Ab and about 1 mg/kg of anti-CTLA-4 Ab; or (xii) about 10 mg/kg anti-PD-1 Ab and about 1 mg/kg of anti-CTLA-4 Ab, and (b) the maintenance phase comprises repeated administration of the anti-PD-1 Ab at a dose of about 3 mg/kg about every 2 weeks.

Because of durability of the clinical effect previously demonstrated with immunotherapy by inhibition of immune checkpoints (*see, e.g.,* WO 2013/173223), the maintenance phase can include, in alternative embodiments, a finite number of doses, *e.g.,* about 1-10 doses, or can involve dosing at long intervals, *e.g.,* about once every 3-6 months or about once every 1-2 years or longer intervals. The maintenance phase can be continued for as long as clinical benefit is observed or until unmanageable toxicity or disease progression occurs.

Given the uncertainty of whether the ipilimumab administered past week 12 contributes to clinical benefit in melanoma and the fact that the U.S. Food and Drug Administration (FDA)- and European Medicines Agency (EMA)-approved schedule for YERVOY® is every 3 weeks for a total of 4 doses, in some embodiments the anti-CTLA-4 Ab is administered during the induction phase about once every 3 weeks for a total of about 4 doses. Accordingly, in certain embodiments, the method comprises (a) an induction phase consisting of about 4 combination doses administered at about 3-week intervals, wherein (i) the anti-PD-1 antibody or antigen-binding portion thereof is administered at about 3 mg/kg body weight and the anti-CTLA-4 antibody or antigen-binding portion thereof is administered at about 1 mg/kg body weight; (ii) the anti-PD-1 antibody or antigen-binding portion thereof is administered at about 1 mg/kg body weight and the anti-CTLA-4 antibody or antigen-binding portion thereof is administered at about 3 mg/kg body weight; (iii) the anti-PD-1 antibody or antigen-binding portion thereof is administered at about 1 mg/kg body weight and the anti-CTLA-4 antibody or antigen-binding portion thereof is administered at about 1 mg/kg body weight; or (iv) the anti-PD-1 antibody or antigen-binding portion thereof is administered at about 3 mg/kg body weight and the anti-CTLA-4 antibody or antigen-binding portion thereof is administered at about 3 mg/kg body weight; and (b) the maintenance phase comprises repeated administration of the anti-PD-1 antibody or antigen-binding portion thereof at a dose of about 3 mg/kg about every 2 weeks. In further embodiments of these methods, the maintenance phase is continued for as long as clinical benefit is observed or until unacceptable or unmanageable toxicity or disease progression occurs.

In certain embodiments of the present methods, the anti-PD-1 Ab is nivolumab. In other embodiments, it is pembrolizumab. In yet other embodiments, the anti-CTLA-4 Ab is ipilimumab. In further embodiments, the anti-CTLA-4 Ab is tremelimumab. Typically, the anti-PD-1 and anti-CTLA-4 Abs are formulated for intravenous administration. In certain embodiments, when the anti-PD-1 and anti-CTLA-4 Abs are administered in combination, they are administered within 30 minutes of each other. Either Ab can be administered first, that is, in certain embodiments, the anti-PD-1 Ab is administered before the anti-CTLA-4 Ab, whereas in other embodiments, the anti-CTLA-4 Ab is administered before the anti-PD-1 Ab. Typically, each Ab is administered by intravenous infusion over a period of 60 minutes. In certain embodiments, the anti-PD-1 and anti-CTLA-4 Abs are administered concurrently, either admixed as a single composition in a pharmaceutically acceptable formulation for concurrent administration, or concurrently as separate compositions with each Ab in a pharmaceutically acceptable formulation.

Certain embodiments of the present methods comprise (a) an induction phase consisting of administration of nivolumab by intravenous infusion followed by administration of ipilimumab by intravenous infusion about every 3 weeks for about 4 combination doses, followed by (b) maintenance dosing with nivolumab administered by intravenous infusion about every 2 weeks starting 3 weeks after the 4th dose of induction therapy or after Day 113 if the 4th dose of induction therapy has not been administered due to treatment delays.

In certain embodiments, the anti-PD-1 Ab or antigen-binding portion thereof is administered at a subtherapeutic dose. In certain other embodiments, the anti-CTLA-4 Ab or antigen-binding portion thereof is administered at a subtherapeutic dose. In further embodiments, both the anti-PD-1 Ab or antigen-binding portion thereof and the anti-CTLA-4 Ab or antigen-binding portion thereof are each administered at a subtherapeutic dose.

### Kits

Also within the scope of the present invention are kits comprising an anti-PD-1 Ab and another anti-cancer agent for therapeutic uses. Kits typically include a label indicating the intended use of the contents of the kit and instructions for use. The term label includes any writing, or recorded material supplied on or with the kit, or which otherwise accompanies the kit. Accordingly, this disclosure provides a kit for treating a subject afflicted with a lung cancer, the kit comprising: (a) a dosage ranging from about 0.1 to about 10 mg/kg body weight of an anti-cancer agent which is an Ab or an antigen-binding portion thereof that specifically binds to the PD-1 receptor and inhibits PD-1 activity; (b) a dosage of another anti-cancer agent which is (i) a platinum-based doublet chemotherapy; (ii) an EGFR-targeted tyrosine kinase inhibitor; (iii) bevacizumab; or (iv) a dosage ranging from about 0.1 to about 10 mg/kg body weight of an antibody or an antigen-binding portion thereof that specifically binds to and inhibits CTLA-4; and (c) instructions for using the anti-PD-1 Ab and the other anti-cancer agent in any of the combination therapy methods disclosed herein. In certain embodiments, the anti-PD-1, the anti-CTLA-4 Ab and/or the TKI can be co-packaged in unit dosage form. In certain embodiments for treating human patients, the kit comprises an anti-human PD-1 Ab disclosed herein, *e.g*., nivolumab or pembrolizumab. In other embodiments, the kit comprises an anti-human CTLA-4 Ab disclosed herein, *e.g*., ipilimumab or tremelimumab.

The present invention is further illustrated by the following examples which should not be construed as further limiting. The contents of all references cited throughout this application are expressly incorporated herein by reference.

### EXAMPLE 1

### Treatment of Non-Small Cell Lung Cancer (NSCLC) with Nivolumab in Combination with Platinum-Based Doublet Chemotherapy (PT-DC)

First-line PT-DC has demonstrated 1-yr overall survival (OS) rates of up to 54% in NSCLC; however, there remains a need for therapies with improved long-term overall survival (OS). Results of a phase 1 multi-cohort study (CA209-012; NCT01454102) evaluating nivolumab in combination with PT-DC for first-line treatment of chemotherapy-naïve patients (pts) with advanced NSCLC are reported.

### Methods

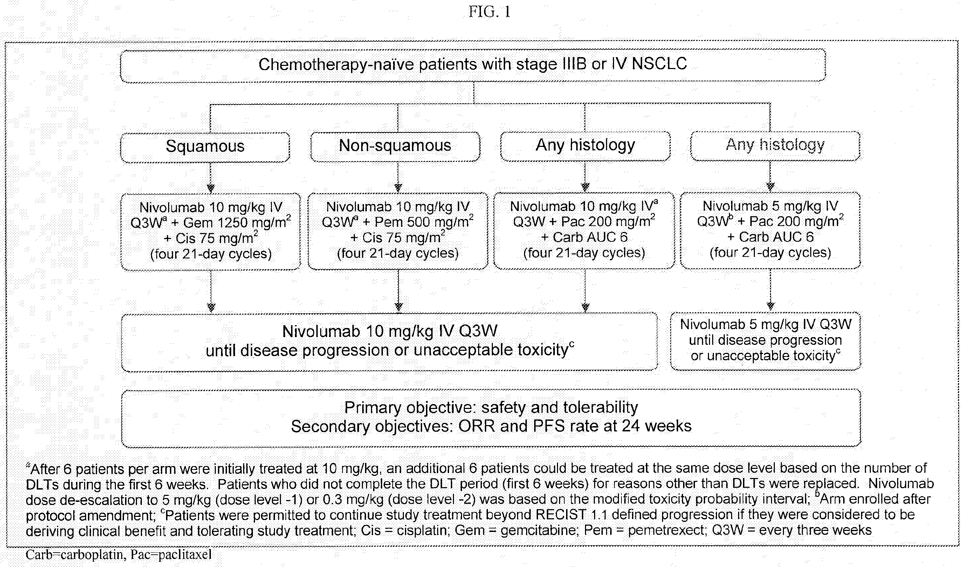

Chemotherapy-naïve pts (N=56) with advanced NSCLC (e.g., stage IIIB or IV NSCLC) were assigned according to histology to 1 of 4 cohorts in a phase 1 dose de-escalation trial (see Figure 1 for the study design). Specifically, Pts received nivolumab 10 mg/kg IV Q3W (N10) plus concurrent IV gemcitabine (gem) 1250 mg/m² + cisplatin (cis) 75 mg/m² (squamous [sq; n=12]) or pemetrexed (pem) 500 mg/m² + cis 75 mg/m² (non-squamous (non-sq); n=15), or nivolumab 10 mg/kg (N10, n=15) or nivolumab 5 mg/kg (N5, n=14) IV Q3W plus IV paclitaxel (pac) 200 mg/m² + carboplatin (carb) AUC6 (sq and non-sq) to assess dose-limiting toxicity (DLT). PT-DC was given for 4 cycles concurrently with nivolumab, followed by nivolumab alone (10 mg/kg or 5 mg/kg, IV Q3W) until progression or unacceptable toxicity. Patients were permitted to continue study treatment beyond Response Evaluation Criteria In Solid Tumors (RECIST) defined progression if they were assessed by the investigator to be deriving clinical benefit and tolerating study treatment. The study used a dose de-escalation design based on the modified toxicity probability interval method, with nivolumab-related dose-limiting toxicities (DLTs) evaluated for the first 2 cycles (6 weeks) of therapy. Addition of the nivolumab 5 mg/kg + paclitaxel (Pac)/carboplatin (Carb) arm was based on a protocol amendment and not based on observed DLT dose de-escalation per protocol.

Efficacy was evaluated using Response Evaluation Criteria In Solid Tumors (RECIST) 1.1 at week 10, week 16, week 22, and every 3 months thereafter until disease progression. Objective response rate (ORR), progression-free survival (PFS) rate, and duration of response (DOR) were calculated by treatment arm and dose level. Time-to-event endpoints were estimated using the Kaplan-Meier method. Adverse events (AEs) were graded according to the National Cancer Institute (NCI) Common Terminology Criteria for Adverse Events (CTCAE) version 4.0. AEs were considered related to study treatment if they were assessed by the investigator to be related to any drug in the combination. High-grade AEs related to chemotherapy were managed with up to 2 dose reductions for chemotherapy before discontinuation of chemotherapy was required. Patients discontinuing chemotherapy for toxicity were permitted to continue study treatment with nivolumab alone. High-grade AEs meeting protocol-specified criteria for nivolumab dose discontinuation required discontinuation of all study treatments.

### Results

### Patient Population and Disposition

This analysis is based on a September 2014 database lock. A total of 56 patients were treated across four arms (Table 1). Overall median follow-up time was 82.6 weeks (range 13.9, 152.6 weeks). Across arms, median follow-up time ranged from 50.5 to 114.9 weeks. At the time of analysis, 53 patients (95%) had discontinued study treatment: 39 (70%) discontinued due to disease progression; 12 (21%) discontinued due to AEs related to any study medication; the remaining 2 patients discontinued due to maximum clinical benefit (n = 1) and the treating medical oncologist electing to withdraw the patient from study treatment (n = 1).

**Table 1. Baseline characteristics of NSCLC patients treated with nivolumab plus platinum-based chemotherapy**

| | Nivolumab 10 mg/kg | | | Nivolumab 5 mg/kg | All Pac/Carb with Nivo | |
|---|---|---|---|---|---|---|
| | Gem/Cis n = 12 | Pem/Cis n = 15 | Pac/Carb n = 15 | Pac/Carb n = 14 | n = 29 | Total N = 56 |
| Median age, years (range) | 67 (49-76) | 60 (34-78) | 58 (34-69) | 64 (47-83) | 62 (34-83) | 63.5 (34-83) |
| Gender, n (%) | | | | | | |
| Male | 7 (58) | 6 (40) | 7 (47) | 6 (43) | 13 (45) | 26 (46) |
| Female | 5 (42) | 9 (60) | 8 (53) | 8 (57) | 16 (55) | 30 (54) |
| Disease stage, n (%) | | | | | | |
| Stage IIIB | 1 (8) | 0 | 0 | 1 (7) | 1 (3) | 2 (4) |
| Stage IV | 11 (92) | 15 (100) | 15 (100) | 13 (93) | 28 (97) | 54 (96) |
| Histology, n (%) | | | | | | |
| Adenocarcinoma | 0 | 15 (100) | 10 (67) | 12 (86) | 22 (76) | 37 (66) |
| Squamous cell carcinoma | 12 (100) | 0 | 3 (20) | 1 (7) | 4 (14) | 16 (29) |
| Large cell carcinoma | 0 | 0 | 0 | 1 (7) | 1 (3) | 1 (2) |
| Other | 0 | 0 | 2 (13) | 0 | 2 (7) | 2 (4) |
| EGFR mutation status,^{a} n(%) | | | | | | |
| Positive | 0 | 4 (27) | 1 (7) | 1 (7) | 2 (7) | 6 (11) |
| Negative | 2 (17) | 10 (67) : | 10 (67) | 12 (86) | 22 (76) | 34 (61) |
| Unknown | 10 (83) | 1 (7) | 4 (27) | 1 (7) | 5 (17) | 16 (29) |
| Prior treatment with erlotinib, n (%) | 0 | 2 (13) | 1 (7) | 0 | 1 (3) | 3 (5) |

| | | | | | | |
|---|---|---|---|---|---|---|
| ^{a}Site-determined EGFR mutation test EGFR = epidermal growth factor receptor | | | | | | |

### Efficacy

Tumor response rates are summarized in Table 2. Across treatment arms, ORRs ranged from 33--47% and were observed in both squamous and non-squamous patients. ORR was similar between the two nivolumab + Pac/Carb arms. Progressive disease (PD) as best overall response was infrequent (0, 0, 27%, and 7% with nivolumab 10 mg/kg + Gem/Cis, nivolumab 10 mg/kg + Pem/Cis, nivolumab 10 mg/kg + Pac/Carb, and nivolumab 5 mg/kg + Pac/Carb, respectively). One patient in the nivolumab 5 mg/kg + Pac/Carb arm exhibited an unconventional "immune-related" response, with a 59% reduction in target lesions in the presence of a new lesion (immune-related response criteria [irRC] not used).

**Table 2. Tumor response in NSCLC patients treated with nivolumab plus platinum-based chemotherapy**

| | Nivolumab (10 mg/kg) | | | Nivolumab 5 mg/kg |
|---|---|---|---|---|
| | Gem/Cis (n = 12) | Pem/Cis (n = 15) | Pac/Carb (n = 15) | Pac/Carb (n = 14) |
| ORR, n (%) [95% CI] | 4 (33) [10, 65] | 7 (47) [21, 73] | 7 (47) [21, 73] | 6 (43) [18, 71] |
| Ongoing responders, n (%) | 1 (25) | 2 (29) | 3 (43) | 4 (67) |
| Best overall response, n (%) | | | | |
| Complete response^{a} | 1 (8) | 0 | 0 | 0 |
| Partial response^{a} | 3 (25) | 7 (47) | 7 (47) | 6 (43) |
| Stable disease | 7 (80) | 7 (47) | 4 (27) | 6 (43) |
| Stable disease (≥ 21 weeks) | 1 (8) | 5 (33) | 1 (7) | 1 (7) |
| Progressive disease | 0 | 0 | 4 (27) | 1 (7) |
| Unable to determine | 1 (8) | 1 (7) | 0 | 1 (7) |
| Confirmed/Overall ORR [95% CI] | 4 (33) [10, 65] | 7 (47) [21, 73] | 7 (47) [21, 72] | 6(43) [18, 71] |
| Confirmed DCR [95% CI] | 11 (92) [62, 100] | 14 (93) [68,100] | 11(73) [45, 92] | 12 (86) [57, 98] |

| | | | | |
|---|---|---|---|---|
| ^{a}All complete and partial responses were confirmed by a subsequent tumor assessment per RECIST 1.1 ^{b}Time from first response to documented progression, death within 100 days of last nivolumab dose, or last tumor assessment (for censored + data). Estimated median DORs were determined from Kaplan-Meier curves + = response ongoing; CI = confidence interval; NR = not reached | | | | |

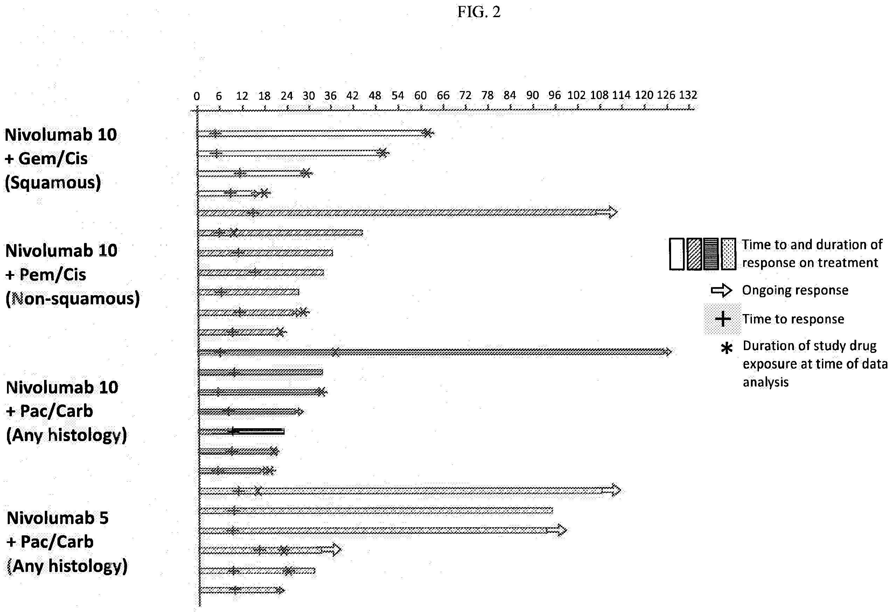

Characteristics of ORs are shown in Figure 2. Responses by first tumor assessment (10 weeks) were observed in 3/4, 3/7, 7/7, and 4/6 patients in the nivolumab 10 mg/kg + Gem/Cis, nivolumab 10 mg/kg + Pem/Cis, nivolumab 10 mg/kg + Pac/Carb, and nivolumab 5 mg/kg + Pac/Carb arms, respectively. 17 of the 24 responders (71%) showed a response at first tumor assessment. For the 6 responding patients who discontinued therapy for reasons other than disease progression and continued to respond off therapy, durations of therapy and response are shown in Table 3.

**Table 3¹. Durations of therapy and response in patients discontinuing therapy for reasons other than disease progression**

| Patient | Duration of therapy (weeks) | Time to response (weeks) | Total duration of response (weeks) | Duration of response off therapy (weeks) |
|---|---|---|---|---|
| 1 | 9.7 | 5.9 | 38.4 | 34.6 |
| 2 | 15.9 | 10.6 | 65.1+ | 59.8+ |
| 3 | 20.0 | 8.9 | 12.3 | 1.2 |
| 4 | 22.7 | 16.1 | 16.9+ | 10.3+ |
| 5 | 24.0 | 9.1 | 22.0 | 7.1 |
| 6 | 36.9 | 5.9 | 92.1+ | 61.1+ |

| | | | | |
|---|---|---|---|---|
| + = ongoing response ¹ as of a March 2014 database lock. | | | | |

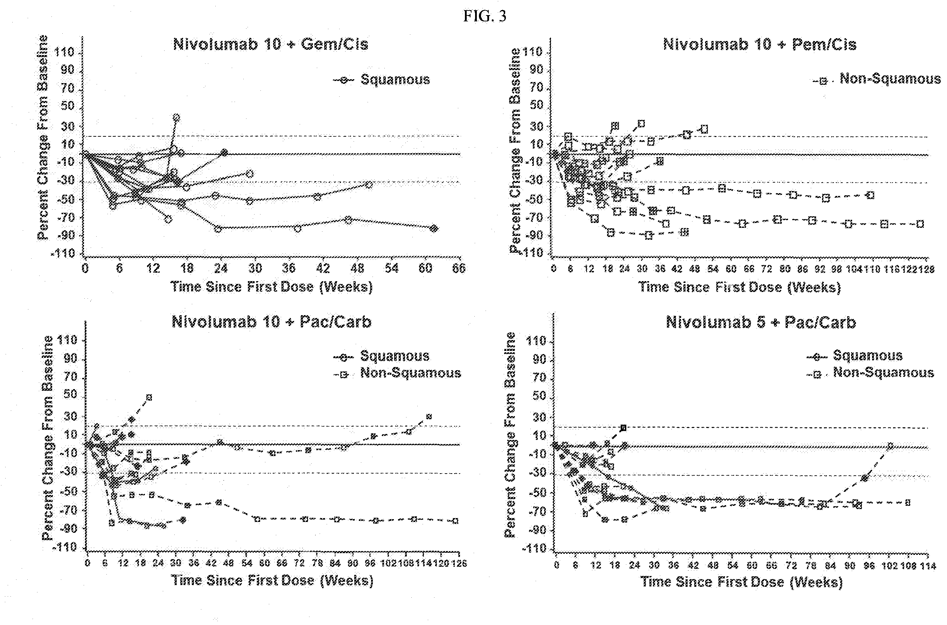

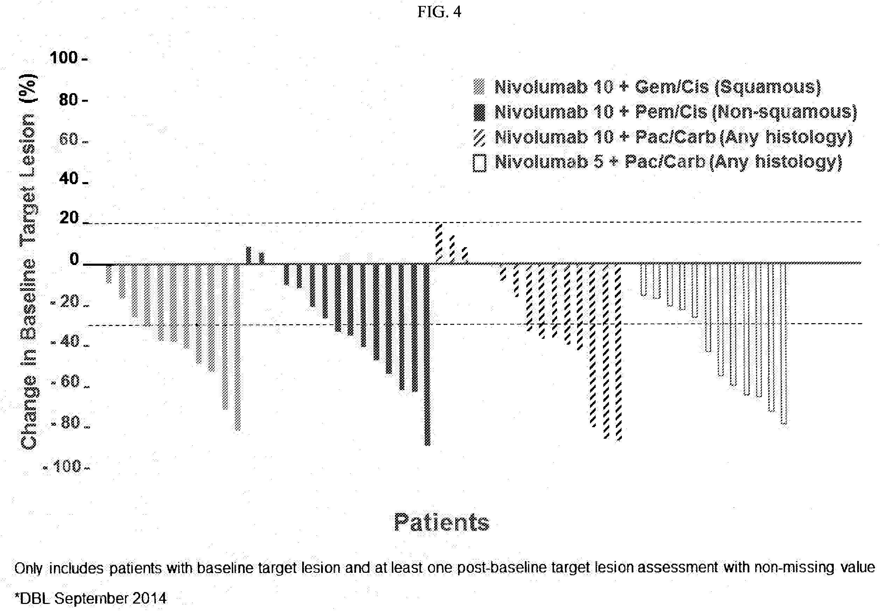

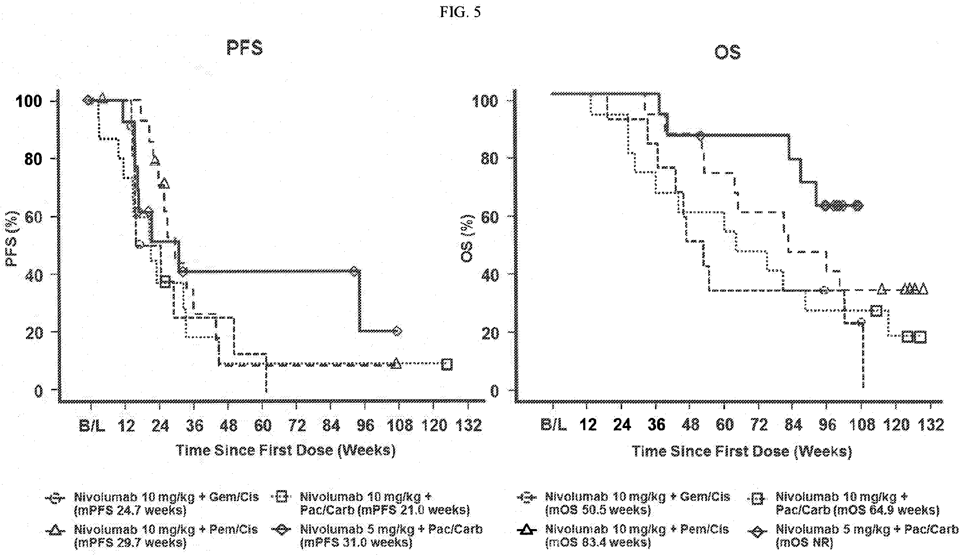

Percent change in target lesions from baseline over time is shown for each treatment arm in Figure 3. Best percent change in target lesion tumor burden is shown in Figure 4. By week 18, one patient in the nivolumab 10 mg/kg + Pem/Cis arm and one patient in the nivolumab 10 mg/kg + Pac/Carb arm had a tumor burden reduction of >80%. PFS and OS outcomes across treatment arms are shown in Table 4 and Figure 5. PFS rates at 24 weeks ranged from 38-71%, with median PFS 21.0-31.0 weeks across treatment arms. One-year OS rates ranged from 50-87%. 18-month OS rates ranged from 33-86%. 24-month OS rates ranged from 25-62% Median OS was NR in the nivolumab 5 mg/kg + Pac/Carb arm.

**Table 4. Survival outcomes in NSCLC patients treated with nivolumab plus platinum-based chemotherapy**

| | | Nivolumab | | Nivolumab |
|---|---|---|---|---|
| | 10 mg/kg | | | 5 mg/kg |
| | Gem/Cis (n = 12) | Pem/Cis (n = 15) | Pac/Carb (n = 15) | Pac/Carb (n = 14) |
| Median DOR (weeks) Min, Max | 45 (6+, 57) | 25.4 (13, 92+) | 23.9 (11+, 119+) | 85.4 (11.4+, 98+) |

| PFS | | | | |
|---|---|---|---|---|
| PFS rate at 24 weeks, % (95% CI) | 51 (19, 76) | 71 (39, 88) | 38 (14, 61) | 51 (21, 75) |
| Median PFS, weeks (range) (95% CI) | 24.7 (14.9, 50) | 29.7 (22.1, 44.1) | 21.0 (9.7, 33.3) | 31.0 (16.0,-) |

| OS | | | | |
|---|---|---|---|---|
| 1-year OS rate, % (95% CI) | 50 (21, 74) | 87 (56, 96) | 60 (32, 80) | 86 (54, 96) |
| 18-month OS rate, % (95% CI) | 33 (10, 59) | 60 (32, 60) | 40 (16, 63) | 86 (54, 96) |
| 24 month OS rate, % (95% CI) | 25 (6, 50) | 33 (12, 56) | 27 (8, 50) | 62 (32, 82) |
| Median OS, months (range) (95% CI) | 11.6 (7.9, 25.2) | 19.2 (12.3, 33.2) | 14.9 (6.2, 20.6) | NR |

### Safety

No DLTs were observed during the first 6 weeks of treatment. Overall, treatment-related AEs of any grade occurred in 93% of patients across all four treatment arms (Table 5). The most common treatment-related AEs (observed in >30% of patients), including fatigue, nausea, decreased appetite, and alopecia, are commonly associated with chemotherapy. Treatment-related grade 3/4 AEs were reported in 45% of patients (Table 5). Overall, the most common treatment-related grade 3/4 AEs were pneumonitis (4 patients, 7%; managed by protocol algorithm), fatigue, and acute renal failure (3 patients each, 5%). The nivolumab 10 mg/kg + Pac/Carb arm had the highest frequency of treatment-related grade 3/4 AEs (11 patients, 73%); many of these were laboratory abnormalities (hemoglobin decreased, lipase increased, neutropenia, neutrophil count decreased, white blood cell count decreased) reported in 1 patient each. Across treatment arms, treatment-related pneumonitis of any grade was reported in 7 patients (13%), with grade 3/4 in 4 patients (7%). The most frequently observed categories of select AEs (defined as AEs with potential immunologic etiologies that require more frequent monitoring and/or unique intervention) were skin (34% any grade; 5% grade 3/4), gastrointestinal (23% any grade; 4% grade 3/4), infusion reaction (23% any grade; 2% grade 3/4), renal (14% any grade; 5% grade 3/4), and pulmonary (13% any grade; 7% grade 3/4).

**Table 5. Treatment-related AEs reported in ≥15% of all NSCLC patients treated with nivolumab plus platinum-based chemotherapy**

| | Nivolumab 10 mg/kg | | | Nivolumab 5 mg/kg | | |
|---|---|---|---|---|---|---|
| | Gem/Cis n = 12 | Pem/Cis n = 15 | Pac/Carb n = 15 | Pac/Carb n = 14 | Total N = 56 | |
| Treatment-related AE,^{a} n (%) | Grade 3/4 | Grade 3/4 | Grade 3/4 | Grade 3/4 | All Grades | Grade 3/4 |
| Patients with any AE | 3 (25) | 7 (47) | 11 (73) | 4 (29) | 52 (93) | 25 (45) |
| Fatigue | 0 | 1 (7) | 2 (13) | 0 | 40 (71) | 3 (5) |
| Nausea | 0 | 1 (7) | 0 | 0 | 26 (46) | 1 (2) |
| Decreased appetite | 0 | 0 | 1 (7) | 0 | 20 (36) | 1 (2) |
| Alopecia | 0 | 0 | 0 | 0 | 17 (30) | 0 |
| Anemia | 1 (8) | 0 | 1 (7) | 0 | 15 (27) | 2 (4) |
| Rash | 0 | 0 | 1 (7) | 1 (7) | 14 (25) | 2 (4) |
| Diarrhea | 0 | 0 | 1 (7) | 0 | 12 (21) | 1 (2) |
| Arthralgia | 0 | 0 | 0 | 0 | 11 (20) | 0 |
| Constipation | 0 | 0 | 0 | 0 | 11 (20) | 0 |
| Peripheral neuropathy | 0 | 0 | 0 | 0 | 10 (18) | 0 |

| | | | | | | |
|---|---|---|---|---|---|---|
| ^{a}Treatment-related AEs (any grade) in any treated patients. Treatment-related AEs occurring in 10-15% of all patients were: dysgeusia (14%), hypersensitivity (14%; 1 grade 3/4 event), vomiting (14%), mucosal inflammation (13%), myalgia (13%), pneumonitis (13%; see text), infusion-related reaction (11%), leukopenia (11%), lymphopenia (11%), peripheral sensory neuropathy (11%), and pruritus (11%). All were grades 1/2 unless stated | | | | | | |

### Conclusions

In chemotherapy-naïve patients with advanced NSCLC, first-line treatment with nivolumab in combination with different platinum-based doublet chemotherapy regimens showed antitumor activity similar to that previously reported for standard platinum-based doublet chemotherapy regimens. These results suggest that nivolumab plus platinum-based doublet chemotherapy was associated with a safety profile reflecting additive toxicities of nivolumab and chemotherapy.

### EXAMPLE 2

### Treatment of Patients with Epidermal Growth Factor Receptor Mutant (EGFR MT) NSCLC Using Nivolumab in Combination with Erlotinib

Preclinical studies demonstrate EGFR signaling in EGFR MT NSCLC leads to upregulation of tumor PD-1 ligand 1 (PD-L1) and suppression of antitumor immunity. Treatment with an anti-PD-1 antibody in an EGFR MT murine NSCLC model relieved immune inhibition, reducing tumor growth and promoting tumor cell apoptosis. The EGFR tyrosine kinase inhibitor (TKI) erlotinib is FDA-approved for the first-line treatment of EGFR MT NSCLC, yielding a median progression-free survival (PFS) of 10.4 months. Interim results from a phase 1 study (CA209-003; NCT00730639) evaluating the safety and activity of nivolumab in combination with erlotinib in an EGFR MT, chemotherapy-naïve, advanced NSCLC cohort are reported herein.

### Methods

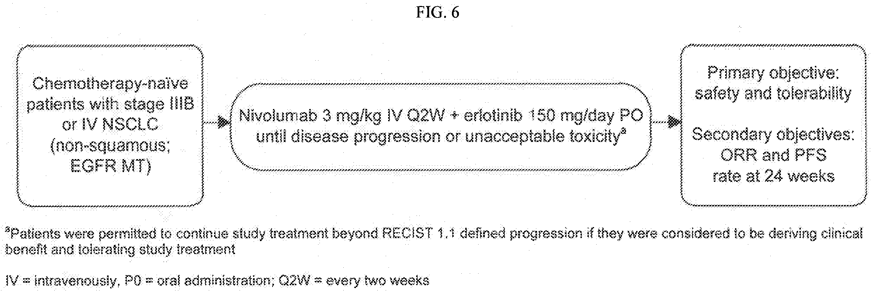

Chemotherapy-naïve patients with stage IIIB or IV NSCLC received nivolumab 3 mg/kg IV Q2W in combination with erlotinib 150 mg PO daily until progression or unacceptable toxicity (see Figure 6 for the study design). Patients had non-squamous histology and EGFR MT NSCLC (based on site-determined EGFR mutation test). Prior use of EGFR TKIs was allowed. Efficacy was evaluated using Response Evaluation Criteria In Solid Tumors (RECIST) 1.1 at week 11, week 17, week 23, and every 3 months thereafter until disease progression. Time-to-event endpoints were estimated using the Kaplan-Meier method. Adverse events (AEs) were graded according to the National Cancer Institute (NCI) Common Terminology Criteria for Adverse Events (CTCAE) version 4.0. AEs were considered related to study treatment if they were assessed by the investigator to be related to any drug in the combination. High-grade AEs meeting protocol-specified criteria for nivolumab dose discontinuation required discontinuation of all study treatments. Patients could continue in the study on nivolumab monotherapy after discontinuation of erlotinib due to erlotinib-related toxicity. Dose modifications for erlotinib were based on those recommended in the label.

### Results

### Patient Population and Disposition

This analysis is based on a September 2014 database lock. Baseline characteristics of the 21 patients treated with nivolumab and erlotinib are shown in Table 6.

**Table 6. Baseline characteristics of NSCLC patients treated with nivolumab plus erlotinib**

| | Nivolumab + erlotinib (N = 21) |
|---|---|
| Median age, years (range) | 63.0 (41-80) |
| Gender, n (%) | |
| Male | 8 (38) |
| Female | 13 (62) |
| Disease stage, n (%) | |
| Stage IIIB | 1(5) |
| Stage IV | 20 (95) |
| Histology, n (%) | |
| Adenocarcinoma | 20 (95%) |
| Squamous cell carcinoma | 0 |
| Large cell carcinoma | 0 |
| Other | 1 (5%) |
| EGFR mutation status,^{a} n (%) | |
| Positive | 21 (100) |
| Exon 19 deletion | 12 (57) |
| L858R | 9 (43) |
| T790M^{b} | 7 (33) |
| S768I^{b} | 1 (5) |
| Prior treatment with erlotinib, n (%) | 20 (95)^{c} |
| Prior systematic therapy (more than 1 sitting per subject may be reflected in this frequency) | 20 (95%) |
| Adjuvant | 0 |
| Neoadjuvant | 1 (5%) |
| Metastatic | 19 (91%) |
| Other agent | 6 (29%) |

| | |
|---|---|
| ^{a}Site-determined EGFR mutation test ^{b}Second mutations ^{c}One patient included in this subgroup of 20 patients had prior erlotinib treatment that was not reported at the time of data analysis | |

All patients had EGFR MT NSCLC. One patient had not received prior treatment with erlotinib. Median follow-up time was 83.7 weeks (range 10.7, 110.3 weeks). At the time of analysis, 17 patients (81%) had discontinued study treatment: 12 (57%) discontinued due to disease progression; 4 (19%) discontinued treatment due to AEs related to any study medication; and 1 patient discontinued due to poor/non-compliance. Seven patients discontinued erlotinib and continued nivolumab alone.

### Efficacy

Tumor responses are shown in Table 7. ORR was 19% (median duration of response [DOR] 70 weeks; range 60.1, 83.7+ weeks). SD as best overall response was observed in 9 patients (43%). Of the 20 patients with acquired erlotinib resistance, 3 (15%) achieved partial response (Table 7). Nine patients (45%) had SD as best overall response. Seven patients had SD for ≥21 weeks. One patient exhibited an unconventional "immune-related" response (ongoing; immunerelated response criteria [irRC] not used), with a 50% reduction in target lesions after progression in non-target lesions (included here as having progressive disease).

**Table 7. Tumor response in NSCLC patients treated with nivolumab plus erlotinib**

| | Prior treatment with erlotinib (n = 20) | No prior treatment with erlotinib (n = 1) | EGFR Mutant (n=7) |
|---|---|---|---|
| Confirmed ORR, n (%) [95% CI] | 4 (19) [5, 42] | | 1 (14) [0.4, 58] |
| Ongoing responders, n (%) | 2 (10) | 1 (100) | |
| Best overall response, n (%) | | | |
| Complete response" | 0 | 0 | 0 |
| Partial response^{a} | 3 (15) | 1 (100) | 1(14) |
| SD | 9 (45) | 0 | 1 (14) |
| SD (≥ 21 weeks) | 7 (33) | - | 1 (14) |
| Progressive disease^{b} | 8 (38) | 0 | 4 (57) |
| Unable to determine | 0 | 0 | |
| Median DOR,^{c} weeks (mix, max) | 70 (60.1, 83.7+) | | NR (7.6+, 7.6+) |

| | | | |
|---|---|---|---|
| ^{a}All complete and partial responses were confirmed by a subsequent tumor assessment per RECIST 1.1 ^{b}One patient with progressive disease has a persistent unconventional response with 50% reduction in target lesions after initial minimal growth in nontarget lesions. The one patient with response who progressed continues on therapy after the site of progression (new supraclavicular lymph node) was removed ^{c}Time from first response to documented progression, death within 100 days of last nivolumab dose, or last tumor assessment (for censored + data). Estimated median DORs were determined from Kaplan-Meier curves + = response ongoing; CI = confidence interval; NR = not reached | | | |

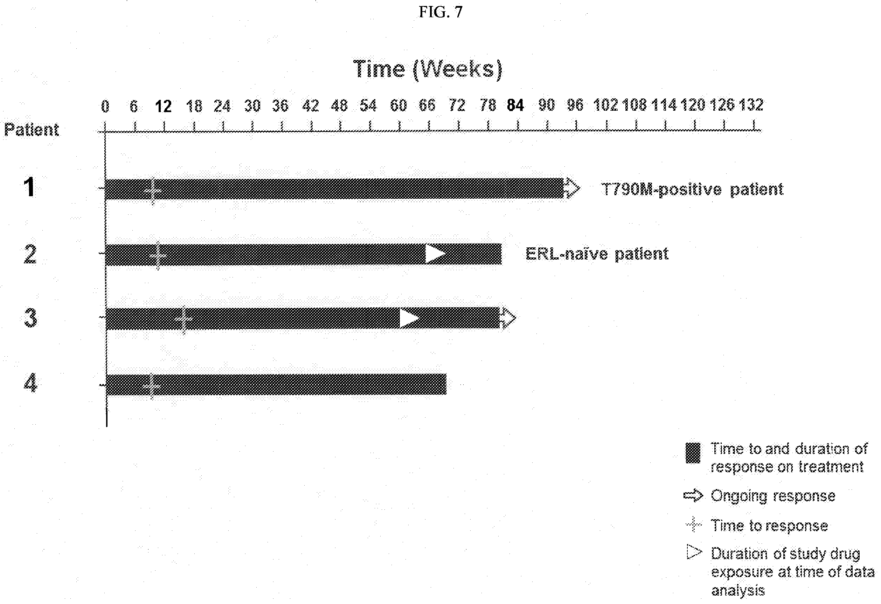

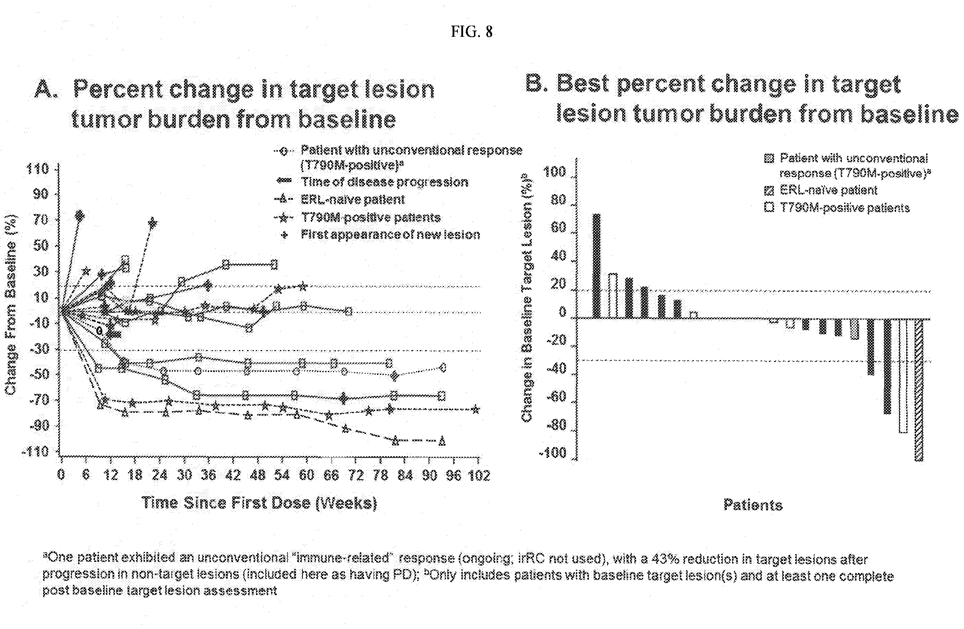

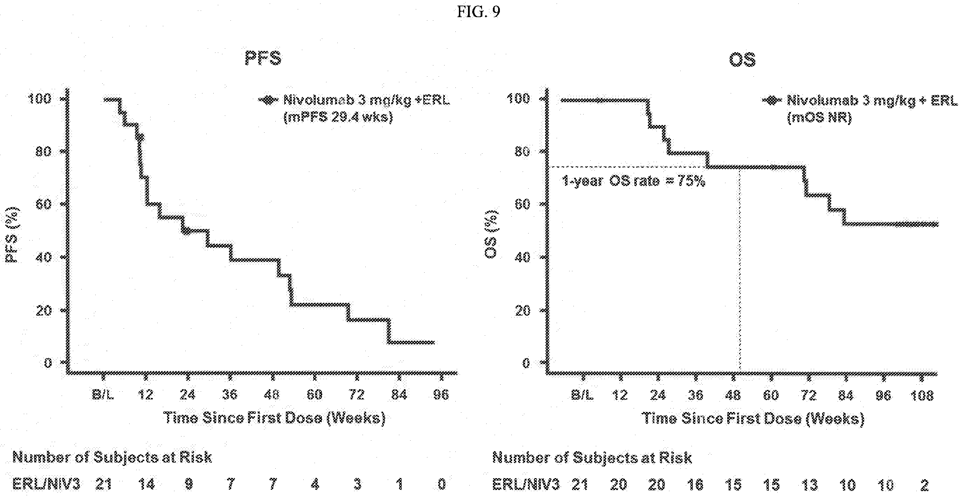

Characteristics of responding patients are shown in Table 8 and Figure 7. Responses were seen in patients who had previously received multiple lines of EGFR inhibitor therapy, as well as in patients with the T790M mutation. Responses were ongoing in 2 patients at the time of analysis. Responses by first tumor assessment (week 11) were observed in 3 responders (75%). Percent change in target lesion tumor burden from baseline is shown over time and according to best percent change in target lesion tumor burden from baseline (Figure 8). PFS and overall survival (OS) outcomes are shown in Table 9 and Figure 9. PFS rate at 24 weeks was 50%; median PFS was 29.4 weeks. 1-year OS rate was 73%. 18-month OS rate was 60%. 24 month OS rate was 45%. Median OS was 19.3 months.

**Table 8. Characteristics of patients responding to nivolumab plus erlotinib**

| Patient | EGFR MT | PFS (weeks) | | Re-biopsy | Erlotinib + nivolumab | |
|---|---|---|---|---|---|---|
| | | Prior erlotinib | Prior afatinib/ cetuximab | | Treatment duration (weeks) | PFS (weeks) |
| 1 | DEL19 | 90 | n/a | T790M⁺ | 66⁺ | 80⁺ |
| 2 | DEL19 | 40 | 15 | T790M- | 61 | 80⁺ |
| 3 | L858R | 26 | n/a | T790M | 108+⁺ | 69 |
| 4^{a} | L858R/S768I | n/a | n/a | n/a | 104⁺ | 93⁺ |
| 5^{b} | L858R | 55 | 34 | T790M⁺ | 104⁺ | 9 |

| | | | | | | |
|---|---|---|---|---|---|---|
| ^{a}Erlotinib-naïve patient ^{b}One patient exhibited an unconventional "immune-related" response (ongoing; irRC not used), with a 50% reduction in target lesions after progression in non-target lesions (included here as having progressive disease) + = ongoing | | | | | | |

**Table 9. Survival outcomes in NSCLC patients treated with nivolumab plus erlotinib**

| | Nivolumab + erlotinib (N = 21) | EGFR Mutant (N=7) |
|---|---|---|
| PFS | | |
| PFS rate at 24 weeks, % (95% CI) | 50 (27, 70) | 0 (4,61) |
| Median PFS, weeks (range) | 29.4 (10.6, 52.7) | 7.9 (1.0, 21.9) |

| OS | | |
|---|---|---|
| 1-year OS rate, % (95% CI) | 75 (51, 89) | 71 (26, 92) |
| 18-month OS rate (95% CI) | 60 (36, 78) | NC |
| 24-month OS rate (95% CI) | 45 (23, 65) | NC |
| Median OS, months (95% CI) | 19.3 (15.6, --) | NR |

| | | |
|---|---|---|
| Note: OS rates from March 2015 database lock. | | |

### Safety

Treatment-related AEs were reported in all 21 patients (Table 10). The most common treatment-related AEs were rash, fatigue, paronychia, diarrhea, and skin fissures. Most patients only reported grade 1 or 2 AEs (16/21 patients, 76%). No pneumonitis (any grade) was observed. Treatment-related grade 3 AEs occurred in 24% of patients (no grade 4 AEs reported). These were increased aspartate aminotransferase (AST; n = 2), diarrhea (n = 2), increased alanine aminotransferase (ALT; n = 1), and weight decrease (n = 1). Treatment-related select AEs (defined as AEs with potential immunologic etiologies that require more frequent monitoring and/or unique intervention) were mostly reported in the skin (71% any grade; 0 grade 3/4), gastrointestinal (24% any grade; 10% grade 3/4), endocrinopathies (19% any grade; 0 grade 3/4), and hepatic (14% any grade; 10% grade 3/4) categories.

Treatment-related AEs led to discontinuation of any study medication in 4 patients (19%), with grade 3/4 events in 3 patients (14%). Individual AEs were diarrhea (n = 2, both grade 3/4), increased AST (n = 1, grade 3/4), and flushing and tubulointerstitial nephritis (n = 1 each, grade 1/2). One patient had both grade 3 diarrhea and grade 2 flushing. At the time of analysis, 8 patients had died (all due to disease progression).

**Table 10. Treatment-related AEs reported in ≥10% of NSCLC patients treated with nivolumab plus erlotinib**

| | Nivolumab + erlotinib (N = 21) | |
|---|---|---|
| Treatment-related AE, n (%) | All Grades | Grade 3/4 |
| Patients with any AE | 21 (100) | 5 (24) |
| Rash | 10 (48) | 0 |
| Fatigue | 6 (29) | 0 |
| Paronychia | 6 (29) | 0 |
| Diarrhea | 4 (19) | 2 (10) |
| Skin fissures | 5 (24) | 0 |
| Dry skin | 4 (19) | 0 |
| Nausea | 4 (19) | 0 |
| Increased ALT | 3 (14) | 1 (5) |
| Alopecia | 3 (14) | 0 |
| Increased AST | 3 (14) | 2 (10) |
| Dry mouth | 3 (14) | 0 |
| Hypothyroidism | 3 (14) | 0 |
| Nail disorder | 3 (14) | 0 |
| Pruritus | 3 (14) | 0 |
| Vomiting | 3 (14) | 0 |

### Conclusions

In chemotherapy-naïve patients with advanced EGFR MT NSCLC with acquired resistance to an EGFR TKI, treatment with nivolumab in combination with erlotinib can provide durable clinical benefit. Data on a single erlotinib-naïve patient suggest that the combination of nivolumab and erlotinib is feasible as first-line therapy for patients with EGFR MT advanced NSCLC. These interim results show that nivolumab in combination with erlotinib was associated with a manageable safety profile, reflecting additive toxicities of both agents.

### EXAMPLE 3

### Treatment of Chemotherapy-Pretreated NSCLC Patients with Nivolumab Maintenance as Monotherapy or in Combination with Bevacizumab (BEV)

Nivolumab has demonstrated durable responses and tolerability in chemotherapy-naive and heavily pretreated patients (pts) with NSCLC. BEV has shown tolerability and enhanced activity in combination with the CTLA-4 immune checkpoint antibody ipilimumab in melanoma. Interim results from a phase 1 study evaluating the safety and efficacy of switching to nivolumab maintenance therapy, as monotherapy or combined with BEV, in pts with advanced NSCLC who responded or had stable disease on first-line platinum (PT)-based chemotherapy are reported.

### Methods

Pts with no progression within 42 days of completing ≥ 4 cycles first-line PT-based chemotherapy (± BEV) were assigned to either nivolumab (N) 5 mg/kg TV Q3W + BEV 15 mg/kg IV Q3W (non-squamous [non-sq] pts only; n=12) or N 3 mg/kg IV Q2W monotherapy (sq arm [n=8] or non-sq arm [n=13]) until progression/unacceptable toxicity (treatment beyond progression allowed based on protocol-defined criteria). ORR was evaluated using RECIST 1.1.

### Results

Median follow-up was 80.4, 25.6, and 35.1 weeks for N + BEV, N monotherapy in sq pts, and N monotherapy in non-sq pts, respectively. Any-grade treatment-related adverse events (AEs) occurred in 11/12 (92%) pts switching to N + BEV and in 13/21 (62%) pts switching to N monotherapy (managed with standard algorithms). No treatment-related grade 4 AEs were reported; grade 3 AEs were pneumonitis (n=2) and cough and tubulointerstitial nephritis (n=1 each) with N + BEV; and pneumonitis (n=2) and pleural effusion (n=1) with N monotherapy. Four pts each in the N + BEV and N monotherapy (non-sq) arms discontinued due to treatment-related AEs. ORR with N + BEV was 8% (1/12, ongoing; duration 11.1+ weeks). ORR with N monotherapy was 10% (2/21; both non-sq, range 15.7, 18.3 weeks). Median progression-free survival (PFS) was 37.1 weeks with N + BEV, 16.0 weeks with sq N monotherapy and 21.4 weeks with non-sq N monotherapy. Stable disease was best overall response in 50% with N + BEV and 48% with N monotherapy (50% sq, 46% non-sq). Median OS was not reached with N + BEV (range 33.3, 86.7+ weeks) or N monotherapy (range 2.1+, 56.3+ weeks); 1-yr OS rate was 75% with N + BEV (not calculated for N monotherapy [insufficient follow-up]).

### Conclusions

Switching to nivolumab either as monotherapy or + BEV demonstrated PFS similar to that seen with other agents approved as maintenance therapy after PT-based chemotherapy in advanced NSCLC. These preliminary data support further evaluation of the safety and efficacy of switching to nivolumab maintenance therapy in chemotherapy-treated pts.

### EXAMPLE 4

### Treatment of NSCLC with Nivolumab in Combination with Ipilimumab

Nivolumab (anti-PD-1) and ipilimumab (anti-CTLA-4) have each shown activity in advanced NSCLC, and clinical data in melanoma have shown improved responses, promising 2-year survival rates and a manageable safety profile with blockade of the PD-1 and CTLA-4 immune checkpoint pathways by combined use of nivolumab and ipilimumab (Wolchok *et al., 2013).* The present phase I study evaluated the safety and efficacy of first-line nivolumab plus ipilimumab (N + I) in chemotherapy-naive patients (pts) with advanced NSCLC.

### Methods

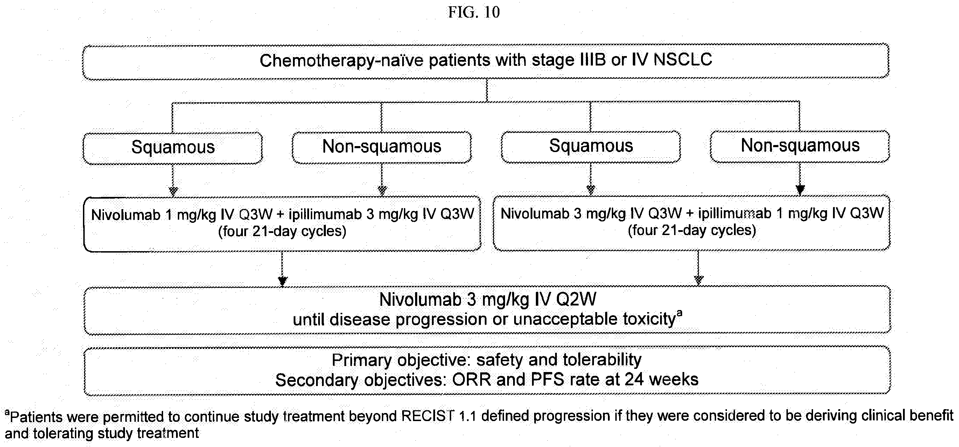

Chemotherapy-naïve patients with stage IIIB or IV NSCLC were assigned based on histology to one of four arms at different combination doses (see Figure 10 for the study design). Specifically, patients (n=49) received N + I at either the 1 + 3 mg/kg or 3 + 1 mg/kg combination dose IV Q3W for 4 cycles (one squamous [sq] and one non-sq cohort per dose level), followed by N 3 mg/kg IV Q2W until progression/unacceptable toxicity. Combination drug doses were based on safety and activity data from the phase 1 CA209-004 study of nivolumab in combination with ipilimumab in patients with advanced melanoma (Wolchok *et al., 2013*). After a protocol amendment, a 1 + 1 mg/kg dose combination cohort was enrolled (n = 31; data immature at time of this analysis). Efficacy was evaluated using Response Evaluation Criteria In Solid Tumors (RECIST) 1.1 at week 10, week 17, week 23, and every 3 months thereafter until disease progression. Time-to-event endpoints were estimated using the Kaplan-Meier method. Adverse events (AEs) were graded according to the National Cancer Institute (NCI) Common Terminology Criteria for Adverse Events (CTCAE) version 4.0. AEs were considered related to study treatment if they were assessed by the investigator to be related to any drug in the combination. No intra-patient dose reductions of ipilimumab or nivolumab were allowed. Immunotherapy-related AEs with nivolumab and ipilimumab were managed with protocol safety guidelines.

### Results

### Patient Population and Disposition

This analysis is based on a March 2014 database lock. A total of 49 patients were treated across four arms. Baseline characteristics are shown in Table 11. Patient disposition is shown in Table 12.

**Table 11. Baseline characteristics of NSCLC patients treated with nivolumab plus ipilimumab**

| | Nivolumab 1 mg/kg + ipilimumab 3 mg/kg | | Nivolumab 3 mg/kg + ipilimumab 1 mg/kg | | |
|---|---|---|---|---|---|
| | Squamous (n = 9) | Non-squamous (n = 15) | Squamous (n = 9) | Non-squamous (n = 16) | Total (N=49) |
| Median age, years (range) | 62 (51-66) | 61 (41-74) | 60 (56-72) | 61 (53-78) | 61 (41-78) |

| Gender, n (%) | | | | | |
|---|---|---|---|---|---|
| Male | 8 (89) | 6 (40) | 8 (89) | 5 (31) | 27 (55) |
| Female | 1 (11) | 9 (60) | 1 (11) | 11 (69) | 22 (45) |

| Disease stage, n (%) | | | | | |
|---|---|---|---|---|---|
| Stage IIIB | 1 (11) | 0 | 1 (11) | 2 (13) | 4(8) |
| Stage IV | 8 (89) | 15 (100) | 8 (89) | 14 (88) | 45 (92) |

| Histology, n (%) | | | | | |
|---|---|---|---|---|---|
| Adenocarcinoma | 0 | 14 (93) | 0 | 16 (100) | 30 (61) |
| Squamous cell carcinoma | 9 (100) | 0 | 9 (100) | 0 | 18 (37) |
| Large cell carcinoma | 0 | 0 | 0 | 0 | 0 |
| Other | 0 | 1 (7) | 0 | 0 | 1 (2) |

| EGFR mutation status,^{a} n (%) | | | | | |
|---|---|---|---|---|---|
| Positive | 0 | 2 (13) | 0 | 5 (31) | 7 (14) |
| Unknown | 3 (33) | 12 (80) | 3 (33) | 11 (69) | 29 (59) |
| Negative | 6 (67) | 1 (7) | 6 (67) | 0 | 13 (27) |
| : Prior treatment with erlotinib, n (%) | 0 | 1 (7) | 0 | 5 (31) | 6 (12) |

| | | | | | |
|---|---|---|---|---|---|
| ^{a}Site-determined EGFR mutation test EGFR = epidermal growth factor receptor | | | | | |

**Table 12. Patient disposition summary**

| | Nivolumab 1 mg/kg + ipilimumab 3 mg/kg | | Nivolumab 3 mg/kg + ipilimumab 1 mg/kg | | |
|---|---|---|---|---|---|
| | Squamous (n + 9) | Non-squamous (n = 15) | Squamous (n = 9) | Non-squamous (n = 16) | Total (N = 49) |
| Median follow-up time, weeks (range) | 32.3 (1.4, 53.1) | 43.3 (4.9, 54.1) | 26.9 (9.7, 50.1) | 34.6 (8.1, 58.1) | 38.1 (1.4, 58.1) |
| Discontinued treatment, n (%) | 7 (78) | 10 (67) | 7 (78) | 14 (88) | 38 (78) |
| Treatment-related AE | 2 (22) | 7 (47) | 2 (22) | 6 (38) | 17 (35) |
| Progressive disease | 4 (44) | 3 (20) | 4 (44) | 8 (50) | 19 (39) |
| Unrelated AE | 1 (11) | 0 | 1 (11) | 0 | 2 (4) |
| Death | 0 | 0 | 0 | 0 | 0 |

### Efficacy

Across treatment arms, ORR was 11-33% (Table 13). ORR was higher in the nivolumab 3 mg/kg + ipilimumab 1 mg/kg compared with the nivolumab 1 mg/kg + ipilimumab 3 mg/kg treatment arms. Median duration of response (DOR) was only reached in the nivolumab 3 mg/kg + ipilimumab 1 mg/kg treatment arm (21 weeks; range 12, 21). Stable disease (SD) as best overall response was reported in 33% of patients overall (22-56% of patients across treatment arms); 7 patients had SD lasting for ≥24 weeks. Across arms, rates of progressive disease as best overall response were 0-44%. Three patients exhibited an unconventional "immune-related" response (immune-related response criteria [irRC] not used). One patient (nivolumab 1 mg/kg + ipilimumab 3 mg/kg squamous arm) had progression of non-target lesions and development of new lesions with simultaneous reduction in target lesions (maximum decrease 55%, most recent decrease 32%). Two patients (in the nivolumab 3 mg/kg + ipilimumab 1 mg/kg arm; one squamous and one non-squamous) had 56% and 64% reductions in target lesions, respectively, in the setting of new lesions appearing.

**Table 13. Tumor response in NSCLC patients treated with nivolumab plus ipilimumab**

| | Nivolumab 1 mg/kg + ipilimumab 3 mg/kg | | Nivolumab 3 mg/kg + ipilimumab 1 mg/kg | |
|---|---|---|---|---|
| | Squamous (n = 9) | Non-squamous (n = 15) | Squamous (n = 9) | Non-squamous (n = 16) |
| ORR, n (%) [95% CI] | 1 (11) [0.3, 48] | 2 (13) [2, 41] | 3 (33) [8, 70] | 2 (13) [2, 38] |
| Ongoing responders, n (%) | 1 (100%) | 2 (100%) | 1 (33%) | 2 (100%) |
| Best overall response, n (%) | | | | |
| Complete response^{a} | 0 | 0 | 0 | 0 |
| Partial response^{a} | 1 (11) | 2 (13) | 3 (33) | 2 (13) |
| SD | 2 (22) | 5 (33) | 5 (56) | 4 (25) |
| Progressive disease | 4 (44) | 4 (27) | 0 | 6 (38) |
| Unable to determine | 2 (22) | 3 (20) | 1 (11) | 2 (13) |
| Estimated median DOR,^{b} weeks (95% CI) | NR | NR | 21 (12,21) | NR |
| Response duration by patient, weeks | 27+ | 6+, 45+ | 12, 14+, 21 | 32+, 49+ |
| Patients with ongoing SD, n (%) | 0 | 1 (20) | 1 (20) | 1 (25) |
| SD duration, weeks | 16,45 | 16, 33, 34, 35+, 47 | 14, 14, 15, 24+, 27 | 13, 16, 22, 36+ |

| | | | | |
|---|---|---|---|---|
| ^{a}All complete and partial responses were confirmed by a subsequent tumor assessment per RECIST 1.1. Patients with an unconfirmed response are not shown ^{b}Time from first response to documented progression, death within 100 days of last nivolumab dose, or last tumor assessment (for censored + data). Estimated median DORs were determined from Kaplan-Meier curves + = response ongoing; CI = confidence interval; NR = not reached | | | | |

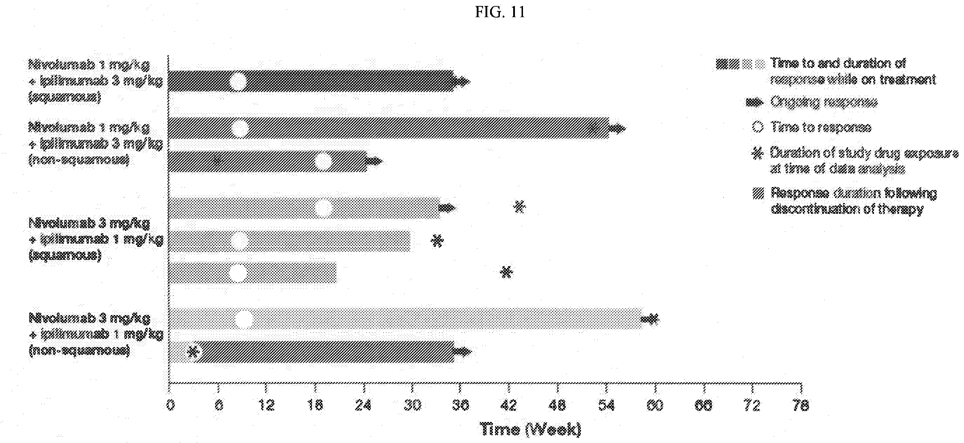

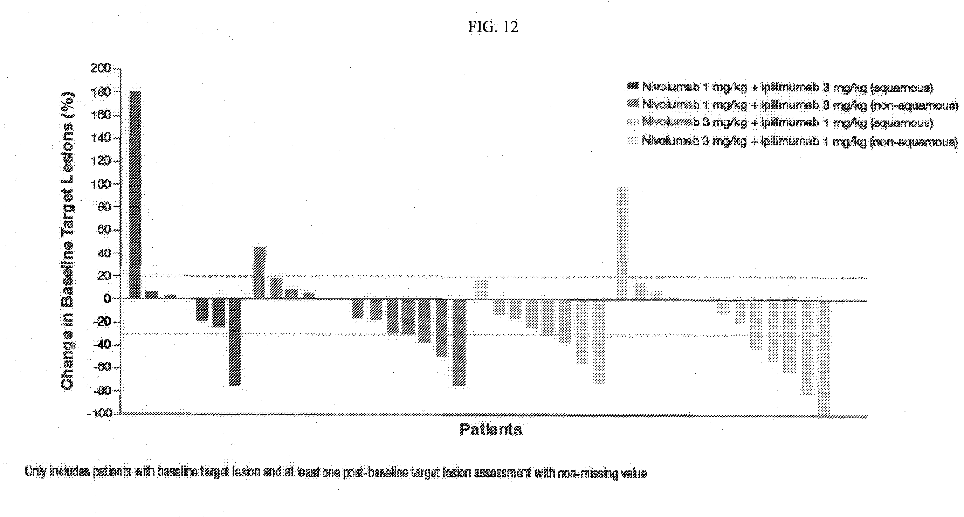

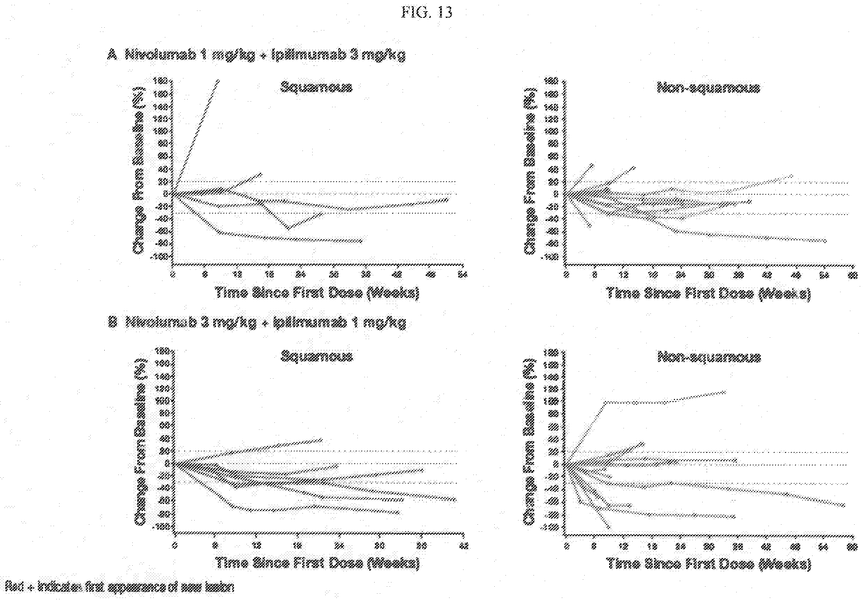

Characteristics of objective responses (ORs) are shown in Figure 11. Overall, responses were ongoing in 6 patients (75%) at the time of analysis. Responses by first tumor assessment (10 weeks) occurred in 6 of 8 responders (75%). Best percent change in target lesion tumor burden from baseline is shown in Figure 12. Overall, the majority of patients with a baseline target lesion and at least one post-baseline target lesion assessment experienced a decrease in tumor burden (24/40; 60%). By week 18, tumor burden reduction of >80% was observed in one patient (nivolumab 3 mg/kg + ipilimumab 1 mg/kg arm; non-squamous). Percent change in target lesions from baseline over time are shown in Figure 13.

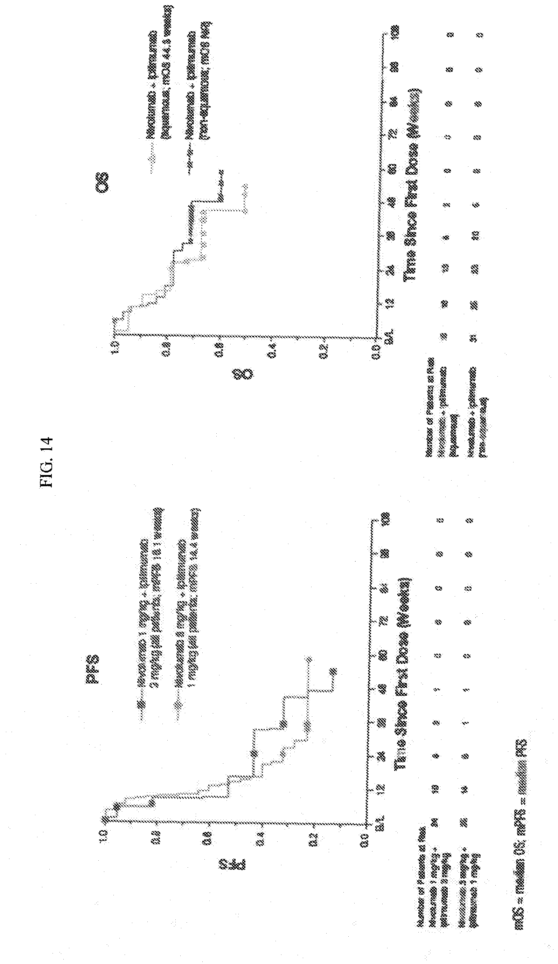

PFS and OS outcomes across treatment arms are shown in Table 14 and Figure 14. PFS rates at 24 weeks ranged from 20 to 51%. Median PFS was 16.1 weeks for the nivolumab 1 mg/kg + ipilimumab 3 mg/kg arm and 14.4 weeks for the nivolumab 3 mg/kg + ipilimumab 1 mg/kg arm (Figure 14); median OS was reached only in the nivolumab 1 mg/kg + ipilimumab 3 mg/kg squamous arm (44.3 weeks).

**Table 14. Survival outcomes in NSCLC patients treated with nivolumab plus ipilimumab**

| | Nivolumab 1 mg/kg + ipilimumab 3 mg/kg | | Nivolumab 3 mg/kg + ipilimumab 1 mg/kg | |
|---|---|---|---|---|
| | Squamous (n = 9) | Non-squamous (n = 15) | Squamous (n = 9) | Non-squamous (n = 16) |
| PFS | | | | |
| PFS rate at 24 weeks, % (95% CI) | 25 (4, 56) | 51 (21,74) | 44 (14, 72) | 20 (5, 43) |
| | 41 (20,61) | | 29 (13,48) | |
| Median PFS, weeks (range) | 8.9 (0.1+, 44.7) | 32.9 (0.1+, 54.1+) | 20.6 (9.7, 33.3+) | 9.9 (4.1+, 58.1+) |
| | 16.1 (0.1+, 54.1+) | | 14.4 (4.1+, 58.1+) | |

| OS | | | | |
|---|---|---|---|---|
| Median OS, weeks (range) | 44.3 (1.4, 53.1+) | NR (4.9+, 54.1+) | NR (9.7, 50.1+) | NR (8.1,58.1+) |
| | NR (1.4, 54.1+) | | NR (8.1, 58.1+) | |

### Exploratory PD-L1 Analysis

### 1. PD-L1 Expression From Pretreatment Tumor Biopsies

Of the 49 treated patients, 38 had pretreatment tumor samples tested for PD-L1 expression by the time of data analysis. 16 were PD-L1+ and 22 were PD-L1-based on an assay cut-off value of 5% PD-L1 expression. Of the 16 PD-L1+ patients, 9 had non-squamous histology and 7 had squamous histology. 11 of the 49 treated patients had not had their tumor samples tested for PD-L1 expression by the time of data analysis.

### 2. Response and Outcomes According to PD-L1 Status

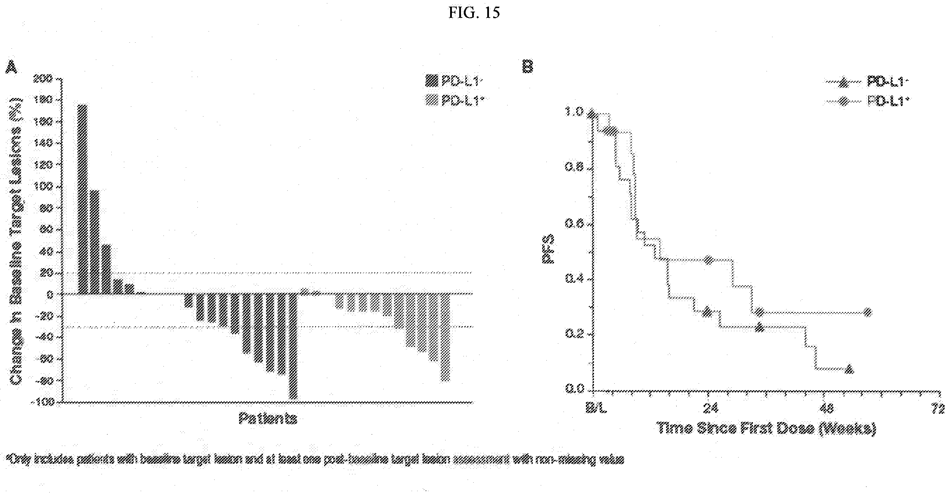

ORR was similar between PD-L1+ and PD-L1- patients (Table 15). Responses were ongoing in 2 PD-L1+ patients and 3 PD-L1- patients. Median PFS was similar between PD-L1+ and PD-L1- patients, and median OS was NR in both subgroups. Best changes in target lesion tumor burden from baseline and PFS by PD-L1 status are shown in Figure 15.

**Table 15. ORR, PFS, and OS according to baseline PD-L1 status in NSCLC patients treated with nivolumab plus ipilimumab**

| | Baseline PD-L1 Expression³ | |
|---|---|---|
| | PD-L1+ | PD-L1⁻ |
| Samples sufficient for PD-L1 analysis, n | 16 | 22 |
| ORR,^{b} n (%) | 3 (19) | 3 (14) |
| Median DOR, weeks (DOR by patient) | NR (21.0, 32.1+, 49.1+) | NR (6.1+, 26.6+, 45.4+) |
| PFS rate at 24 weeks, % (95% CI) | 47 (20, 70) | 29 (12, 48) |
| Median PFS, weeks (range) | 14.4 (1.4, 58.1+) | 13.6 (0.1+, 54.1+) |
| Median OS, weeks (range) | NR (1.4, 58.1+) | NR (5.4, 54.1+) |

| | | |
|---|---|---|
| ^{a}PD-L1+ defined as PD-L1 expression in at least 5% of tumor cells ^{b}ORR is based on confirmed partial responses or complete responses only (per RECIST 1.1) | | |

### Safety

Overall, 88% of patients experienced any-grade treatment-related AEs (Table 16). AEs were managed using protocol safety guidelines. The majority of toxicity occurred during the induction phase (nivolumab + ipilimumab Q3W for four cycles) compared with the maintenance phase (nivolumab 3 mg/kg Q2W until progression). Grade 3/4 AEs were reported in 36-54% of patients across the combination doses during the induction phase versus 21-27% of patients during the maintenance phase. Grade 3/4 treatment-related AEs were reported in 49% of patients across treatment arms (Table 16). Treatment-related grade 3/4 pneumonitis was observed in 3 patients (6%); all cases were reversible with corticosteroids and drug discontinuation. Any-grade treatment-related serious AEs (SAEs) were reported in 29 patients (36%), with grade 3/4 in 22 patients (28%). The most common grade 3/4 SAEs were pneumonitis (3 patients, 6%), diarrhea and colitis in 4 patients (8%) each, and increased ALT and AST in 3 patients (6%) each.

**Table 16. Treatment-related AEs (based on grade 3/4 AEs reported in ≥2% of all patients) in NSCLC patients treated with nivolumab plus ipilimumab**

| | Nivolumab 1 mg/kg + ipilimumab 3 mg/kg | | | | Nivolumab 3 mg/kg + ipilimumab 1 mg/kg | | | | | |
|---|---|---|---|---|---|---|---|---|---|---|
| | Squamous (n = 9) | | Non-squamous (n = 15) | | Squamous (n = 9) | | Non-squamous (n = 16) | | Total (N = 49) | |
| Treatment-related AE, n (%) | All Grades | Grade 3/4 | All Grades | Grade 3/4 | All Grades | Grade 3/4 | All Grades | Grade 3/4 | All Grades | Grade 3/4 |
| Patients with any AE | 7 (78) | 4 (44) | 15 (100) | 10 (67) | 7 (78) | 2 (22) | 14 (88) | 8 (50) | 43 (88) | 24 (49) |
| Diarrhea | 3 (33) | 1 (11) | 7 (47) | 2 (13) | 2 (22) | 1 (11) | 3 (19) | 1 (6) | 15 (31) | 5 (10) |
| Increased ALT | 0 | 0 | 4 (27) | 3 (20) | 1 (11) | 1 (11) | 0 ....... | 0 | 5 (10) | 4 (8) |
| Increased AST | 0 | 0 | 4 (27) | 3 (20) | 1 (11) | 1 (11) | 0 | 0 | 5(10) | 4 (8) |
| Colitis | 0 | 0 | 1 (7) | 1 (7) | 0 | 0 | 4 (25) | 3(19) | 5 (10) | 4 (8) |
| Increased lipase | 0 | 0 | 3 (20) | 2 (13) | 2 (22) | 1 (11) | 2 (13) | 1 (6) | 7 (14) | 4 (8) |
| Fatigue | 3 (33) | 0 | 10 (67) | 2 (13) | 2 (22) | 0 | 7 (44) | 1 (6) | 22 (45) | 3 (6) |
| Pneumonitis | 2 (22) | 1 (11) | 2 (13) | 1 (7) | 1 (11) | 0 | 1 (6) | 1 (6) | 6 (12) | 3 (6) |
| Adrenal insufficiency | 1 (11) | 1 (11) | 1 (7)) | 0 | 0 | 0 | 1 (6) | 1 (6) | 3 (6) | 2 (4) |
| Increased amylase | 0 | 0 | 3 (20) | 1 (7) | 1 (11) | 0 | 2 (13) | 1 (6) | 6 (12) | 2 (4) |
| Rash | 1 (11) | 0 | 4 (27) | 1 (7) | 2 (22) | 0 | 5 (31) | 1 (6) | 12 (24) | 2 (4) |

| | | | | | | | | | | |
|---|---|---|---|---|---|---|---|---|---|---|
| ALT = alanine aminotransferase; AST = aspartate aminotransferase | | | | | | | | | | |

Treatment-related select AEs (defined as AEs with potential immunologic etiologies that require more frequent monitoring and/or unique intervention) are shown in Table 17. Treatment-related AEs led to discontinuation of any study drug in 17 patients (35%), and included pneumonitis (n = 4), increased ALT or AST (n = 3 each), colitis (n = 3), diarrhea (n = 3), and allergic nephritis, ulcerative colitis, impaired gastric emptying, Miller Fisher syndrome, and pulmonary hemorrhage (n = 1 each). The rate of discontinuation due to grade 3/4 AEs was highest in the nivolumab 1 mg/kg + ipilimumab 3 mg/kg non-squamous arm (6 patients, 40%) compared with the other arms (11-31%). The majority of treatment-related AEs leading to discontinuation (n = 16) occurred during concurrent nivolumab + ipilimumab dosing (treatment cycles 1-4). In total, 3 patients died due to drug-related toxicities, with each of the below reported in one patient: respiratory failure following grade 3 colitis; pulmonary hemorrhage; and toxic epidermal necrolysis in a patient with a history of ulcerative colitis.

**Table 17. Treatment-related select AEs reported in NSCLC patients treated with nivolumab plus ipilimumab**

| | Nivolumab 1 mg/kg + ipilimumab 3 mg/kg | | Nivolumab 3 mg/kg + ipilimumab 1 mg/kg | | Total (N = 49) | |
|---|---|---|---|---|---|---|
| | Squamous (n = 9) | Non-squamous (n = 15) | Squamous (n = 9) | Non-squamous (n = 16) | | |
| Treatment-related select AE, n (%) | Grade 3/4 | Grade 3/4 | Grade 3/4 | Grade 3/4 | All Grades | Grade 3/4 |
| Skin | 0 | 1 (7) | 0 | 1 (6) | 24 (49) | 2 (4) |
| Gastrointestinal | 1 (11) | 3 (20) | 1 (11) | 3 (19) | 18 (37) | 8 (16) |
| Endocrinopathies | 2 (22) | 0 | 0 | 1 (6) | 11 (22) | 3 (6) |
| Hepatic | 0 | 3 (20) | 1 (11) | 0 | 8 (16) | 4 (8) |
| Pulmonary | 1 (11) | 1 (7) | 0 | 1 (6) | 6 (12) | 3 (6) |
| Infusion reaction | 0 | 0 | 0 | 0 | 4 (8) | 0 |
| Renal | 0 | 1 (7) | 0 | 0 | 1 (2) | 1 (2) |

### Conclusions

These results suggest that treatment with nivolumab + ipilimumab immunotherapy may provide a feasible combination regimen for chemotherapy-naïve patients diagnosed with advanced NSCLC. The nivolumab + ipilimumab regimen provided durable ORR as first-line treatment for chemotherapy-naïve patients with NSCLC, regardless of histology. Activity was seen with nivolumab + ipilimumab regardless of tumor PD-L1 status, suggesting the combination may be suitable for PD-L1- or PD-L1+ patients.

### EXAMPLE 5

### Treatment of NSCLC with Nivolumab

In the phase 1 CA209-003 study, treatment with nivolumab resulted in durable objective responses (ORs) and prolonged stable disease (SD) in patients with advanced NSCLC 10 (Topalian SL, et al.). The present study reports OS outcomes and clinical activity for the overall NSCLC cohort and across different NSCLC subgroups, results from an exploratory analysis to investigate potential predictive markers associated with nivolumab clinical activity, and safety in all patients with >1 year of follow-up.

### Methods

### Study Design

Patients with NSCLC enrolled between October 2008 and January 2012 received nivolumab 1, 3, and 10 mg/kg intravenously (IV) every 2 weeks (Q2W) in 8-week treatment cycles (4 doses/cycle to a maximum of 12 cycles; 96 weeks), with tumor assessment (Response Evaluation Criteria In Solid Tumors [RECIST] 1.0) after each 4-dose cycle (Topalian SL, et al.). The protocol was amended to collect OS data (January 2012). The current efficacy analysis is based on a September 2013 database lock. Safety results are based on a March 2013 database lock.

### Subgroup Analysis

Objective response rate (ORR) was analyzed according to select patient characteristics (age, gender, Eastern Cooperative Oncology Group performance status [ECOG PS], histology, number of prior therapies, prior tyrosine kinase inhibitor [TKI] therapy, epidermal growth factor receptor [EGFR] and Kirsten rat sarcoma viral oncogene homolog [KRAS] mutational status).

### Exploratory PD-L1 Analysis

PD-L1 expression in archived tumor samples was measured using the Dako immunohistochemistry (IHC) assay. Positive staining with this assay was defined as tumor membrane staining at any intensity, and was analyzed with cut-off values of 1% and 5% expression to assess PD-L1 positivity (Butte MJ, et al.). Tumor PD-L1 expression was evaluated for potential association with clinical endpoints.

### Results

### Key Patient Characteristics

Characteristics of the 129 patients in the NSCLC cohort are shown in Table 18. In this heavily pretreated NSCLC population, 54% had received ≥3 prior therapies. In total, 33 patients received nivolumab 1 mg/kg, 37 patients received nivolumab 3 mg/kg, and 59 patients received nivolumab 10 mg/kg.

**Table 18. Baseline characteristics of patients with NSCLC**

| Variable | All Treated NSCLC Population (N = 129) |
|---|---|
| Age, median years (range) | 65 (38-85) |
| Sex, % (n) | |
| Male | 61 (79) |
| Female | 39 (50) |
| Tumor histology, % (n) | |
| Squamous | 42 (54) |
| Non-squamous | 57 (74) |
| Unknown | 1 (1) |
| ECOG PS, % (n) | |
| 0-1 | 98 (127) |
| 2 | 2 (2) |
| Number of prior therapies, % (n) | |
| 1-2 | 46 (59) |
| 3-5 | 54 (70) |

| Nature of prior therapy, % (n) | |
|---|---|
| Platinum-based chemotherapy | 99 (128) |
| TKI | 28 (36) |
| Surgery | 66 (85) |
| Radiotherapy | 58 (75) |
| Hormonal, immunologic, or biologic therapy | 12 (16) |
| Other | 7(9) |

### Efficacy

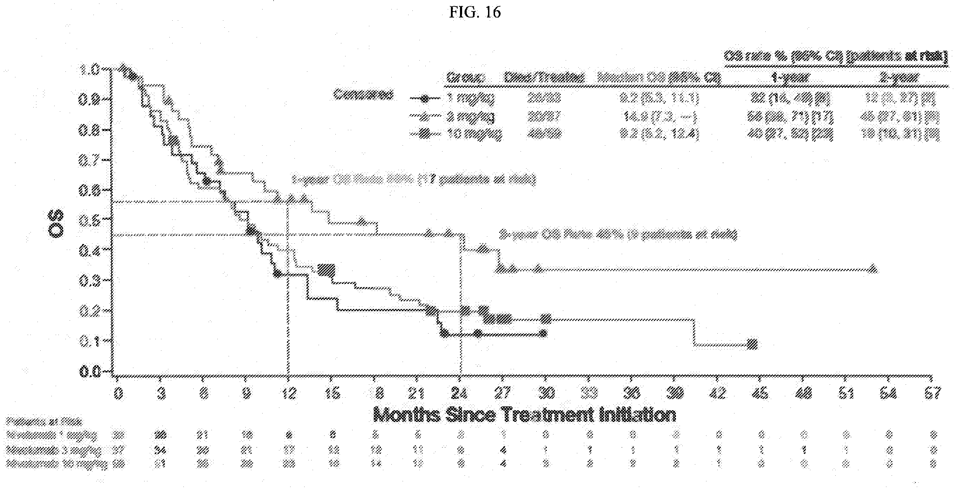

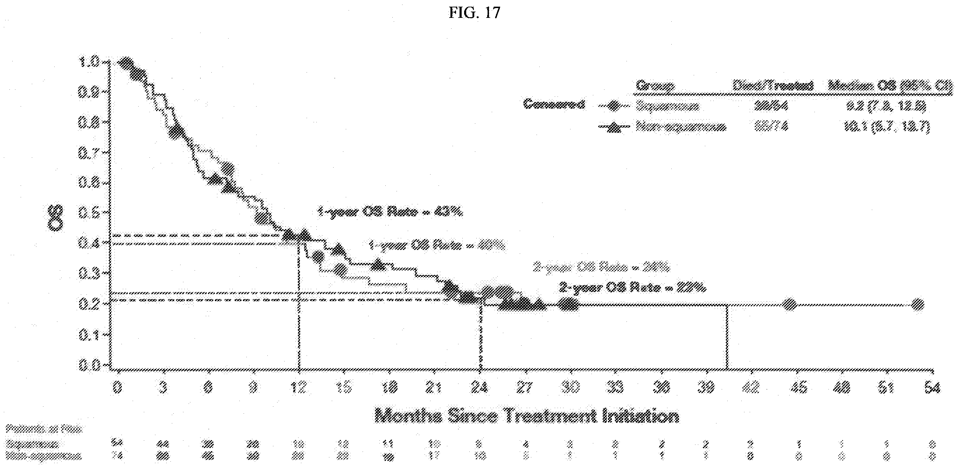

In the NSCLC cohort, 1- and 2-year OS rates were 42% (95% CI 34-51) and 24% (95% CI 16-32), respectively. OS by nivolumab dose is shown in Figure 16. At the 3 mg/kg nivolumab dose, 1- and 2-year OS rates were 56% and 45%, respectively. 1- and 2-year OS rates were similar between histologies (Figure 17).

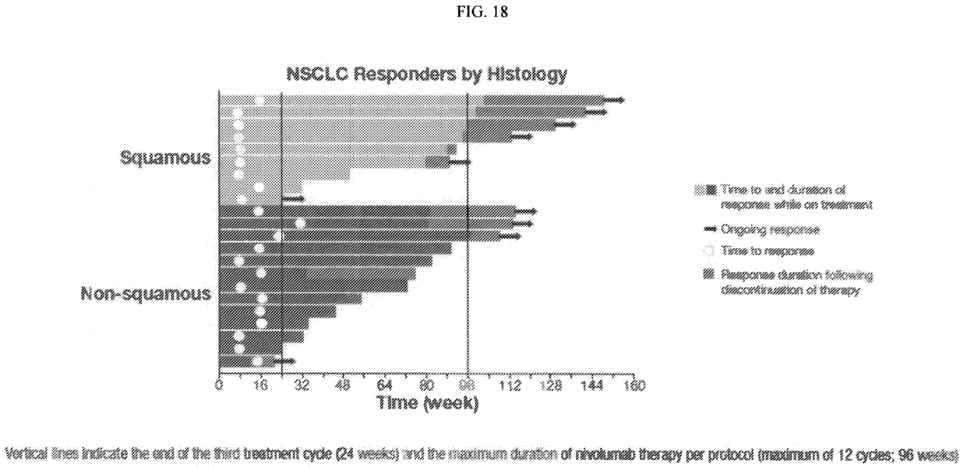

Median OS across doses was 9.9 months, with a median follow-up of 27 months (based on 35 patients still alive at the time of analysis; range 21-54 months). Median OS was 14.9 months at the 3 mg/kg nivolumab dose (currently being evaluated in phase 3 registrational studies; Table 19), Median OS was 9.2 and 10.1 months for patients with squamous and non-squamous NSCLC, respectively (Table 20). ORR was 17% across doses and 24% with nivolumab 3 mg/kg (Table 19). Median duration of response was 74 weeks (17 months). Across doses, 10% of patients had SD for ≥24 weeks, with 5% of these patients maintaining SD for ≥48 weeks. Six patients had unconventional "immune-related" responses and were not included as responders; OS for these patients was 7.3, 11.2, 12.4, 23+, 26.7, and 44.4+ months. ORR was similar across histologies (Table 20). Responses were durable and occurred early (Figure 18). 50% of patients with ORs (11/22) demonstrated response at first assessment (8 weeks). Responses were ongoing in 45% of patients (10/22) at the time of analysis. 6/16 (38%) responders who discontinued for reasons other than disease progression responded for >30 weeks after the last nivolumab dose; responses in 5 of 6 (83%) of these patients were ongoing at the time of reporting.

**Table 19. Efficacy of nivolumab monotherapy by dose in patients with NSCLC**

| Dose, mg/kg | ORR, %^{a} (n/N) | Estimated Median DOR,^{b} wks (range) | SD Rate,^{a} ≥24 wks, % (n/N) | Median PFS,^{c,d} mo (95% CI) | Median OS,^{c,d} mo (95% CI) | OS Rate,^{d,e} % (95% CI) [pts at risk, n] | |
|---|---|---|---|---|---|---|---|
| | | | | | | 1-year | 2-year |
| All doses | 17.0 (22/129) | 74.0 (6.1+, 133.9+) | 10.1 (13/129) | 2.3 (1.8, 3.7) | 9.9 (7.8, 12.4) | 42 (34, 51) [48] | 24 (16, 32) [20] |
| 1 | 3.0 (1/33) | 63.9 (63.9, 63.9) | 15.2 (5/33) | 1.8 (1.7,3.3) | 9.2 (5.3, 11.1) | 32 (16, 49) [8] | 12 (3, 27) [2] |
| 3 | 24.3 (9/37) | 74.0 (16.1+, 133.9+) | 8.1 (3/37) | 1.9 (1.7, 7.3) | 14.9 (7.3, NE) | 56 (38, 71) [17] | 45 (27, 61) [9] |
| 10 | 20.3 (12/59) | 83.1 (6.1+, 132.7+) | 8.5 (5/59) | 3.7 (1.9,3.8) | 9.2 (5.2, 12.4) | 40 (27, 52) [23] | 19 (10, 31) [9] |

| | | | | | | | |
|---|---|---|---|---|---|---|---|
| ^{a}Tumors were assessed after each cycle per RECIST v1.0. Six patients with unconventional "immune-related" responses were not included as responders. CIs for ORRs and SD rates were calculated using the Clopper-Pearson method ^{b}Time from first response to documented progression, death, or last tumor assessment (for censored data denoted by +); estimated median DORs were determined from Kaplan-Meier curves ^{c}Median values for time-to-event endpoints (PFS, OS, DOR) were estimated using the Kaplan-Meier method ^{d}Survival data were collected retrospectively ^{e}OS rates were estimated using the Kaplan-Meier method, with the 95% CIs based on Greenwood's formula DOR = duration of response; NE = not estimable; PFS = progression-free survival | | | | | | | |

**Table 20. Efficacy of nivolumab monotherapy by histology in patients with NSCLC**

| NSCLC Histology^{a} | Dose, mg/kg | ORR,^{b} % (n/N) | SD Rate, ≥24 wksb % (n/N) | Median OS,^{c,d} mo (95% CI) | OS Rate,^{d,e} % (95% CI) [pts at risk, n] | |
|---|---|---|---|---|---|---|
| | | | | | 1-year | 2-year |
| Squamous | All doses | 16.7 (9/54) | 14.8 (8/54) | 9.2 (7.3, 12.5) | 40 (27, 54) [19] | 24 (13, 37) [9] |
| | 1 | 0 (0/15) | 26.7 (4/15) | 8.0 (2.4,13.3) | 27 (7, 51) [3] | 9 (1, 32) [1] |
| | 3 | 22.2 (4/18) | 5.6 (1/18) | 9.5 (5.3, NE) | 49 (23, 71) [7] | 41 (17, 64) [5] |
| | 10 | 23.8 (5/21) | 14.3 (3/21) | 10.5 (4.9, 16.7) | 43 (22, 62) [9] | 22 (7, 42) [3] |
| Non-squamous | All doses | 17.6 (13/74) | 6.8 (5/74) | 10.1 (5.7, 13.7) | 43 (32, 54) [28] | 23 (13, 34) [10] |
| | 1 | 5.6 (1/18) | 5.6 (1/18) | 9.9 (5.3, 22.5) | 36 (15, 58) [5] | 15 (3, 36) [1] |
| | 3 | 26.3 (5/19) | 10.5 (2/19) | 18.2 (5.2, NE) | 62 (37, 80) [10] | 48 (22, 69) [4] |
| | 10 | 18.9 (7/37) | 5.4 (2/37) | 7.4 (4.5, 12.4) | 36 (21,52) [13] | 15 (6,29) [5] |

| | | | | | | |
|---|---|---|---|---|---|---|
| ^{a}One patient with unknown histology ^{b}Tumors were assessed after each cycle per RECIST v1.0. Six patients with unconventional "immune-related" responses were not included as responders. CIs for ORRs and SD rates were calculated using the Clopper-Pearson method ^{c}Median values for time-to-event endpoints (PFS, OS, DOR) were estimated using the Kaplan-Meier method ^{d}Survival data were collected retrospectively ^{e}OS rates were estimated using the Kaplan-Meier method, with the 95% CIs based on Greenwood's formula Efficacy in Patient Subgroups | | | | | | |

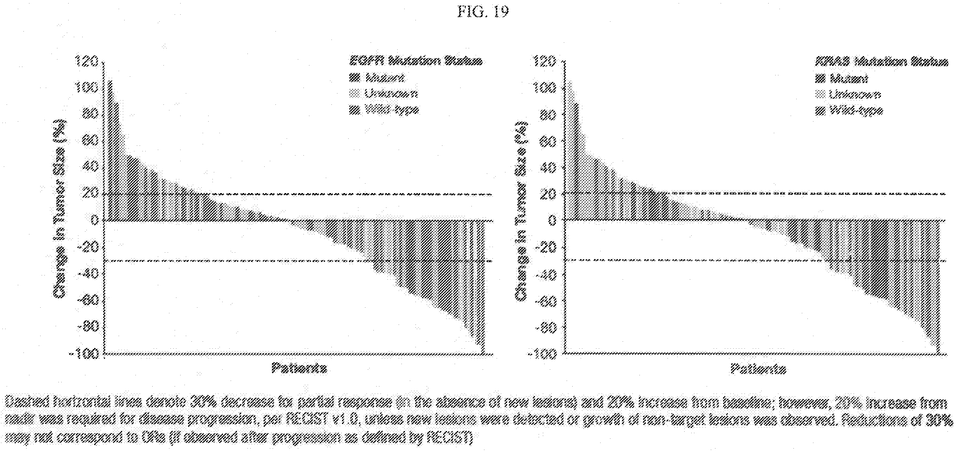

ORRs across select patient subgroups are shown in Table 21. Responses were seen across all patient subgroups analyzed. No apparent association was noted between best change in target lesion tumor burden and EGFR or KRAS mutation status (Figure 19).

**Table 21. ORR by select patient baseline characteristics**

| Subgroup | ORR, % (n/N) [95% CI]^{a} |
|---|---|
| Age | |
| <70 yr | 17 (15/90) [9.6, 26.0] |
| ≥70 yr | 18 (7/39) [7.5, 33.5] |

| Sex | |
|---|---|
| Female | 18 (9/50) [8.6, 31.4] |
| Male | 17 (13/79) [9.1, 26.5] |

| ECOG PS | |
|---|---|
| 0 | 11 (3/27) [2.4, 29.2] |
| 1-2 | 19 (19/102) [11.6,27.6] |

| Histology | |
|---|---|
| Squamous | 17 (9/54) [7.9,29.3] |
| Non-squamous | 18 (13/74) [9.7, 28.2] |

| Prior therapies | |
|---|---|
| <3 | 12 (7/59) [4.9, 22.9] |
| ≥3 | 21 (15/70) [12.5, 32.9] |

| Prior TKI therapy | |
|---|---|
| Yes | 11 (4/36) [3.1, 26.1] |
| No | 19 (18/93) [11.9, 28.9] |

| *EGFR* status | |
|---|---|
| Mutant | 17 (2/12) [2.1, 48.4] |
| Wild-type | 20 (11/56) [10.2, 32.4] |
| Unknown | 15 (9/61) [7.0, 26.2] |

| *KRAS* status | |
|---|---|
| Mutant | 14 (3/21) [3.0, 36,3] |
| Wild-type | 25 (9/36) [12.1, 42.2] |
| Unknown^{b} | 14 (10/72) [6.9, 24.1] |

| | |
|---|---|
| ^{a}CIs for ORRs were calculated using the Clopper-Pearson method. ^{b}Unknown includes both squamous and non-squamous patients. | |

### Exploratory Analysis: PD-L1 Expression From Pretreatment Tumor Biopsies

Of the 129 treated patients, 68 (53%) had evaluable archived tumor tissue samples that were measured for PD-L1 expression (29 squamous, 39 non-squamous). Among these 68 patients, 38 (56%) were PD-L1+ using the 1% cut-off and 33 (49%) were PD-L1+ using the 5% cut-off (Table 22). 61 patients had unknown PD-L1 expression status due to lack of tissue provided for analysis.

### Exploratory Analysis: Association of PD-L1 Expression With Clinical Outcomes

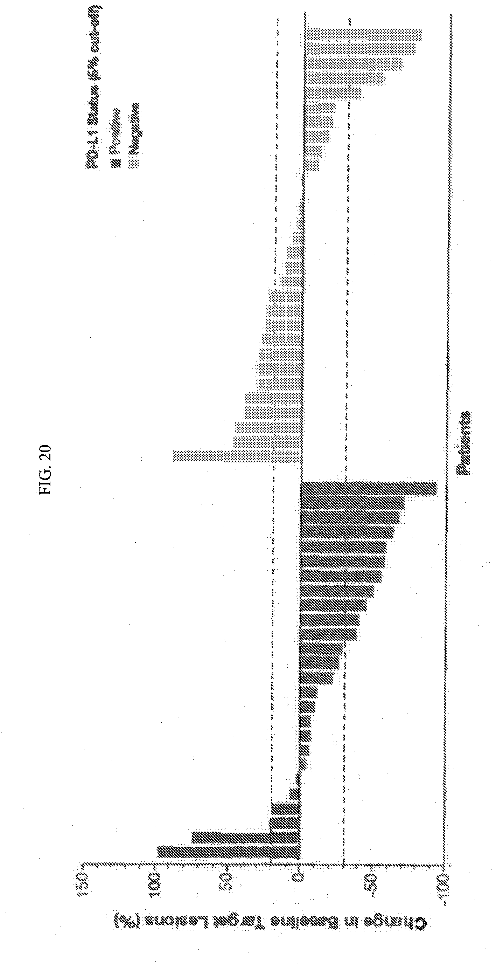

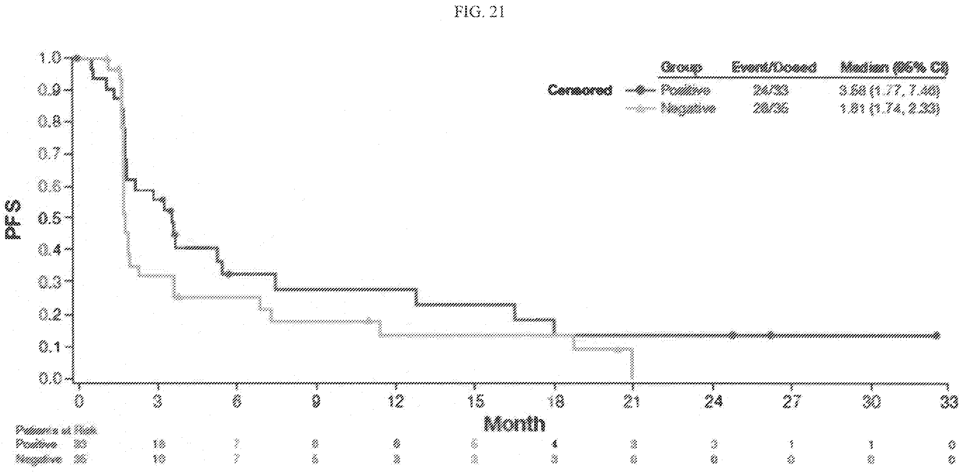

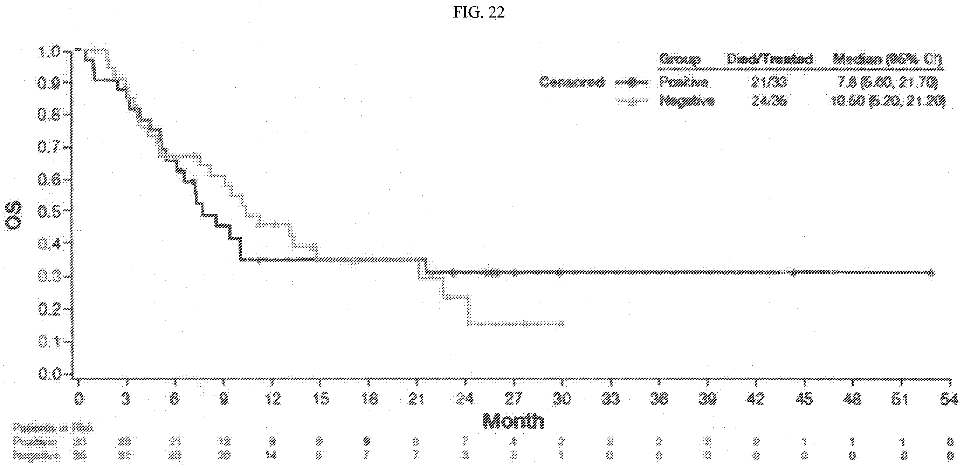

Nivolumab activity was observed in patients with PD-L1+ tumors (Table 22 and Figure 20), as well as in some patients with PD-L1- tumors (Table 22); more patients with PD-L1+ than PD-L1- tumors had a decrease in tumor burden (Figure 20). There was no clear association between ORR and tumor histology (Table 23). PD-L1 expression appeared to have no clear association with PFS (Figure 21) or OS (Figure 22). Median PFS was 3.6 months and 1.8 months in patients with PD-L1+ and PD-L1- tumors, respectively. Median OS was 7.8 months and 10.5 months in patients with PD-L1+ and PD-L1-tumors, respectively.

**Table 22. ORR by tumor PD-L1 expression in patients with NSCLC**

| PD-L1 Cut-off | PD-L1 Status | Evaluable Biopsies, % (n/N) | ORR,^{a} % (n/N) |
|---|---|---|---|
| 1% | Positive | 56 (38/68) | 13 (5/38) |
| | Negative | 44 (30/68) | 17 (5/30) |
| 5% | Positive | 49 (33/68) | 15 (5/33) |
| | Negative | 51 (35/68) | 14 (5/35) |

| | | | |
|---|---|---|---|
| ^{a}ORR of patients evaluable for PD-L1; ORR includes complete or partial responders determined by RECIST 1.0 | | | |

**Table 23. ORR by tumor PD-L1 expression (5% cut-off) and by histology**

| | | PD-L1+ | | PD-L1- | |
|---|---|---|---|---|---|
| Tumor Histology | Total Count^{a} | Count, N | ORR,^{b} % (n/N) | Count, N | ORR,^{b} % (n/N) |
| NSCLC (all patients)^{c} | 129 | 33 | 15 (5/33) | 35 | 14 (5/35) |
| Squamous | 54 | 15 | 13 (2/15) | 14 | 21 (3/14) |
| Non-squamous | 74 | 18 | 17 (3/18) | 21 | 10 (2/21) |

| | | | | | |
|---|---|---|---|---|---|
| "The PD-L1 status of some patients was unknown at the time of data collection: squamous (n = 25), non-squamous (n = 35), NSCLC (all; n - 61) ^{b}ORR is based on RECIST 1.0 and defined as ([complete responses + partial responses] ÷ n) × 100 ^{c}Includes one patient with unknown histology | | | | | |

### Safety

A maximum tolerated dose was not reached up to the maximum planned dose of 10 mg/kg. Spectrum, frequency, and severity of treatment-related adverse events (AEs) were similar in the NSCLC cohort compared with the total population (across all tumor types). Rate of grade 3-4 treatment-related AEs: 14% and 17% in NSCLC cohort (n = 129) and overall study patient population (N = 306), respectively. Similar to the overall study population, the most common AEs in the NSCLC cohort included fatigue (24%), decreased appetite (12%), and diarrhea (10%). Select AEs, defined as AEs with potential immunologic etiologies that require more frequent monitoring and/or unique intervention, are shown in Table 24. 12 (9%) patients had treatment-related pulmonary events. 4 (3%) patients had grade 3-5 treatment-related pneumonitis. Toxicities were manageable using systemic glucocorticoids and/or other immunosuppressive agents. Three treatment-related deaths occurred; all were associated with pneumonitis. Two deaths occurred early in the trial; the third occurred after the database lock. Algorithms are in place to assist with the early identification and management of pneumonitis.

**Table 24. Treatment-related select AE categories (≥1%) occurring in NSCLC patients (N = 129) treated with nivolumab^{a}**

| | Treatment-Related Select AE | |
|---|---|---|
| Category | Any Grade,^{b} % (n) | Grade 3-4, % (n) |
| Patients with any treatment-related select AE | 41 (53) | 5 (6) |
| Skin | 16 (20) | 0 |
| Gastrointestinal | 12 (15) | 1 (1) |
| Pulmonary | 7 (9)^{c} | 2 (3) |
| Endocrinopathies | 6 (8) | 0 |
| Hepatic | 5 (6) | 1 (1) |
| Infusion reaction | 4 (5) | 1 (1) |
| Renal | 3 (4) | 0 |

| | | |
|---|---|---|
| ^{a}Safety results based on a March 2013 database lock ^{b}Grades 1-5 ^{c}Two patients had treatment-related grade 2 pneumonitis, which occurred prior to the date of the safety analysis, but are not included in the table because these data were not available until after this analysis. One additional patient had treatment-related grade 5 pneumonitis, but is not included in this table because the event occurred after the date of the safety analysis | | |

### Conclusions

In this heavily pretreated, advanced NSCLC population, nivolumab produced durable responses and an encouraging survival profile, exhibited responses after discontinuation of therapy, and demonstrated a safety profile managed by protocol algorithms, with no new safety signals emerging with all patients having >1 year of follow-up. Responses to nivolumab were observed across a broad array of NSCLC patient populations regardless of histology or KRAS or EGFR mutation status, including heavily pretreated patients and older patients. In an exploratory analysis of potential associations of biomarkers with nivolumab activity in NSCLC, nivolumab clinical activity was observed in patients with tumor PD-L1+ and PD-L1- expression.

### EXAMPLE 6

### Treatment of NSCLC with Nivolumab

### Study Design

This data is based on a March 2015 database lock. One arm was developed to test the effect of nivolumab monotherapy on NSCLC. Patients were treated with 3 mg/kg nivolumab (nivo) q2w (once every 2 weeks). Baseline characteristics of the patients are shown in Table 25.

**Table 25. Baseline Characteristics.**

| | Arm F |
|---|---|
| | N=52 |
| **Age** | |
| Median (min, max) | 67 (43, 85) |

| **Gender** | |
|---|---|
| Male | 26 (50%) |
| Female | 26 (50%) |

| **Disease Stage** | |
|---|---|
| Stage IIIB | 3 (6%) |
| Stage IV | 49 (94%) |

| **Histology** | |
|---|---|
| Adenocarcinoma | 36 (69%) |
| Squamous | 13 (25%) |
| Other | 3 (6%) |

The mutation status and prior systematic therapy of each patient can be seen in Table 26. The median follow-up time and subject disposition summary can be seen in Table 27.

**Table 26. Mutation Status and Prior Systematic Therapy**

| | Arm F |
|---|---|
| | N=52 |
| EGFR mutation positive | 7 (14%) |
| ALK positive | 0 |
| KRAS positive | 9 (17%) |
| Prior systemic therapy* | 19 (37%) |
| Adjuvant | 11 (21%) |
| Neoadjuvant | 2 (4%) |
| Metastatic | 8 (15%) |
| Erlotinib | 7 (14%) |

| | |
|---|---|
| *More than one setting per subject may be reflected in the frequency. | |

**Table 27. Follow-up Time and Subject Disposition Summary**

| | Arm F |
|---|---|
| Median follow-up (weeks) | 62.2 |
| (Min, Max) | (1, 130.7) |
| Total subjects treated | 52 |
| Discontinued subjects | 47 (90%) |
| Toxicity | 6 (12%) |
| PD | 35 (67%) |
| Death | 2 (4%) |
| Subject request to DC | 1 (2%) |

The most comment treatment-related adverse events reported in greater than 5% of patients are seen in Table 28.

**Table 28. Treatment-related adverse events.**

| | Nivo 3 (n=52) | |
|---|---|---|
| Patients, n (%) | All Gr | GR 3/4 |
| Total patients with an event | 37 (71.2) | 10 (19.2) |
| Fatigue | 15 (28.8) | 0 |
| Rash | 10 (19.2) | 2 (3.8) |
| Nausea | 7 (13.5) | 0 |
| Diarrhea | 6 (11.5) | 1 (1.9) |
| Pruritus | 6 (11.5) | 0 |
| Arthralgia | 5 (9.6) | 0 |
| Constipation | 3 (5.8) | 0 |
| Hypothyriodism | 3 (5.8) | 0 |
| Pneumonitis | 3 (5.8) | 1 (1.9) |
| Vomiting | 3 (5.8) | 0 |

A summary of the overall response of each arm of the study is in Table 29.

**Table 29. BOR Summary.**

| | Arm F (n=52) | Squamous (n=13) | Non-squamous (n=39) |
|---|---|---|---|
| Best Overall Response | | | |
| CR | 4 (8%) | 1 (8%) | 3 (8%) |
| PR | 8 (15%) | 1 (8%) | 7 (18%) |
| Unconfirmed PR | 0 | 0 | 0 |
| SD | 14 (27%) | 6 (46%) | 0 |
| SD 21 weeks) | 10 (19%) | 3 (23%) | 8 (21%) |
| PD | 20 (38%) | 5 (38%) | 7 (18%) |
| Unable to determine | 6 (12%) | 0 | 6 (15%) |
| Confirmed ORR | 12 (23%) | 2 (15%) | 10 (26%) |
| 95% CI | (13%, 37%) | (2%, 45%) | (13%, 42%) |
| Overall ORR | 12 (23%) | 2 (15%) | 10 (26%) |
| 95% CI | (13%, 37%) | (2%, 45%) | (13%, 42%) |
| Confirmed DCR | 26 (50%) | 8 (62%) | 18 (46%) |
| 95% CI | (36%, 64%) | (32%, 86%) | (30%, 63%) |
| Overall DCR | 26 (50%) | 8 (62%) | 18 (46%) |
| 95% CI | (36%, 64%) | (32%, 86%) | (30%, 63%) |

The duration of response, 80% target lesion reduction, progression-free survival and overall survival summary is in Table 30.

**Table 30. Results Summary.**

| | Arm F |
|---|---|
| | N=52 |
| Number of responders | 12 (23%) |
| Ongoing responder | 8 (67%) |
| Median DOR (wks) | NR |
| Min, Max | (18.1, 112.3+) |
| >80% target lesion reduction at 18 wks | 3 (6%) |
| PFS rate at 24 weeks | 41 % |
| (95% CI) | (27%, 54%) |
| Median PFS (wks) | 15.6 |
| (95% CI) | (10, 29) |
| OS rate at 12 months | 74% |
| 95% CI | (0.60, 0.84) |
| OS rate at 18 months | NC |
| (95% CI) | |
| Median OS (mos) | 22.6 |
| (95% CI) | (14.8 --) |

### EXAMPLE 7

### Treatment of NSCLC with Nivolumab Monotherapy or Nivolumab + Ililimumab v. Platinum Doublet Chemotherapy

In a phase 3 CA209-227 study, treatment with nivolumab monotherapy or nivolumab combined with ipilimumab is tested to determine if there is an improvement in overall survival (OS) compared to platinum doublet chemotherapy in chemotherapy-naive subjects with stage IV or recurrent NSCLC. A formal pairwise comparison of OS among experimental arms is conducted.

The study also compares the progression-free survival (PFS) and the objective response rate (ORR), based on Blinded Independent Central Review (BICR) assessment of nivolumab monotherapy and nivolumab in combination with ipilimumab, to platinum-doublet chemotherapy in subjects with previously untreated stage IV or recurrent NSCLC, Differences in PFS and ORR between nivolumab combined with ipilimumab and nivolumab monotherapy in subjects with stage IV or recurrent NSCLC are evaluated.

The study also evaluates whether PD-L1 expression is a predictive biomarker for OS or PFS. And evaluates the proportion of treated patients exhibiting disease-related symptom improvement by 12 weeks as measured by the Lung Cancer Symptom Score (LCSS) in subjects receiving nivolumab monotherapy, nivolumab in combination with ipilimumab and in subjects receiving platinum doublet chemotherapy.

Other objectives of the study include: 1) assessing the overall safety and tolerability of nivolumab and nivolumab in combination with ipilimumab compared to platinum-doublet chemotherapy; 2) characterizing pharmacokinetics of nivolumab in combination with ipilimumab and explore exposure-safety and exposure-efficacy relationships; 3) characterizing immunogenicity of nivolumab in combination with ipilimumab; 4) characterizing immune correlates of nivolumab, nivolumab in combination with ipilimumab and platinum-doublet chemotherapy; 5) assessing predictive tumor and peripheral biomarkers of clinical response to nivolumab and nivolumab in combination with ipilimumab; and 6) assessing overall health status using the EQ-5D index and visual analogue scale in subjects treated with nivolumab in combination with ipilimumab and in those treated with platinum doublet chemotherapy.

### Methods

### Study Design

The study is an open label 4-arm, randomized, Phase 3 study in adult (greater than or equal to 18 years of age) male and female subjects, with stage IV or recurrent non-small cell lung cancer (NSCLC), PD-L1 positive or negative, previously untreated for advanced disease.

Key inclusion criteria include: 1) ECOG Performance Status of greater than or equal to 1 2) Patients with histologically confirmed Stage IV or recurrent NSCLC (per the 7th International Association for the Study of Lung Cancer classification squamous or nonsquamous histology), with no prior systemic anticancer therapy (including EGFR and ALK inhibitors) given as primary therapy for advanced or metastatic disease; and 3) Measurable disease by CT or MRI per RECIST 1.1 criteria.

Key exclusion criteria include: 1) Subjects with known EGFR mutations which are sensitive to available targeted inhibitor therapy; 2) Subjects with known ALK translocations which are sensitive to available targeted inhibitor therapy; 3) Subjects with untreated CNS metastases; 4) Subjects with an active, known or suspected autoimmune disease (subjects with type I diabetes mellitus, hypothyroidism only requiring hormone replacement, skin disorders (such as vitiligo, psoriasis, or alopecia) not requiring systemic treatment, or conditions not expected to recur in the absence of an external trigger are permitted to enroll); and 5) Subjects with a condition requiring systemic treatment with either corticosteroids (> 10 mg daily prednisone equivalent) or other immunosuppressive medications within 14 days of randomization (inhaled or topical steroids, and adrenal replacement steroid > 10 mg daily prednisone equivalent, are permitted in the absence of active autoimmune disease).

Subjects are randomized 1:1:1:1, and stratified by histology (Squamous versus Non-squamous) and PD-L1 status. PD-L1 status is determined by immunohistochemical (IHC) staining of PDL-1 protein in a tumor sample submitted prior to randomization. Subjects are identified as PD-L1 positive if greater than or equal to 5% tumor cell membrane staining in a minimum of a hundred evaluable tumor cells is observed, or PD-L1 negative if less than 5% tumor cell membrane staining in a minimum of a hundred evaluable tumor cells is observed.

Subjects receive open-label treatment from one of four study arms. The dosing schedule is shown in **Table 31.** The maintenance schedule for Nivolumab (Arm B) and optional Pemetrexed (Arm D) is shown in **Table 32.**

**Table 31. Dosing Schedule***

| | **Week 1** | **Week 2** | **Week 3** | **Week 4** | **Week 5** | **Week 6** |
|---|---|---|---|---|---|---|
| **ArmA:** Nivolumab | **Day1** | | **Day1** | | **Day1** | |
| 240mg q 2 weeks^{a} | Nivolumab | | Nivolumab | | Nivolumab | |

| Arm B: Nivolumab | **Day1** | | | **Day1** | | |
|---|---|---|---|---|---|---|
| 1 mg/kg + Ipilimumab 1 mg/kg q 3w x4 followed by maintenance nivolumab ^{a} | Nivolumab + Ipilimumab | | | Nivolumab + Ipilimumab | | |

| ArmC**:** | **Day1** | | **Day1** | | **Day1** | |
|---|---|---|---|---|---|---|
| Nivolumab 3 mg/kg q 2 weeks + Ipilimumab 1 mg/kg q 6 weeks ^{a} | Nivolumab + Ipilimumab | | Nivolumab | | Nivolumab | |

| **ArmD** | **Day1**: | **Day8** | | **Day1:** | **Day8** | |
|---|---|---|---|---|---|---|
| Platinum doublet chemotherapy: q 3 w x 4-6 followed by optional maintenance Pemetrexed for nonsquamous histology | Gemcitabine/Cisplatin or Gemcitabine/Carboplatin or Pemetrexed/Cisplatin or Pemetrexed/Carboplatin | Gemcitabine | | Gemcitabine/cisplatin or Gemcitabine/carboplatin or Pemetrexed/cisplatin or Pemetrexed/carboplatin | Gemcitabine | |

| | | | | | | |
|---|---|---|---|---|---|---|
| * Both nivolumab and ipilimumab may be administered as 30 minute infusions ^{a} continues until disease progression, discontinuation due to unacceptable toxicity, withdrawal of consent, or study closure | | | | | | |

**Table 32. Maintenance Schedule for Nivolumab (Arm B) and Optional Pemetrexed (Arm D)**

| | **Week 1** | **Week 2** | **Week 3** | **Week 4** | **Week 5** | **Week 6** |
|---|---|---|---|---|---|---|
| **ArmB:** | Day1 | | Day1 | | Day1 | |
| Nivolumab 3 mg/kg q 2 weeks^{a} | Nivolumab | | Nivolumab | | Nivolumab | |

| **ArmD:** | Day1 | | | Day1 | | |
|---|---|---|---|---|---|---|
| (pemetrexed 500 mg/m²) | Pemetrexed | | | Pemetrexed | | |

| | | | | | | |
|---|---|---|---|---|---|---|
| ^{a} continues until disease progression, discontinuation due to unacceptable toxicity, withdrawal of consent, or study closure. | | | | | | |

On-study tumor assessment begins at Week 6 post randomization (± 7 days) and is performed every 6 weeks (± 7 days) until Week 48. After Week 48, tumor assessment is performed every 12 weeks (± 7 days) until progression or treatment discontinuation, whichever occurs later. Subjects receiving nivolumab or nivolumab plus ipilimumab beyond investigator-assessed RECIST 1.1-defined progression must also continue tumor assessments until such treatment is discontinued. Enrollment will end after approximately 1200 subjects are randomized. The primary endpoint of the study is Overall Survival (OS). The duration of the study from start of enrollment to analysis of the primary OS endpoint is expected to be approximately 48 months.

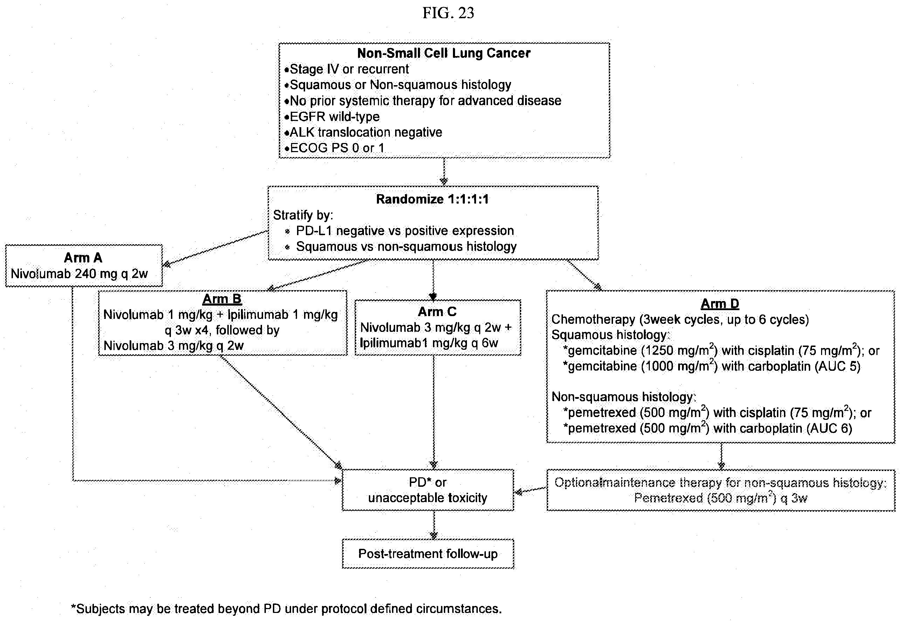

The study design schematic is presented in **Figure 23****.**

### Study Arms

### Nivolumab Monotherapy (Arm A)

Nivolumab 240 mg is administered intravenously (IV) on day 1 of each cycle over 30 minutes every 2 weeks until disease progression, discontinuation due to unacceptable toxicity, withdrawal of consent or study closure. Treatment beyond initial investigator-assessed RECIST 1.1-defined progression is permitted if the subject has investigator-assessed clinical benefit and is tolerating treatment. Upon completion of dosing, subjects enter the Follow-up Phase.

Study assessments are collected as outlined in **Table 33.**

**Table 33. On Study Assessments Treatment Phase-Arms A (CA209227) ^{a}**

| **Procedure** | **Cycle 1 Day 1** | **Each Subsequent Cycle Day1** | **Every 2 Cycles Day 1** | **Every 3 Cycles Day 1** | **Notes For purposes of this table, a cycle refers to the nivolumab every 2 weeks regimen.** |
|---|---|---|---|---|---|
| **SafetyAssessments** | | | | | |
| Physical Measurements & ECOG Performance Status | X | X | | | |
| Vital Signs and Oxygen Saturation | | X | | | |
| Adverse Event Assessments | Continuously during the study | | | | |
| Review of Concomitant Medications | X | X | | | |
| Laboratory Tests | X | X | | X (TSH) | Within 72 hrs prior to dosing to include CBC w/ differential, AST, ALT, ALP, T.Bili, BUN or serum urea level, creatinine, albumin, Ca, Mg, Na, K, Cl, LDH, glucose, amylase, lipase, TSH (with reflexive Free T4 and Free T3). Thyroid Function Testing to be evaluated every 6 weeks |
| | | | | | **Note:** C1D1 labs do not need to be repeated if they were performed within 14 days of dosing. |
| Pregnancy Test (WOCBP only) | X | | X | | To be evaluated at least every 4 weeks |

| **EfficacyAssessments** | | | | | |
|---|---|---|---|---|---|
| Radiographic Tumor Assessment (CT/MRI of chest, abdomen, pelvis) | **FIRST** tumor assessment should first be performed at 6 weeks (± 7 days) from randomization date. | | | | |
| | **SUBSEQUENT** tumor assessments should occur every 6 weeks (± 7 days) up to first 12 months (week 48), then every 12 weeks until disease progression. | | | | |
| | * Subjects with a history of brain metastasis may have surveillance MRI approximately every 12 weeks from the date of first dose, or sooner if clinically indicated. | | | | |

| **ExploratoryBiomarker Assessments** | | | | | |
|---|---|---|---|---|---|
| Single Nucleotide Polymorphisms (SNPs) | X | | | | Obtained prior to dosing. |
| Serum for Soluble Factors and miRNA Analyses | C1D1 pre-dose, C1D1 post-dose, C2D1, C3D1, C4D1, C5D1 | | | | Obtained prior to dosing. |
| Myeloid Derived Suppressor Cells (MDSCs) | X | | | | Obtained prior to dosing. |
| Peripheral Blood Mononuclear Cells (PBMCs) | C1D1 pre-dose, C1D1 post-dose, C2D1, C3D1, C4D1, C5D1 | | | | Collected in USA and Canada Only. Obtained prior to dosing. |
| **Optional** Tumor Biopsy Gene Expression Profiling | X | | | | As feasible obtained at baseline or any time on treatment |

| **Pharmacotinetic(PK)and ImmunogenicityAssessments** | | | | | |
|---|---|---|---|---|---|
| PK Samples | Throughout the study | | | | |
| Immunogenicity Samples | Throughout the study | | | | |
| Patient Reported Outcomes Assessment (PRO) | X | X | | After 6 months | For C1D1 - LCSS and EQ-5D assessments performed after randomization, PRIOR to first dose (day -3 to +1). |
| | | | | | For on-study visits: Assessments (LCSS and EQ-5D) will be performed PRIOR to any study procedures and treatment. |
| | | | | | Assessments will be performed at each cycle on Day 1 for the first 6 months on study, then every 6 weeks thereafter for the remainder of the **treatment period** |
| Health Resource Utilization | | X | | | Except cycle 1. To include: concomitant medication collection |
| Clinical Drug Supplies | | | | | |
| IVRS Vial Assignment | X | X | | | Within 1 day prior to dosing |
| Nivolumab 240 mg q 2 weeks | X | X | | | See, e.g., Table 31 |

| | | | | | |
|---|---|---|---|---|---|
| ^{a} If a dose is delayed, the procedures scheduled for that same timepoint should be delayed to coincide with when the time point's dosing actually occur. ^{b} continues until disease progression, discontinuation due to unacceptable toxicity, withdrawal of consent, or study closure. | | | | | |

### Nivolumab plus Ipilimumab (Arm B)

Nivolumab 1 mg/kg is administered IV over 30 minutes combined with ipilimumab 1 mg/kg administered IV over 30 minutes every 3 weeks x4, followed by nivolumab 3 mg/kg administered IV over 30 minutes every 2 weeks. Nivolumab 1 mg/kg and ipilimumab 1 mg/kg will both be administered on day 1 of each 3 week treatment cycle x4. Following the 4th treatment cycle, nivolumab 3 mg/kg will be administered every 2 weeks until disease progression, unacceptable toxicity, withdrawal of consent, or study closure. Treatment beyond initial investigator-assessed RECIST 1.1-defined progression is permitted if the subject has investigator-assessed clinical benefit and is tolerating treatment. Upon completion of dosing, subjects enter the Follow-up Phase.

Study assessments are collected as outlined in **Table 34**.

**Table 34. On Study Assessments Treatment Phase-Arms B (CA209227)^{a}**

| **Procedure** | **Cycle 1 Day 1** | **Each Subsequent Cycle Day1** | **Every 2 Cycles Day 1** | **Every 3 Cycles Day 1** | **Notes For the purposes of this table, a cycle refers to the nivolumab + ipilimumab every 3 weeks x4 regimen until for the maintenance phase when it refers to nivolumab every 2 weeks.** |
|---|---|---|---|---|---|
| **SafetyAssessments** | | | | | |
| Physical Measurements & ECOG Performance Status | X | X | | | |
| Vital Signs and Oxygen Saturation | | X | | | |
| Adverse Event Assessments | Continuously during the study | | | | |
| Review of Concomitant Medications | X | X | | | |
| Laboratory Tests | X | X | X(TSH) | | Within 72 hrs prior to dosing to include CBC w/ differential, AST, ALT, ALP, T.Bili, BUN or serum urea level, creatinine, albumin, Ca, Mg, Na, K, Cl, LDH, glucose, amylase, lipase, TSH (with reflexive Free T4 and Free T3). Thyroid Function Testing to be evaluated every 6 weeks |
| | | | | | **Note:** C1D1 labs do not need to be repeated if they were performed within 14 days of dosing. |
| Pregnancy Test (WOCBP only) | X | X | | | To be evaluated at least every 3 weeks |
| **EfficacyAssessments** | | | | | |
| Radiographic Tumor Assessment (CT/MRI chest, abdomen, pelvis) | **FIRST** tumor assessment should first be performed at 6 weeks (± 7 days) from randomization date. | | | | |
| | **SUBSEQUENT** tumor assessments should occur every 6 weeks (± 7 days) up to first 12 months (week 48), then every 12 weeks until disease progression. | | | | |
| | * Subjects with a history of brain metastasis may have surveillance MRI approximately every 12 weeks from the | | | | |
| | date of first dose, or sooner if clinically indicated. | | | | |

| **ExploratoryBiomarker Assessments** | | | | | |
|---|---|---|---|---|---|
| Single Nucleotide Polymorphisms (SNPs) | X | | | | Obtained prior to dosing. |
| Serum for Soluble Factors and miRNA Analyses | C1D1 pre-dose, C1D1 post-dose, C2D1, C3D1, C5D1 | | | | Obtained prior to dosing. |
| Myeloid Derived Suppressor Cells (MDSCs) | X | | | | Obtained prior to dosing. |
| Peripheral Blood Mononuclear Cells (PBMCs) | C1D1 pre-dose, C1D1 post-dose, C2D1, C3D1, C5D1 | | | | Collected in USA and Canada Only. Obtained prior to dosing. |
| **Optional** Tumor Biopsy for Gene Expression Profiling | X | | | | As feasible obtained at baseline or any time on treatment |

| **Pharmacokinetic(PK)and ImmunogenicityAssessments** | | | | | |
|---|---|---|---|---|---|
| PK Samples | Throughout the study | | | | |
| Immunogenicity Samples | Throughout the study | | | | |
| Patient Reported Outcomes Assessment (PRO) | X | X | | After 6 months | For C1D1 - LCSS and EQ-5D assessments performed after randomization PRIOR to first dose (day -3 to +1). |
| | | | | | For on-study visits: Assessments (LCSS and EQ-5D) will be performed PRIOR to any study procedures and treatment. |
| | | | | | Assessments will be performed at each cycle on Day 1 for the first 6 months on study, then every 6 weeks thereafter for the remainder of the **treatment period** |
| Health Resource Utilization | | X | | | Except cycle 1. To include: concomitant medication collection. |

| **ClinicalDrugSupplies** | | | | | |
|---|---|---|---|---|---|
| IVRS Vial Assignment | X | X | | | Within 1 day prior to dosing |
| Nivolumab **1** mg/kg + Ipilimumab **1** mg/kg q 3w x4 | X | X | | | See, e.g., Table 31. |

| **MainteancePhaseDoseSchedule** | | | | | |
|---|---|---|---|---|---|
| Nivolumab 3 mg/kg^{b} | X | X | | | See, e.g., Table 32 |

| | | | | | |
|---|---|---|---|---|---|
| ^{a} If a dose is delayed, the procedures scheduled for that same time point should be delayed to coincide with when the time point's dosing actually occur ^{b} continue until disease progression, discontinuation due to unacceptable toxicity, withdrawal of consent, or study closure | | | | | |

### Nivolumab plus ipilimumab (Arm C)

Nivolumab 3 mg/kg is administered IV over 30 minutes every 2 weeks, and ipilimumab I mg/kg is administered IV over 30 minutes at 1 mg/kg every 6 weeks following the administration of nivolumab until progression, discontinuation due to toxicity, withdrawal of consent, or study closure. Subjects may discontinue only one study drug and continue treatment with the other if certain circumstances are met. Treatment beyond initial investigator-assessed RECIST 1.1 defined progression is permitted if the subject has investigator assessed clinical benefit and is tolerating treatment. Upon completion of dosing, subjects enter the Follow-up Phase.

Study assessments are collected as outlined in **Table 35.**

**Table 35. On Study Assessments Treatment Phase-Arms C (CA209227)^{a}**

| **Procedure** | **Cycle 1 Day 1** | **Each Subsequent Cycle Day1** | **Every 2 Cycles Day 1** | **Every 3 Cycles Day 1** | **Notes For the purposes of this table, a cycle refers to nivolumab every 2 weeks.** |
|---|---|---|---|---|---|
| **SafetyAssessments** | | | | | |
| Physical Measurements & ECOG Performance Status | X | X | | | |
| Vital Signs and Oxygen Saturation | | X | | | |
| Adverse Event Assessments | Continuously during the study | | | | |
| Review of Concomitant Medications | X | X | | | |
| Laboratory Tests | X | X | | X(TSH) | Within 72 hrs prior to dosing to include CBC w/ differential, AST, ALT, ALP, T.Bili, BUN or serum urea level, creatinine, albumin, Ca, Mg, Na, K, Cl, LDH, glucose, amylase, lipase, TSH (with reflexive Free T4 and Free T3). Thyroid Function Testing to be evaluated every 6 weeks |
| | | | | | **Note:** C1D1 labs do not need to be repeated if they were performed within 14 days of dosing. |
| Pregnancy Test (WOCBP only) | X | | X | | To be evaluated at least every 4 weeks |

| **EfficacyAssessments** | | | | | |
|---|---|---|---|---|---|
| Radiographic Tumor Assessment (CT/MRI of chest, abdomen, pelvis) | **FIRST** tumor assessment should first be performed at 6 weeks (± 7 days) from randomization date. | | | | |
| | **SUBSEQUENT** tumor assessments should occur every 6 weeks (± 7 days) up to first 12 months (week 48), then every 12 weeks until disease progression. | | | | |
| | * Subjects with a history of brain metastasis may have surveillance MRI approximately every 12 weeks from the date of first dose, or sooner if clinically indicated. | | | | |

| **ExplorataryBiomarkerAssessments** | | | | | |
|---|---|---|---|---|---|
| Single Nucleotide Polymorphisms (SNPs) | X | | | | Obtained prior to dosing. |
| Serum for Soluble Factors and miRNA Analyses | C1D1 pre-dose, C1D1 post-dose, C2D1, C3D1, C4D1, C5D1 | | | | Obtained prior to dosing. |
| Myeloid Derived Suppressor Cells (MDSCs) | X | | | | Obtained prior to dosing. |
| Peripheral Blood Mononuclear Cells (PBMCs) | C1D1 pre-dose, C1D1 post-dose, C2D1, C3D1, C4D1, C5D1 | | | | CollectedinUSAandCanada Only. Obtained prior to dosing. |
| **Optional** Tumor Biopsy Gene Expression Profiling | X | | | | As feasible obtained at baseline or any time on treatment |

| **Pharmacokinetic(PK)and ImmunogenicityAssessments** | | | | | |
|---|---|---|---|---|---|
| PK Samples | Throughout the study | | | | |
| Immunogenicity Samples | Throughout the study | | | | |
| Patient Reported Outcomes Assessment (PRO) | X | X | | After 6 months | For C1D1 - LCSS and EQ-5D assessments performed after randomization PRIOR to first dose (day -3 to +1). |
| | | | | | For on-study visits: Assessments (LCSS and EQ-5D) will be performed PRIOR to any study procedures and treatment. |
| | | | | | Assessments will be performed at each cycle on Day **1** for the first 6 months on study, then every 6 weeks thereafter for the remainder of the **treatment period** |
| **Health Resource Utilization** | | X | | | Except cycle 1. To include: concomitant medication collection |

| **ClinicalDrugSupplies** | | | | | |
|---|---|---|---|---|---|
| IVRS Vial Assignment | X | X | | | Within 1 day prior to dosing |
| Nivolumab 3 mg/kg q 2 weeks + Ipilimumab 1 mg/kg q 6 weeks^{b} | X | X | | | See, e.g., Table 31. |

| | | | | | |
|---|---|---|---|---|---|
| ^{a} If a dose is delayed, the procedures scheduled for that same timepoint should be delayed to coincide with when the time point's dosing actually occur ^{b} continues until disease progression, discontinuation due to unacceptable toxicity, withdrawal of consent, or study closure | | | | | |

### Platinum Doublet Chemotherapy and Optional Continuation Maintenance (Arm D)

Platinum-doublet chemotherapy is administered IV in 3-week cycles for up to a maximum of 6 cycles of chemotherapy. Chemotherapy treatment continues until disease progression, unacceptable toxicity or completion of the 4-6 cycles, whichever comes first. Platinum-doublet chemotherapy regimens is dependent on NSCLC histology. Subjects with mixed histology are classified according to the predominant histology.

Squamous histology subjects are given receive Gemcitabine (1250 mg/mg²) with cisplatin (75 mg/m²); or Gemcitabine (1000 mg/mg²) with carboplatin (AUC 5). Gemcitabine is administered on Day 1 and Day 8 of each cycle.

Non-squamous histology subjects receive Pemetrexed (500 mg/m²) with cisplatin (75 mg/m²), administered on Day 1 of each cycle; or Pemetrexed (500 mg/m²) with carboplatin (AUC 6), administered on Day 1 of each cycle.

Subjects with non-squamous histology who have stable disease or response after Cycle 4 are permitted to continue pemetrexed alone as maintenance therapy until disease progression or unacceptable toxicity. Upon completion of chemotherapy, subjects enter Follow-up Phase.

Study assessments are collected as outlined in **Table 36.**

**Table 36. On Study Assessments Treatment Phase-Arms D (CA209227)^{a}**

| **Procedure** | **Cycle 1 Day 1** | **Each Subsequent Cycle Day1** | **Every 2 Cycles Day 1** | **Every 3 Cycles Day 1** | **Notes For purposes of this table, a cycle refers to the platinum doublet chemotherapy every 3 weeks regimen.** |
|---|---|---|---|---|---|
| **SafetyAssessments** | | | | | |
| Physical Measurements & ECOG Performance Status | X | X | | | See, e.g., Table 32 |
| Vital Signs and Oxygen Saturation | | X | | | |
| Adverse Event Assessments | Continuously during the study | | | | Monitoring for adverse events related to chemotherapy drugs in Arm D subjects should follow recommendations specified in the local labels. |
| Review of Concomitant Medications | X | X | | | |
| Laboratory Tests | | X | X (TFTs) | | Within 72 hrs prior to dosing to include CBC w/ differential, AST, ALT, ALP, T.Bili, BUN or serum urea level, creatinine, albumin, Ca, Mg, Na, K, Cl, LDH, glucose, amylase, lipase, TSH (with reflexive Free T4 and Free T3). Thyroid Function Testing to be evaluated every 6 weeks. |
| | | | | | CBC required prior to gemcitabine dosing on Day 8 of each cycle. |
| | | | | | **Note:** C1D1 labs do not need to be repeated if they were performed within 14 days of dosing. |
| Pregnancy Test (WOCBP only) | X | X | | | To be evaluated at least every 3 weeks |

| **EfficacyAssessments** | | | | | |
|---|---|---|---|---|---|
| Radiographic Tumor Assessment (CT/MRI of chest, abdomen, pelvis) | **FIRST** tumor assessment should first be performed at 6 weeks (± 7 days) from randomization date. | | | | |
| | **SUBSEQUENT** tumor assessments should occur every 6 weeks (± 7 days) up to first 12 months (week 48), then every 12 weeks until disease progression. | | | | |
| | * Subjects with a history of brain metastasis may have surveillance MRI approximately every 12 weeks from the date of first dose, or sooner if clinically indicated. | | | | |

| **ExplorataryBiomarkerAssessments** | | | | | |
|---|---|---|---|---|---|
| Single Nucleotide Polymorphisms (SNPs) | X | | | | Obtained prior to dosing. |
| Serum for Soluble Factors and miRNA Analyses | C1D1 pre-dose, C1D1 post-dose, C2D1, C3D1, C5D1 | | | | Obtained prior to dosing. |
| Myeloid Derived Suppressor Cells (MDSCs) | X | | | | Obtained prior to dosing. |
| Peripheral Blood Mononuclear Cells (PBMCs) | C1D1 pre-dose, C1D1 post-dose, C2D1, C3D1, C5D1 | | | | CollectedinUSAandCanada Only. Obtained prior to dosing. |
| **Optional** Tumor Biopsy Gene Expression Profiling | X | | | | As feasible obtained at baseline or any time on treatment |
| Patient Reported Outcomes Assessment (PRO) | X | X | | After 6 months | For C1D1 - LCSS and EQ-5D assessments performed after randomization PRIOR to first dose (day -3 to +1). |
| | | | | | For on-study visits: Assessments (LCSS and EQ-5D) will be performed PRIOR to any study procedures and treatment. |
| | | | | | Assessments will be performed at each cycle on Day 1 for the first 6 months on study, then every 6 weeks thereafter for the remainder of the **treatment period** |
| Health Resource Utilization | | X | | | Except cycle 1. To include: concomitant medication collection |

| **ClinicalDrugSupplies** | | | | | |
|---|---|---|---|---|---|
| IVRS Vial Assignment | X | X | | | Within 1 day prior to dosing |
| Platinum doublet Chemotherapy | X | X | | | For Platinum doublet |
| | | | | | chemotherapy options & dose levels, refer to, e.g., Table 31 and Table 32. Gemcitabine will be provided on day 1 and day 8 of each dosing cycle. |

| **MaintenancePhaseDaseSchedule:** Optional maintenance Pemetrexed for nonsquamous histology subjects only | | | | | |
|---|---|---|---|---|---|
| Pemetrexed 500 mg/m² | Week **1** Day 1 and Week 4 Day 1 | | | | See, e.g., Table 32. |

| | | | | | |
|---|---|---|---|---|---|
| ₐII a dose is delayed, me procedures scheduled for that same time point should be delayed to coincide with wnen the time point's dosing actually occur | | | | | |

### Post-Treatment Follow-up

The post-treatment follow-up begins when the decision to discontinue a subject from all treatment is made; this includes optional continuation maintenance therapy. Subjects who discontinue treatment for reasons other than disease progression will continue to have tumor assessments (if clinically feasible) according to the schedule in **Table 37** until progression or the start of any subsequent therapy, whichever occurs first. Subjects are followed for drug-related toxicities until these toxicities resolve, return to baseline or are deemed irreversible. All adverse events are documented for a minimum of 100 days after the last dose of study medication. After completion of the first two follow-up visits, subjects are followed every 3 months for survival. Study assessments are to be collected as outlined in **Table 37.**

**Table 37. Follow-up and Survival Procedures (CA209227) - All subjects**

| **Procedure** | **Follow-Up Visits 1 & 2^{a}** | **Survival Follow-up Visits^{b}** | **Notes** |
|---|---|---|---|
| **SAFETY ASSESSMENTS** | | | |
| Targeted Physical Examination | X | | To assess for potential late emergent study drug related issues. |
| Vital Signs | X | | |
| Adverse Event Assessment | X | X | |
| Review of Concomitant Medications | X | | |
| Laboratory Tests | X | | Required at Visit 1. Repeat at Visit 2 only if study drug related toxicity persists. |

| **EFFICACY ASSESSMENTS** | | | |
|---|---|---|---|
| Radiographic Tumor Assessment (CT/MRI of chest, abdomen, pelvis and known sites of disease) | X | X | For subjects who discontinue study treatment for reasons |
| | | | other than PD, follow up scans should be performed every 6 weeks (± 1 wk) up to first 12 months (week 48), then every 12 weeks until PD, lost to follow-up, or withdrawal of consent |
| | | | *Radiographic assessments for subjects who have not experienced PD ***must*** be obtained everv6weeks (± 7 days), and ***not*** delayed until follow-up visits 1 & 2. |
| Patient Reported Outcomes Assessment (PRO) | X | EQ-5D only | Both the LCSS and EQ-5D will be given in FU Visits **1** & 2. In Survival Visits, EQ-5D is collected every 3 months for the first year of the Follow-Up Phase, then every 6 months thereafter. |
| Pharmacokinetic & Immunogenicity Assessments | X | | |
| Collection of Survival Status and Subsequent Therapy Information | X | X | Collect **every 3 months** in Survival Visits until death, lost to follow-up, or withdrawal of study consent. May be performed by phone contact or office visit. |

| | | | |
|---|---|---|---|
| ^{a} Follow-Up Visit 1 to occur 35 days from the last dose (± 7 days) or coinciding with the date of discontinuation of study drug (± 7 days) if the date of discontinuation is greater than 42 days from the last dose. Follow-Up Visit 2 to occur 80 days from Follow-Up Visit **1** (± 7 days). ^{b} Survival Follow-Up Visits to occur approximately every 3 months from Follow-Up Visit 2. | | | |

### Sample Size

The sample size is calculated to compare OS between nivolumab and platinum doublet chemotherapy, and to compare OS between nivolumab in combination with ipilimumab and platinum doublet chemotherapy, at a Type I error level of 0.0167 (two-sided) and 90% power for each comparison. The number of events and power are calculated assuming an exponential distribution in platinum doublet chemotherapy arm and a piecewise mixture distribution in each of the experimental treatment arms.

Approximately 1200 subjects are randomized to the 4 treatment groups in a 1:1:1:1 ratio. The final analysis is conducted after 257 events occur in the control group, and these events will be monitored by the un-blinded independent statistician supporting the DMC. Assuming a 20% screening failure rate, it is estimated that approximately 1500 subjects will be enrolled in order to have 1200 subjects randomized. assuming a piecewise constant accrual rate (8 subjects/month during Month 1 to 2, 40 subjects/month during Month 3 to 4, 85 subjects/month during Month 5 to 6, 138 subjects/month during Month 7 to 8, 170 subjects/month after Month 8), it will take approximately 48 months to obtain the required number of death for the final OS analysis (14 months for accrual and 34 months for survival follow up).

### End Point

OS is a primary endpoint for this study. If OS superiority is demonstrated for at least one comparison, a gate keeping testing approach for the key secondary endpoints will be applied to additional experimental vs. control comparisons as described in the statistical analysis plan. Key secondary endpoints include PFS and ORR based on BICR assessments.

Each of the three primary OS analyses will be conducted using a two-sided log-rank test stratified by histology and PD-L1 status in all randomized subjects using Hochberg's procedure to address multiplicity. Hazard ratios (HR) and corresponding two-sided (1-adjusted α) % confidence intervals (CI) will be estimated using a Cox proportional hazard model, with treatment group as a single covariate, stratified by the above factors. OS curves, OS medians with 95% CIs, and OS rates at 12 and 24 months with 95% CIs will be estimated using Kaplan-Meier methodology. If OS superiority is demonstrated for at least one comparison, a gatekeeping testing approach for the key secondary endpoints will be applied to additional experimental vs. control comparisons as described in the statistical analysis plan. The key secondary endpoints will be tested in the following hierarchical order:
1) PFS (based on BICR assessments) analyses will be conducted using a two-sided log-rank test stratified by histology and PD-L1 status in all randomized subjects to compare each of the three experimental treatments to the control group. HRs and corresponding two-sided (1-adjusted α) % CIs will be estimated using a Cox proportional hazard model, with treatment group as a single covariate, stratified by the above factors. PFS curves, PFS medians with 95% CIs, and PFS rates at 6 and 12 months with 95% CIs will be estimated using Kaplan-Meier methodology.
2) ORR (based on BICR assessments) analyses will be conducted using a two-sided Cochran-Mantel-Haenszel (CMH) test stratified by PD-L1 status and histology to compare each of the three experiment treatments to the control group. Associated odds ratios and (1-adjusted α) % CI will also be calculated. Additionally, ORRs and their corresponding 95% exact CIs will be calculated using the Clopper-Pearson method for each of the four treatment groups.
3) Pairwise comparison of OS among experimental arms will be conducted using a two-sided log-rank test stratified by histology and PD-L1 status. HRs and corresponding two-sided (1-adjusted α) % CIs will be estimated using a Cox proportional hazard model, with treatment group as a single covariate, stratified by the above factors.

Descriptive analyses of PFS, and ORR will be performed to evaluate differences between Nivolumab monotherapy and Nivolumab in combination of Ipilimumab groups. These include HRs and medians with corresponding two- sided 95% CIs for PFS, as well as an ORR odds ratio with corresponding 95% CI.

### Analyses

Analyses of PD-L1 expression will be descriptive. Distribution of PD-L1 expression will be examined based on overall population. Potential associations between PD-L1 expression and efficacy measures (ORR, OS and PFS) will be assessed. If there is an indication of a meaningful association, further evaluation will be conducted to explore PD-L1 expression as a predictive biomarker by estimating the interaction effect between PD-L1 expression and treatment.

The results will show whether in chemotherapy-naive subjects with stage IV or recurrent NSCLC, the administration of nivolumab or nivolumab in combination with ipilimumab will improve overall survival (OS) compared with platinum-doublet chemotherapy.

The present application claims benefit to U.S. Provisional Application Nos. 61/993,917, filed May 15, 2014; 62/005,640, filed May 30, 2014; and 62/152,669, filed April 24, 2015 which are incorporated herein by reference in their entireties.

### REFERENCES

Brahmer et al. (2010) J Clin Oncol 28:3167-75.
Brahmer et al. (2012) N Engl J Med 366:2455-65.
Butte MJ, et al. Immunity 2007;27:111-22.
Creelan et al. (2014) Cancer Control 21(1):80-89.
Drake et al. (2013) BJU Int 112(Supplement 3):1-17.
Escudier et al. (2010) J Clin Oncol 28(13):2144-50.
Flies et al. (2011) Yale J Biol Med 84:409-21.
Hamid and Carvajal (2013) Expert Opin Biol Ther 13(6):847-61.
Hamid et al. (2013) N Engl J Med 369:134-144.
Hanna et al. (2004) J Clin Oncol 22:1589-97.
Herbst et al. (2013) J Clin Oncol 31(suppl):3000. Abstract.
Hipp et al. (2008) Blood 111:5610-20.
Hodi et al. (2010) N Engl J Med 363:711-23.
Houben et al. (2009) Mol Cancer Ther 28:433-40.
Johnson et al. (2013) Cancer Immunol Res 1:373-77.
Khleif (2013) In: Proceedings from the European Cancer Congress 2013; September 27-October 1, 2013; Amsterdam, The Netherlands. Abstract 802.
Ko et al. (2009) Clin Cancer Res 15:2148-57.
Ko et al. (2010) Cancer Res 70:3526-36.
McDermott and Atkins (2013) Cancer Med 2(5):662-73.
Molhoek et al. (2009) Cancer Immunol Immunother 58:867-76.
Motzer et al. (2007) N Engl J Med 356:115-24.
Motzer et al. (2009) Clin Genitourin Cancer 7(1):28-33
Motzer et al. (2013) N Engl J Med 369:722-31.
NCCN GUIDELINES® (2014), available at: http://www.nccn.org/professionals/physician_gls/f_guidelines.asp, last accessed May 14, 2014.
NCCN GUIDELINES®, Version 3.2014 - Non-Small Cell Lung Cancer, available at: http://www.nccn.org/professionals/physician_gls/pdf/nscl.pdf, last accessed May 14, 2014.
Ozao-Choy et al. (2009) Cancer Res 69:2514-22.
Pardoll (2012) Nat Rev Cancer 12:252-64.
PCT Publication No. WO 2007/113648, published October 11, 2007 by PFIZER PRODUCTS INC.
PCT Publication No. WO 2012/122444, published September 13, 2012 by PROVECTUS PHARMACEUTICALS, INC. *et al.*
PCT Publication No. WO 2012/145493, published October 26, 2012 by Amplimmune, Inc.
PCT Publication No. WO 2013/173223, published November 21, 2013 by Bristol-Myers Squibb Co.
PCT Publication No. WO 2013/014668, published January 31, 2013 by CURETECH LTD. *et al*. PCT Publication No. WO/2000/044777, published August 3, 2000 by IMCLONE SYSTEMS INCORPORATED *et al.*
Rini et al. (2010) J Clin Oncol 28(13): 2137-43.
Rini et al. (2011) Cancer 117:758-67.
Siegel et al. (2014) CA Cancer J Clin 64(1):9-29.
Sjoblom et al. (2006) Science 314:268-74.
Sonpavde et al. (2008) Expert Opin Investig Drugs 17(5):741-8.
Sternberg et al. (2010) J Clin Oncol 28(6):1061-8.
Topalian et al. (2012a) N Engl J Med 366:2443-54.
Topalian et al. (2012b) Curr Opin Immunol 24:207-12.
Topalian et al. (2014) J Clin Oncol 32(10):1020-30.
USAN Council Statement (2013) Pembrolizumab: Statement on a nonproprietary name adopted by the USAN Council (ZZ-165), November 27, 2013.
U.S. Patent No. 5,952,199, issued September 14, 1999 to DAVIS-SMYTH et al.
U.S. Patent No. 5,977,318, issued November 2, 1999 to Linsley et al.
U.S. Patent No. 6,051,227, issued April 18, 2000 to Allison et al.
U.S. Patent No. 6,682,736, issued January 27, 2004 to Hanson et al.
U.S. Patent No. 6,808,710, issued October 26, 2004 to Wood et al.
U.S. Patent No. 6,984,720, issued January 10, 2006 to Korman et al.
U.S. Patent No. 7,034,121, issued April 25, 2006 to Carreno et al.
U.S. Patent No. 7,169,901, issued January 30, 2007 to Baca et al.
U.S. Patent No. 7,297,334, issued November 20, 2007 to Baca et al.
U.S. Patent No. 7,423,125, issued September 9, 2008 to Alitalo et al.
U.S. Patent No. 7,498,414, issued March 3, 2009 to Zhu et al.
U.S. Patent No. 7,605,238, issued October 20, 2009 to Korman et al.
U.S. Patent No. 7,488,802, issued February 10, 2009 to Collins et al.
U.S. Patent No. 7,943,743, issued May 17, 2011 to Korman et al.
U.S. Patent No. 7,972,596, issued July 5, 2011 to Wu et al.
U.S. Patent No. 8,008,449, issued August 30, 2011 to Korman et al.
U.S. Patent No. 8,034,905, issued October 11, 2011 to Kavlie et al.
U.S. Patent No. 8,168,757, issued May 1, 2012 to Finnefrock et al.
U.S. Patent No. 8,217,149, issued July 10, 2012 to Irving et al.
U.S. Patent No. 8,354,509, issued January 15, 2013 to Carven et al.
U.S. Patent No. 8,609,089, issued December 17, 2013, to Langermann et al.
U.S. Patent No. 8,686,119, issued April 1, 2014 to Rotem-Yehudar et al.
U.S. Patent No. 8,779,105, issued July 15, 2014 to Korman et al.
U.S. Patent No. 8,900,587, issued December 2, 2014 to Carven et al.
U.S. Publ. No. 2012/263677, published October 18, 2012 to Eagle et al.
Yang et al. (2007) J Immunother 30(8):825-30.
Wang et al. (2014) In vitro characterization of the anti-PD-1 antibody nivolumab, BMS-936558, and in vivo toxicology in non-human primates, Cancer Imm Res, in press.
Wolchok et al. (2013) N Engl J Med 369(2):122-33.

The following exemplary embodiments form part of the disclosure of the invention
1. A method for treating a subject afflicted with a lung cancer comprising administering to the subject a combination of therapeutically effective amounts of:
   (a) an anti-cancer agent which is an antibody or an antigen-binding portion thereof that binds specifically to a Programmed Death- 1 (PD-1) receptor and inhibits PD-1 activity; and
   (b) another anti-cancer agent.
2. The method of embodiment 1, wherein the lung cancer is non-small cell lung cancer (NSCLC).
3. The method of embodiment 2, wherein the NSCLC has a squamous histology.
4. The method of embodiment 2, wherein the NSCLC has a non-squamous histology.
5. The method of embodiment 1, wherein the anti-PD-1 antibody or antigen-binding portion thereof cross-competes with nivolumab for binding to human PD-1.
6. The method of embodiment 1, wherein the anti-PD-1 antibody or antigen-binding portion thereof is a chimeric, humanized or human monoclonal antibody or a portion thereof.
7. The method of any one of embodiments 1-4, wherein the anti-PD-1 antibody or antigen-binding portion thereof comprises a heavy chain constant region which is of a human IgGl or IgG4 isotype.
8. The method of embodiment 1, wherein the anti-PD-1 antibody is nivolumab.
9. The method of embodiment 1, wherein the anti-PD-1 antibody is pembrolizumab.
10. The method of embodiment 1, wherein the other anti-cancer agent is a platinum-based doublet chemotherapy (PT-DC).
11. The method of embodiment 10, wherein the anti-PD-1 antibody or antigen-binding portion thereof is administered at a dose ranging from 0.1 to 10.0 mg/kg body weight once every 2, 3 or 4 weeks.
12. The method of embodiment 11, wherein the anti-PD-1 antibody or antigen-binding portion thereof is administered at a dose of 5 or 10 mg/kg body weight once every 3 weeks.
13. The method of any of embodiments 10-12, wherein the PT-DC was administered concurrently with the anti-PD-1 antibody or antigen-binding portion thereof for 4 doses of the anti-PD-1 antibody or antigen-binding portion thereof, followed by repeated administration of the anti-PD-1 antibody or antigen-binding portion thereof alone.
14. The method of embodiment 13, wherein the PT-DC is a combination of gemcitabine and cisplatin.
15. The method of embodiment 14, wherein the gemcitabine is administered at a dose of 1250 mg/m² in combination with cisplatin administered at a dose of 75 mg/m².
16. The method of embodiment 13, wherein the PT-DC is a combination of pemetrexed and cisplatin.
17. The method of embodiment 16, wherein the pemetrexed is administered at a dose of 500 mg/m² in combination with cisplatin administered at a dose of 75 mg/m².
18. The method of embodiment 10, wherein the PT-DC is a combination of paclitaxel and carboplatin.
19. The method of embodiment 18, wherein the paclitaxel is administered at a dose of 200 mg/m in combination with carboplatin administered at a target area under the curve of 6 mg/mL/mindose (AUC6).
20. The method of embodiment 1, wherein the other anti-cancer agent is an EGFR-targeted tyrosine kinase inhibitor (TKI).
21. The method of embodiment 20, wherein the EGFR-targeted TKI is erlotinib.
22. The method of embodiment 21, wherein the erlotinib is orally administered at a dose of 150 mg daily.
23. The method of embodiment 22, wherein the anti-PD-1 antibody or antigen-binding portion thereof is administered at a dose of 3 mg/kg body weight once every 2 weeks.
24. The method of any of embodiments 20-23, wherein the combination of the anti-PD-1 antibody or antigen-binding portion and erloiinib is administered for as long as clinical benefit is observed or until unmanageable toxicity or disease progression occurs,
25. The method of embodiment 1, wherein the other anti-cancer agent is bevacizumab.
26. The method of embodiment 21, wherein the bevacizumab is intravenously administered at a dose of 15 mg/kg once every 3 weeks.
27. The method of embodiment 26, wherein the anti-PD-1 antibody or antigen-binding portion thereof is administered at a dose of 5 mg/kg body weight once every 3 weeks.
28. The method of embodiment 26, wherein the anti-PD-1 antibody or antigen-binding portion thereof is administered at a dose of 3 mg/kg body weight once every 2 weeks.
29. The method of any of embodiments 25-28, wherein the combination of the anti-PD-1 antibody or antigen-binding portion and bevacizumab is administered for as long as clinical benefit is observed or until unmanageable toxicity or disease progression occurs.
30. The method of embodiment 1, wherein the other anti-cancer agent is an antibody or an antigen-binding portion thereof that binds specifically to Cytotoxic T-Lymphocyte Antigen-4 (CTLA-4) and inhibits CTLA-4 activity.
31. The method of embodiment 30, wherein the anti-CTLA-4 antibody or antigen-binding portion thereof cross-competes with ipilimumab for binding to human CTLA-4.
32. The method of embodiment 30, wherein the anti-CTLA-4 antibody or antigen-binding portion thereof is a chimeric, humanized or human monoclonal antibody or a portion thereof.
33. The method of any one of embodiments 30-32, wherein the anti-CTLA-4 antibody or antigen-binding portion thereof comprises a heavy chain constant region which is of a human IgGl isotype.
34. The method of embodiment 30, wherein the anti-CTLA-4 antibody is ipilimumab.
35. The method of embodiment 30, wherein the anti-CTLA-4 antibody is tremelimumab.
36. The method of embodiment 30, comprising:
   (a) an induction phase, wherein the anti-PD-1 and anti-CTLA-4 antibodies or antigen- binding portions thereof are administered in combination in 2, 4, 6, 8 or 10 doses, each dose ranging from 0.1 to 10.0 mg/kg body weight administered at least once every 2, 3, or 4 weeks; followed by
   (b) a maintenance phase, wherein no anti-CTLA-4 antibody or antigen-binding portion thereof is administered and the anti-PD-1 antibody or antigen-binding portion thereof is repeatedly administered at a dose ranging from 0.1 to 10 mg/kg at least once every 2, 3 or 4 weeks.
37. The method of embodiment 36, wherein:
   (a) the induction phase comprises 4 combination doses administered at 3-week intervals, wherein:
      (i) the anti-PD-1 antibody or antigen-binding portion thereof is administered at 3 mg/kg body weight and the anti-CTLA-4 antibody or antigen-binding portion thereof is administered at 1 mg/kg body weight;
      (ii) the anti-PD-1 antibody or antigen-binding portion thereof is administered at 1 mg/kg body weight and the anti-CTLA-4 antibody or antigen-binding portion thereof is administered at 3 mg/kg body weight;
      (iii) the anti-PD-1 antibody or antigen-binding portion thereof is administered at 1 mg/kg body weight and the anti-CTLA-4 antibody or antigen-binding portion thereof is administered at 1 mg/kg body weight; or
      (iv) the anti-PD-1 antibody or antigen-binding portion thereof is administered at 3 mg/kg body weight and the anti-CTLA-4 antibody or antigen-binding portion thereof is administered at 3 mg/kg body weight; and
   (b) the maintenance phase comprises repeated administration of the anti-PD-1 antibody or antigen-binding portion thereof at a dose of 3 mg/kg every 2 weeks for as long as clinical benefit is observed or until unmanageable toxicity or disease progression occurs.
38. The method of any embodiment 36, wherein the anti-PD-1 and anti-CTLA-4 antibodies are formulated for intravenous administration.
39. The method of embodiment 36, wherein the anti-PD-1 antibody or antigen-binding portion thereof and the anti-CTLA-4 antibody or antigen-binding portion thereof are administered sequentially to the subject during the induction phase.
40. The method of embodiment 39, wherein the anti-PD-1 and anti-CTLA-4 antibodies are administered within 30 minutes of each other.
41. The method of embodiment 39, wherein
   (a) the anti-PD-1 antibody or antigen-binding portion thereof is administered before the anti-CTLA-4 antibody or antigen-binding portion thereof; or
   (b) the anti-CTLA-4 antibody or antigen-binding portion thereof is administered before the anti-PD-1 antibody or antigen-binding portion thereof.
42. The method of embodiment 36, wherein the anti-PD-1 antibody or antigen-binding portion thereof and the anti-CTLA-4 antibody or antigen-binding portion thereof are administered concurrently in separate compositions.
43. The method of embodiment 36, wherein the anti-PD-1 antibody or antigen-binding portion thereof and the anti-CTLA-4 antibody or antigen-binding portion thereof are admixed as a single composition for concurrent administration.
44. The method of embodiment 36, wherein the anti-PD-1 antibody or antigen-binding portion thereof is administered at a subtherapeutic dose.
45. The method of embodiment 36, wherein the anti-CTLA-4 antibody or antigen-binding portion thereof is administered at a subtherapeutic dose.
46. The method of embodiment 36, wherein the anti-PD-1 antibody or antigen-binding portion thereof and the anti-CTLA-4 antibody or antigen-binding portion thereof are each administered at a subtherapeutic dose.
47. The method of embodiment 36, wherein administration of the anti-PD-1 antibody in the maintenance phase is continued for as long as clinical benefit is observed or until unmanageable toxicity or disease progression occurs.
48. A kit for treating a subject afflicted with a lung cancer, the kit comprising:
   (a) a dosage ranging from 0.1 to 10 mg/kg body weight of an anti-cancer agent which is an antibody or an antigen-binding portion thereof that specifically binds to the PD-1 receptor and inhibits PD-1 activity;
   (b) a dosage of another anti-cancer agent which is
      (i) a platinum-based doublet chemotherapy;
      (ii) an EGFR-targeted tyrosine kinase inhibitor;
      (iii) bevacizumab; or
      (iv) a dosage ranging from 0.1 to 10 mg/kg body weight of an antibody or an antigen-binding portion thereof that specifically binds to and inhibits CTLA-4; and
   (c) instructions for using the anti-PD-1 antibody and the other anti-cancer agent in the method of any of embodiments 1, 10, 20, 25 or 30.

## Claims

1. An antibody or an antigen-binding portion thereof that binds specifically to a Programmed Death-1 (PD-1) receptor and inhibits PD-1 activity ("anti-PD-1 antibody") for use in a method for treating a subject afflicted with a lung cancer, wherein the subject is administered a combination of:
(a) the anti-PD-1 antibody; and
(b) another anti-cancer agent selected from the group consisting of:
(i) a platinum-based doublet chemotherapy (PT-DC), wherein the PT-DC is a combination of (i) gemcitabine at a dose of 1250 mg/m² and cisplatin at a dose of 75 mg/m², (ii) pemetrexed at a dose of 500 mg/m² and cisplatin at a dose of 75 mg/m², or (iii) paclitaxel at a dose of 200 mg/m² and carboplatin at a target area under the curve of 6 mg/mL/mindose (AUC6);
(ii) an EGFR-targeted tyrosine kinase inhibitor;
(iii) bevacizumab; and
(iv) an antibody or an antigen-binding portion thereof that specifically binds to and inhibits CTLA-4.

2. The anti-PD-1 antibody for use according to claim 1, wherein the lung cancer is non-small cell lung cancer (NSCLC).

3. The anti-PD-1 antibody for use according to claim 2, wherein the NSCLC has a squamous histology.

4. The anti-PD-1 antibody for use according to claim 2, wherein the NSCLC has a non-squamous histology.

5. The anti-PD-1 antibody for use according to any one of claims 1 to 4, wherein the anti-PD-1 antibody cross-competes with nivolumab for binding to human PD-1.

6. The anti-PD-1 antibody for use according to any one of claims 1 to 5, wherein the anti-PD-1 antibody is nivolumab.

7. The anti-PD-1 antibody for use according to any one of claims 1 to 5, wherein the anti-PD-1 antibody is pembrolizumab.

8. The anti-PD-1 antibody for use according to any one of claims 1 to 7, wherein the PT-DC is a combination of gemcitabine at a dose of 1250 mg/m² and cisplatin at a dose of 75 mg/m².

9. The anti-PD-1 antibody for use according to any one of claims 1 to 7, wherein the PT-DC is a combination of pemetrexed at a dose of 500 mg/m² and cisplatin at a dose of 75 mg/m².

10. The anti-PD-1 antibody for use according to any one of claims 1 to 7, wherein the PT-DC is a combination of paclitaxel at a dose of 200 mg/m² and carboplatin administered at a target area under the curve of 6 mg/mL/min dose (AUC6).

11. The anti-PD-1 antibody for use according to any one of claims 1 to 10, wherein the anti-PD-1 antibody is administered at a dose of 3 mg/kg body weight once every 2 weeks.

12. The anti-PD-1 antibody for use according to any one of claims 1 to 11, wherein administration of the anti-PD-1 antibody is continued for as long as clinical benefit is observed or until unmanageable toxicity or disease progression occurs.

13. The anti-PD-1 antibody for use according to any one of claims 1 to 12, wherein the lung cancer is EGFR mutation-positive (EGFR MT) NSCLC.

14. The anti-PD-1 antibody for use according to claim 13, wherein the lung cancer is T790M positive.

15. A kit for use in treating a subject afflicted with a lung cancer, the kit comprising:
(a) a dosage ranging from 0.1 mg/kg to 10 mg/kg body weight of an antibody or an antigen-binding portion thereof that specifically binds to the Programmed Death-1 (PD-1) receptor and inhibits PD-1 activity ("anti-PD-1 antibody");
(b) a dosage of another anti-cancer agent which is
(i) a platinum-based doublet chemotherapy (PT-DC), wherein the PT-DC is a combination of (i) gemcitabine at a dose of 1250 mg/m² and cisplatin at a dose of 75 mg/m², (ii) pemetrexed at a dose of 500 mg/m² and cisplatin at a dose of 75 mg/m², or (iii) paclitaxel at a dose of 200 mg/m² and carboplatin at a target area under the curve of 6 mg/mL/min dose (AUC6);
(ii) an EGFR-targeted tyrosine kinase inhibitor;
(iii) bevacizumab; or
(iv) a dosage ranging from 0.1 to 10 mg/kg body weight of an antibody or an antigen-binding portion thereof that specifically binds to and inhibits CTLA-4; and
(c) instructions for using the anti-PD-1 antibody and the other anti-cancer agent according to any one of claims 1 to 14.
